# EUROPÄISCHE PATENTSCHRIFT

(11) **EP 2 086 969 B1**
(45) Veröffentlichungstag und Bekanntmachung des Hinweises auf die Patenterteilung: **02.03.2011**
(21) Anmeldenummer: 07802224.1
(22) Anmeldetag: 08.09.2007
(51) Int. Cl.: C07D 417/04, C07D 417/14, A61K 31/4427, A61P 9/00

(54) **3-CYANO-5-THIAZAHETEROARYL-DIHYDROPYRIDINE UND IHRE VERWENDUNG ZUR BEHANDLUNG KARDIOVASKULÄRER ERKRANKUNGEN**
3-CYANO-5-THIAZAHETEROARYL-DIHYDROPYRIDINE AND THE USE THEREOF FOR THE TREATMENT OF CARDIOVASCULAR DISEASES
3-CYANO-5-THIAZAHÉTÉROARYL-DIHYDROPYRIDINE ET SON UTILISATION POUR TRAITER DES MALADIES CARDIOVASCULAIRES

(30) Priorität: 22.09.2006 DE 102006044696
(43) Veröffentlichungstag der Anmeldung: 12.08.2009
(73) Patentinhaber: Bayer Schering Pharma Aktiengesellschaft, 13353 Berlin (DE)
(72) Erfinder: BÄRFACKER, Lars, 46047 Oberhausen (DE); KOLKHOF, Peter, 42113 Wuppertal (DE); SCHLEMMER, Karl-Heinz, 42113 Wuppertal (DE); GROSSER, Rolf, 51373 Leverkusen (DE); NITSCHE, Adam, 50259 Pulheim (DE)
(86) Internationale Anmeldenummer: PCT/EP2007/007844
(87) Internationale Veröffentlichungsnummer: WO 2008/034534

(56) Entgegenhaltungen:
- EP-A- 0 183 091
- WO-A-2005/097118
- MANNHOLD ET AL: "Calcium- and calmodulin-antagonism of elnadipine derivatives" EUR. J. CHEM., Bd. 27, 1992, Seiten 229-235, XP002464901 in der Anmeldung erwähnt

## Beschreibung

Die vorliegende Anmeldung betrifft neue, Aryl-substituierte 3-Cyano-5-thiazolyl- und 3-Cyano-5-thiadiazolyl-1,4-dihydropyridine, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von kardiovaskulären Erkrankungen.

Aldosteron spielt eine Schlüsselrolle in der Aufrechterhaltung der Flüssigkeits- und Elektrolythomöostase, indem es im Epithel des distalen Nephrons die Natriumretention und Kaliumsekretion fördert, was zur Konstanthaltung des Extrazellulärvolumens und damit zur Blutdruckregulation beiträgt. Daneben entfaltet Aldosteron direkte Effekte auf die Struktur und Funktion des Herz- und Gefäßsystems, wobei die dafür zugrunde liegenden Mechanismen noch nicht erschöpfend geklärt sind [R.E. Booth, J.P. Johnson, J.D. Stockand, Adv. Physiol. Educ. 26 (1), 8-20 (2002)].

Aldosteron ist ein Steroidhormon, das in der Nebennierenrinde gebildet wird. Seine Produktion wird indirekt ganz wesentlich in Abhängigkeit von der Nierendurchblutung reguliert. Jede Abnahme der Nierendurchblutung führt in der Niere zu einer Ausschüttung des Enzyms Renin in den Blutkreislauf. Dieses wiederum aktiviert die Bildung von Angiotensin II, das einerseits verengend auf die arteriellen Blutgefäße wirkt andererseits aber auch die Bildung von Aldosteron in der Nebennierenrinde stimuliert. Somit fungiert die Niere als Blutdruck- und damit indirekter Volumen-Sensor im Blutkreislauf und wirkt über das Renin-Angiotensin-Aldosteron-System kritischen Volumenverlusten entgegen, indem einerseits der Blutdruck gesteigert wird (Angiotensin 11-Wirkung), andererseits durch verstärkte Rückresorption von Natrium und Wasser in der Niere der Füllungszustand des Gefäßsystems wieder ausgeglichen wird (Aldosteron-Wirkung).

Dieses Regelsystem kann in vielfältiger Weise krankhaft gestört sein. So führt eine chronische Minderdurchblutung der Nieren (z.B. infolge einer Herzinsuffizienz und des hierdurch verursachten Blutrückstaus im Venensystem) zu einer chronisch überhöhten Ausschüttung von Aldosteron. Diese wiederum zieht eine Expansion des Blutvolumens nach sich und verstärkt hiermit die Herzschwäche durch ein Volumenüberangebot an das Herz. Eine Stauung von Blut in den Lungen mit Atemnot und Ödembildung in den Extremitäten sowie Aszites und Pleuraergüsse können die Folge sein; die Nierendurchblutung sinkt weiter ab. Überdies führt die übersteigerte Aldosteron-Wirkung zu einer Minderung der Kalium-Konzentration im Blut und in der Extrazellulärflüssigkeit. In ohnehin vorgeschädigten Herzmuskeln kann die Unterschreitung eines kritischen Mindestwertes tödlich endende Herzrhythmusstörungen auslösen. Hierin dürfte eine der Hauptursachen des häufig bei Patienten mit Herzinsuffizienz eintretenden *plötzlichen Herztodes* zu suchen sein.

Zusätzlich wird Aldosteron auch für eine Reihe der typischerweise bei Herzinsuffizienten zu beobachtenden Umbauprozesse des Herzmuskels verantwortlich gemacht. Somit ist der *Hyperaldosteronismus* eine entscheidende Komponente in der Pathogenese und Prognose der Herzinsuffizienz, die ursprünglich durch unterschiedliche Schädigungen, wie z.B. einen Herzinfarkt, eine Herzmuskelentzündung oder Bluthochdruck, ausgelöst werden kann. Diese Annahme wird durch die Tatsache erhärtet, dass in umfangreichen klinischen Studien in Patientenkollektiven mit chronischer Herzinsuffizienz bzw. nach akutem Myokardinfarkt durch Anwendung von Aldosteron-Antagonisten die Gesamtmortalität deutlich gesenkt wurde [B. Pitt, F. Zannad, W.J. Remme et al., N. Engl. J. Med. 341, 709-717 (1999); B. Pitt, W. Remme, F. Zannad et al., N. Engl. J. Med. 348, 1309-1321 (2003)]. Dies konnte unter anderem durch eine Senkung der Inzidenz des plötzlichen Herztodes erreicht werden.

Neueren Studien zufolge findet man auch bei einem nicht unerheblichen Teil der Patienten, die unter einer essentiellen Hypertonie leiden, eine sogenannte normokaliämische Variante des primären Hyperaldosteronismus [Prävalenz bis 11% aller Hypertoniker: L. Seiler und M. Reincke, Der Aldosteron-Renin-Quotient bei sekundärer Hypertonie, Herz 28, 686-691 (2003)]. Als beste Diagnostikmethode dient beim normokaliämischen Hyperaldosteronismus der Aldosteron/Renin-Quotient der entsprechenden Plasmakonzentrationen, so dass auch relative Aldosteron-Erhöhungen in Bezug auf die Renin-Plasmakonzentrationen diagnostizierbar und letztlich therapierbar werden. Deshalb ist ein im Zusammenhang mit einer essentiellen Hypertonie diagnostizierter Hyperaldosteronismus ein Ansatzpunkt für eine kausale und prophylaktisch sinnvolle Therapie.

Weitaus seltener als die oben aufgeführten Formen des Hyperaldosteronismus sind solche Krankheitsbilder, bei denen die Störung entweder in den Hormon-produzierenden Zellen der Nebenniere selbst zu finden ist oder deren Anzahl oder Masse durch eine Hyperplasie oder Wucherung vermehrt ist. Adenome oder diffuse Hyperplasien der Nebennierenrinde sind die häufigste Ursache des auch als *Conn-Syndrom* bezeichneten primären Hyperaldosteronismus, dessen Leitsymptome Hypertonie und hypokaliämische Alkalose sind. Auch hier steht neben der chirurgischen Entfernung des krankhaften Gewebes die medikamentöse Therapie mit Aldosteron-Antagonisten im Vordergrund [H.A. Kühn und J. Schirmeister (Hrsg.), Innere Medizin, 4. Aufl., Springer Verlag, Berlin, 1982].

Ein anderes typischerweise mit Erhöhung der Aldosteron-Konzentration im Plasma einhergehendes Krankheitsbild ist die fortgeschrittene Leberzirrhose. Die Ursache der Aldosteronerhöhung liegt hier vorwiegend im infolge der Leberfunktionsstörung eingeschränkten Abbau des Aldosterons. Volumenüberlastung, Ödeme und Hypokaliämie sind die typischen Folgen, die in der klinischen Praxis durch Aldosteron-Antagonisten erfolgreich gelindert werden können.

Die Wirkungen von Aldosteron werden über den in den Zielzellen intrazellulär lokalisierten Mineralokorticoid-Rezeptor vermittelt. Die bislang verfügbaren Aldosteron-Antagonisten besitzen wie Aldosteron selbst eine Steroid-Grundstruktur. Begrenzt wird die Anwendbarkeit derartiger steroidaler Antagonisten durch ihre Wechselwirkungen mit den Rezeptoren anderer Steroidhormone, die zum Teil zu erheblichen Nebenwirkungen wie Gynäkomastie und Impotenz und zum Abbrechen der Therapie führen [M.A. Zaman, S. Oparil, D.A. Calhoun, Nature Rev. Drug Disc. 1, 621-636 (2002)].

Die Anwendung wirkstarker, nicht-steroidaler und für den Mineralokorticoid-Rezeptor selektiver Antagonisten bietet die Möglichkeit, dieses Nebenwirkungsprofil zu umgehen und dadurch einen deutlichen Therapievorteil zu erzielen.

Aufgabe der vorliegenden Erfindung ist die Bereitstellung neuer Verbindungen, die als selektive Mineralokorticoid-Rezeptor-Antagonisten für die Behandlung von Erkrankungen, insbesondere von kardiovaskulären Erkrankungen, eingesetzt werden können.

4-Fluorenonyl-1,4-dihydropyridin-Derivate als Mineralokorticoid-Rezeptor-Antagonisten werden in WO 2005/087740 offenbart. In WO 2005/097118 werden Verbindungen mit 4-Aryl-1,4-dihydropyridin-Kemstruktur als Aldosteron-Rezeptor-Antagonisten beansprucht. 4-Aryl-3-cyano-1,4-dihydropyridin-5-carbonsäureester und -amide werden in WO 2006/066011 als zum Teil duale Modulatoren von Steroidhormon-Rezeptoren und des L-Typ-Calciumkanals beschrieben. In EP 0 116 708-A1, EP 0 177 965-A2, EP 0 183 091-A2 und DE 3 709 352-A1 werden unter anderem Thiazolyl- und Thiadiazolyl-substituierte 1,4-Dihydropyridine als Calcium-Antagonisten oder -Agonisten zur Behandlung kardiovaskulärer Erkrankungen beansprucht. Über Struktur-Aktivitätsbeziehungen verschiedener 5-Heteroaryl-1,4-dihydropyridin-3-carbonsäureester wird in R. Mannhold et al., Eur. J. Med. Chem. 27, 229-235 (1992) berichtet.

Gegenstand der vorliegenden Erfindung sind Verbindungen der allgemeinen Formel (I) 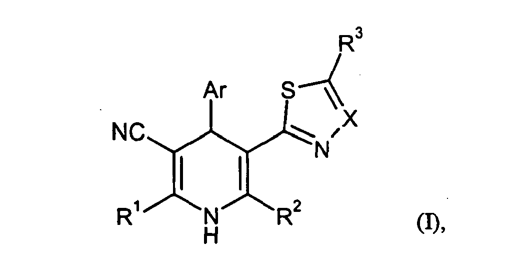 in welcher
- Ar oder: für (C₆-C₁₀)-Aryl oder 5- bis 10-gliedriges Heteroaryl mit bis zu zwei Ring-Heteroatomen aus der Reihe N, O und/oder S steht, welche jeweils ein- bis dreifach, gleich oder ver- schieden, mit Substituenten ausgewählt aus der Reihe Halogen, Cyano, (C₁-C₄)-Alkyl, (C₁- C₄)-Alkoxy und (C₁-C₄)-Alkylthio substituiert sein können, wobei die genannten Alkyl-, Alkoxy- und Alkylthio-Reste ihrerseits mit Cyano oder bis zu dreifach mit Fluor substituiert sein können,
- Ar: für eine Gruppe der Formel 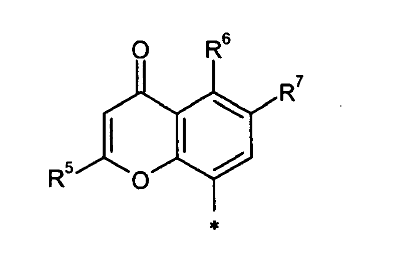
steht, worin
- *: die Verknüpfungsstelle mit dem Dihydropyridin-Ring,
- R⁵: Wasserstoff oder (₁-C₄)-Alkyl,
- R⁶: Wasserstoff, Fluor, Chlor, Cyano, Nitro, Trifluormethyl oder (C₁-C₄)-Alkyl, und
- R⁷: Wasserstoff oder Fluor bedeuten,
- R¹: für (C₁-C₆)-Alkyl, das mit Phenyl substituiert sein kann, oder für (C₁-C₆)-Alkylthio steht, wobei die genannten Alkyl- und Alkylthio-Reste ihrerseits bis zu dreifach mit Fluor sub- stituiert sein können,
- R²: für (C₁-C₆)-Alkyl, das mit Cyano, Hydroxy, (C₁-C₄)-Alkoxy, Trifluormethoxy, Amino, Mono-(C₁C₄)-alkylamino, Di-(C₁-C₄)-alkylamino, (C₃-C₆)-Cycloalkyl, Phenyl oder bis zu dreifach mit Fluor substituiert sein kann, oder für (C₃-C₆)-Cycloalkyl steht,
- X: für N oder C-R⁴ steht, und
- R³ und R⁴ oder: gleich oder verschieden sind und unabhängig voneinander für Wasserstoff, Halogen, Amino, Mono-(C₁-C₄)-alkylamino oder Di-(C₁-C₄)-alkylamino, für (C₁-C₄)-Alkyl, das mit Hydroxy, (C₁-C₄)-Alkoxy, Amino, Mono-(C₁-C₄)-alkylamino, Di-(C₁-C₄)-alkylamino oder bis zu dreifach mit Fluor substituiert sein kann, oder für Phenyl, das mit Halogen, Cyano, (C₁-C₄)-Alkyl, Trifluormethyl, (C₁-C₄)-Alkoxy Trifluormethoxy substituiert sein kann, stehen
oder
- R³ und R⁴,: wenn letzteres vorhanden ist, miteinander verknüpft sind und zusammen mit den Kohlenstoffatomen, an die sie gebunden sind, einen anellierten 5- bis 7-gliedrigen Cyclo- alkyl-Ring, welcher mit (C₁-C₄)-Alkyl oder (C₁-C₄)-Alkoxy substituiert sein kann und in welchem eine Ring-CH₂-Gruppe gegen ein O-Atom ausgetauscht sein kann, oder einen anellierten Phenyl- oder Pyridyl-Ring, welcher jeweils mit Halogen, Cyano, (C₁-C₄)-Alkyl, Trifluormethyl, (C₁-C₄)-Alkoxy oder Trifluormethoxy substituiert sein kann, bilden,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfasst Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
(C₁-C₆-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, n-Propyl, Isopropyl, n-Butyl, iso-Butyl, sec.-Butyl, tert.-Butyl, 1-Ethylpropyl, n-Pentyl, iso-Pentyl und n-Hexyl.
(C₃-C₆)-Cycloalkyl steht im Rahmen der Erfindung für einen monocyclischen, gesättigten Carbocyclus mit 3 bis 6 Ring-Kohlenstoffatomen. Beispielhaft und vorzugsweise seien genannt: Cyclopropyl, Cyclobutyl, Cyclopentyl und Cyclohexyl.
Ein anellierter 5- bis 7-gliedriger Cycloalkyl-Ring steht im Rahmen der vorliegenden Erfindung für einen bis auf die Doppelbindung der Anellierungsposition gesättigten Carbocyclus mit 5 bis 7 Ringatomen.
(C₁-C₄)-Alkoxy steht im Rahmen der Erfindung für einen geradkettigen oder verzweigten Alkoxyrest mit 1 bis 4 Kohlenstoffatomen. Beispielhaft und vorzugsweise seien genannt: Methoxy, Ethoxy, n-Propoxy, Isopropoxy, n-Butoxy und tert.-Butoxy.
(C₁-C₆)-Alkylthio und (C₁C₄)-Alkylthio stehen im Rahmen der Erfindung für einen geradkettigen oder verzweigten Alkylthiorest mit 1 bis 6 bzw. 1 bis 4 Kohlenstoffatomen. Bevorzugt ist ein geradkettiger oder verzweigter Alkylthiorest mit 1 bis 4 Kohlenstoffatomen. Beispielhaft und vorzugsweise seien genannt: Methylthio, Ethylthio, n-Propylthio, Isopropylthio, n-Butylthio, tert.-Butylthio, n-Pentylthio und n-Hexylthio.
Mono-(C₁-C₄)-alkylamino steht im Rahmen der Erfindung für eine Amino-Gruppe mit einem geradkettigen oder verzweigten Alkylsubstituenten, der 1 bis 4 Kohlenstoffatome aufweist. Beispielhaft und vorzugsweise seien genannt: Methylamino, Ethylamino, n-Propylamino, Isopropylamino, n-Butylamino und tert.-Butylamino.
Di-(C₁-C₄)-alkylamino steht im Rahmen der Erfindung für eine Amino-Gruppe mit zwei gleichen oder verschiedenen geradkettigen oder verzweigten Alkylsubstituenten, die jeweils 1 bis 4 Kohlenstoffatome aufweisen. Beispielhaft und vorzugsweise seien genannt: *N,N* Dimethylamino, *N,N* Diethylamino, *N-*Ethyl-*N-*methylamino, *N-*Methyl-*N-*n-propylamino, *N-*Isopropyl-*N-*n-propylamino, *N,N*-Diisopropylamino, *N-*n-Butyl-*N-*methylamino und *N*-tert-Butyl-*N*-methylamino.
(C₆-C₁₀)-Aryl steht im Rahmen der Erfindung für einen aromatischen Carbocyclus mit 6 oder 10 Ring-Kohlenstoffatomen. Bevorzugte Arylreste sind Phenyl und Naphthyl.
5- bis 10 gliedriges Heteroaryl, steht im Rahmen der Erfindung für einen mono- oder gegebenenfalls bicyclischen aromatischen Heterocyclus (Heteroaromaten) mit insgesamt 5 bis 10 Ringatomen, der bis zu zwei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Pyridyl, Pyrimidinyl, Pyridazinyl, Pyrazinyl, Benzofuranyl, Benzothienyl, Benzimidazolyl, Benzoxazolyl, Benzothiazolyl, Indolyl, Indazolyl, Chinolinyl, Isochinolinyl, Naphthyridinyl, Chinazolinyl, Chinoxalinyl, Phthalazinyl. Bevorzugt sind monocyclische 5- oder 6-gliedrige Heteroaryl-Reste mit bis zu zwei Ring-Heteroatomen aus der Reihe N, O und/oder S wie beispielsweise Furyl, Thienyl, Thiazolyl, Oxazolyl, Isothiazolyl, Isoxazolyl, Pyrazolyl, Imidazolyl, Pyridyl, Pyrimidinyl, Pyridazinyl, Pyrazinyl.
Halogen schließt im Rahmen der Erfindung Fluor, Chlor, Brom und Iod ein. Bevorzugt sind Fluor, Chlor oder Brom.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Bevorzugt sind Verbindungen der Formel (I), in welcher
- Ar: für eine Gruppe der Formel 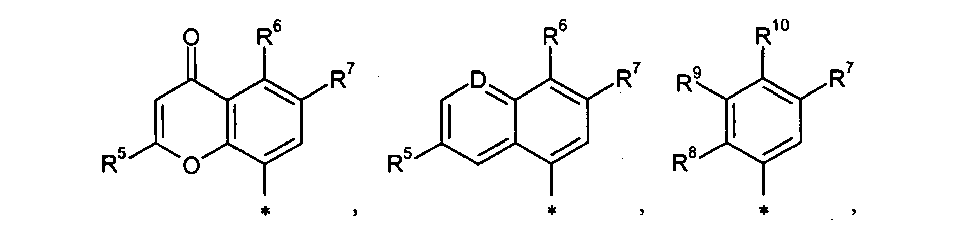 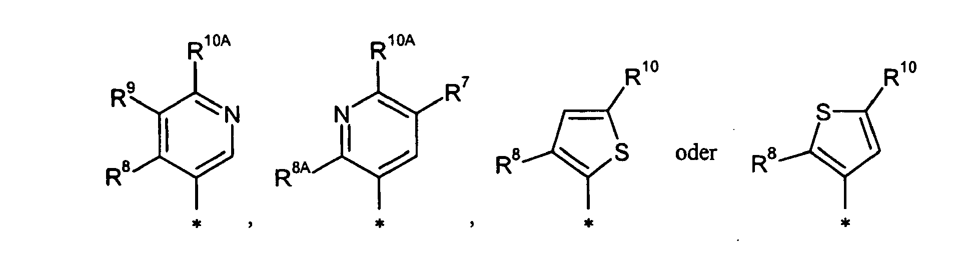
steht, worin
- *: die Verknüpfungsstelle mit dem Dihydropyridin-Ring,
- D: N oder CH,
- R⁵: Wasserstoff, Methyl oder Ethyl,
- R⁶: Wasserstoff, Fluor, Chlor oder Cyano,
- R⁷: Wasserstoff oder Fluor,
- R⁸: Fluor, Chlor, Brom, Cyano oder (C₁-C₄)-Alkyl, (C₁-C₄)-Alkoxy oder (C₁-C₄)- Alkylthio, welche jeweils bis zu dreifach mit Fluor substituiert sein können,
- R^{8A}: Cyano oder (C₁-C₄)-Alkyl, (C₁-C₄)-Alkoxy oder (C₁-C₄)-Alkylthio, welche jeweils bis zu dreifach mit Fluor substituiert sein können,
- R⁹: Wasserstoff, Fluor, Chlor oder Methyl,
- R¹⁰: Wasserstoff, Cyano, Fluor, Chlor oder Brom, nd
- R^{10A}: Wasserstoff oder Cyano bedeuten,
- R¹ für: (C₁-C₄)-Alkyl, das bis zu dreifach mit Fluor substituiert sein kann, steht,
- R² für: (C₁C₄)-Alkyl, das mit (C₁-C₄)-Alkoxy oder bis zu dreifach mit Fluor substituiert sein kann,steht,
- X: für N oder C-R⁴ steht, und
- R³ und R⁴ gleich: oder verschieden sind und unabhängig voneinander für Wasserstoff, Fluor, Chlor, Amino, Mono-(C₁-C₄)-alkylamino, Di-(C₁-C₄)-alkylamino oder (C₁-C₄)-Alkyl stehen oder
- R³ und R⁴,: wenn letzteres vorhanden ist, miteinander verknüpft sind und zusammen mit dem Thiazol-Ring, an den sie gebunden sind, eine Gruppe der Formel

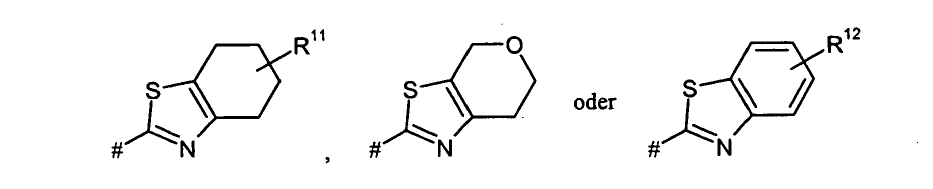

bilden, worin
- #: die Verlrnüpfungsstelle mit dem Dihydropyridin-Ring,
- R¹¹: Wasserstoff, (C₁-C₄)-Alkyl oder (C₁-C₄)-Alkoxy,
und
- R¹²: Wasserstoff, Fluor, Chlor, Brom, (C₁-C₄)-Alkyl, Trifluormethyl, (C₁-C₄)-Alkoxy oder Trifluormethoxy
bedeuten,
sowie ihre Salze, Solvate und Solvate der Salze.

Besonders bevorzugt sind Verbindungen der Formel (I), in welcher
- Ar: für eine Gruppe der Formel 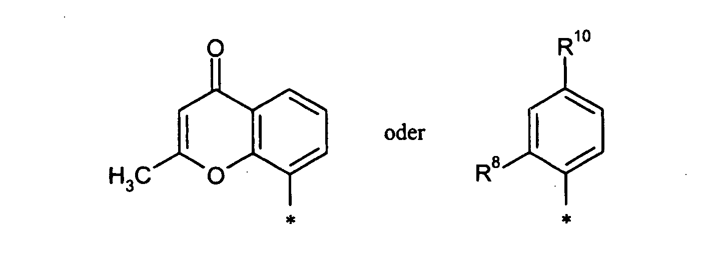
steht, worin
- *: die Verlrnüpfungsstelle mit dem Dihydropyridin-Ring,
- R⁸: Fluor, Chlor, Brom, Cyano, Methyl, Ethyl, Trifluormethyl, Methoxy oder Trifluor- methoxy, und
- R¹⁰: Fluor, Chlor oder Cyano bedeuten,
- R¹: für Methyl oder Trifluormethyl steht,
- R²: für Methyl, Ethyl, n-Propyl oder Methoxymethyl steht,
- X: für C-R⁴ steht,
- R³: für Wasserstoff, Methyl oder Ethyl steht,
- R⁴: für Wasserstoff, Methyl, Ethyl oder n-Propyl steht, oder

- R³ und R⁴: miteinander verknüpft sind und zusammen mit dem Thiazol-Ring, an den sie gebunden sind, eine Gruppe der Formel 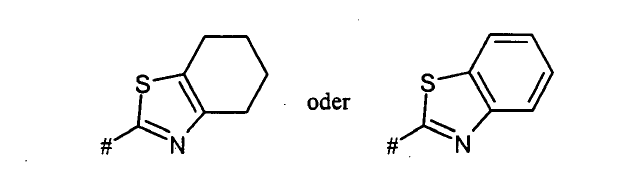
bilden, worin
# die Verknüpfungsstelle mit dem Dihydropyridin-Ring bedeutet, sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichnet, dass man eine Verbindung der Formel (II) 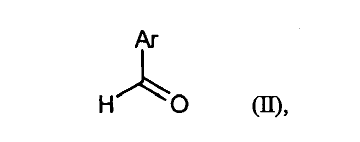 in welcher Ar die oben angegebene Bedeutung hat,
entweder

### [A] in einem einstufigen Verfahren (Eintopf-Reaktion) mit einer Verbindung der Formel (III)

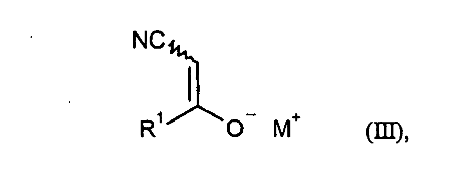

in welcher R¹ die oben angegebene Bedeutung hat
und
- M⁺: für ein Alkalimetall-Ion wie Li⁺, Na⁺ oder K⁺ steht,
und einer Verbindung der Formel (IV) 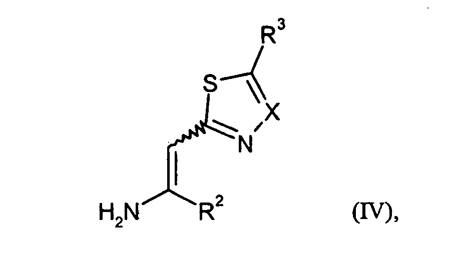 in welcher R², R³ und X jeweils die oben angegebenen Bedeutungen haben, umsetzt oder

### [B] in einem einstufigen Verfahren (Eintopf-Reaktion) mit einer Verbindung der Formel (V)

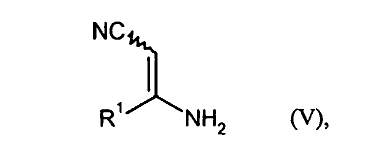

in welcher R¹ die oben angegebenen Bedeutungen hat,
und einer Verbindung der Formel (VI) 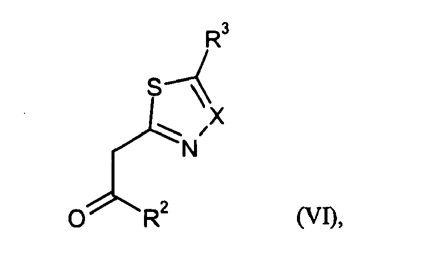 in welcher R², R³ und X jeweils die oben angegebenen Bedeutungen haben, umsetzt
oder

### [C] in einem zweistufigen Verfahren zunächst mit einer Verbindung der Formel (VI) in eine Verbindung der Formel (VII)

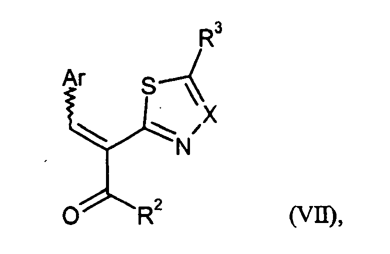

in welcher Ar, R², R³ und X jeweils die oben angegebenen Bedeutungen haben, überführt und diese dann in einem zweiten Schritt mit einer Verbindung der Formel (V) umsetzt,
und gegebenenfalls die resultierenden Verbindungen der Formel (I) nach dem Fachmann bekannten Methoden in ihre Enantiomere und/oder Diastereomere trennt und/oder mit den entsprechenden (i) Lösungsmitteln und/oder (ii) Basen oder Säuren in ihre Solvate, Salze und/oder Solvate der Salze überführt.

Die Umsetzungen nach Verfahren [A], [B] und [C] erfolgen im Allgemeinen in inerten Lösungsmitteln, gegebenenfalls in Gegenwart einer Säure und/oder Base, in einem Temperaturbereich von +20°C bis zum Siedepunkt des Lösungsmittels bei Normaldruck.

Inerte Lösungsmittel für die Verfahren [A] und [B] sowie die zweite Stufe des Verfahrens [C] sind beispielsweise Alkohole wie Methanol, Ethanol, n-Propanol, Isopropanol, n-Butanol oder *tert.-*Butanol, oder andere Lösungsmittel wie Acetonitril, Tetrahydrofuran, Dioxan, 1,2-Dimethoxyethan, Toluol oder Eisessig. Bevorzugt werden die Umsetzungen in Ethanol oder Isopropanol bei der jeweiligen Rückfluss-Temperatur unter Normaldruck durchgeführt.

Die Umsetzung nach Verfahren [A] wird bevorzugt in Gegenwart einer Säure wie beispielsweise Essigsäure, Trifluoressigsäure, p-Toluolsulfonsäure, Methansulfonsäure oder Tetrabutylammoniumhydrogensulfat durchgeführt; besonders bevorzugt ist der Zusatz von Essigsäure.

Die Umsetzungen gemäß Verfahren [B] sowie der zweiten Stufe von Verfahren [C] können gegebenenfalls vorteilhaft unter Zusatz einer Base durchgeführt werden. Hierfür eignen sich beispielsweise Alkali- oder Erdalkalicarbonate wie Natrium-, Kalium- oder Cäsiumcarbonat, oder Alkoholate wie Natrium- oder Kaliummethanolat, Natrium- oder Kaliumethanolat oder Natrium- oder Kalium-*tert.*-butylat. Bevorzugt wird Kalium-*tert.*-butylat verwendet.

Inerte Lösungsmittel für die erste Stufe des Verfahrens [C] sind beispielsweise Halogenkohlenwasserstoffe wie Dichlormethan, Trichlormethan, Tetrachlormethan, Trichlorethan oder 1,2-Dichlorethan, oder andere Lösungsmittel wie Acetonitril, Pyridin, Benzol, Toluol, Xylol, Chlorbenzol, Hexan oder Cyclohexan. Bevorzugt erfolgen die Umsetzungen in Dichlormethan oder Toluol bei der jeweiligen Rückfluss-Temperatur unter Normaldruck.

Die Umsetzung gemäß der ersten Stufe von Verfahren [C] wird bevorzugt in Gegenwart einer Säure in Kombination mit Piperidin oder Pyridin als Base und/oder einem wasserentziehenden Mittel, wie beispielsweise Molekularsieb, durchgeführt. Als Säuren eignen sich beispielsweise Essigsäure oder p-Toluolsulfonsäure. Besonders bevorzugt ist eine Reaktionsführung unter Zusatz von Piperidiniumacetat [siehe auch das nachfolgende Reaktionsschema 11; zur Synthese von 1,4-Dihydropyridinen allgemein vgl. auch D.M. Stout, A.I. Meyers, Chem. Rev. 1982, 82, 223-243; H. Meier et al., Liebigs Ann. Chem. 1977, 1888; H. Meier et al., ibid. 1977, 1895; H. Meier et al., ibid. 1976, 1762; F. Bossert et al., Angew. Chem. 1981, 93, 755].

Die Verbindungen der Formel (II) sind kommerziell erhältlich, literaturbekannt oder können in Analogie zu literaturbekannten Verfahren hergestellt werden (vgl. nachfolgende Reaktionsschemata 1-7). Die Verbindungen der Formeln (III), (IV), (V) und (VI) sind kommerziell erhältlich, literaturbekannt oder nach literaturbekannten Methoden herstellbar [zu den Verbindungen der Formel (VI) vgl. auch nachfolgende Reaktionsschemata 8-10].

Die Herstellung der erfindungsgemäßen Verbindungen kann durch die folgenden Syntheseschemata veranschaulicht werden: 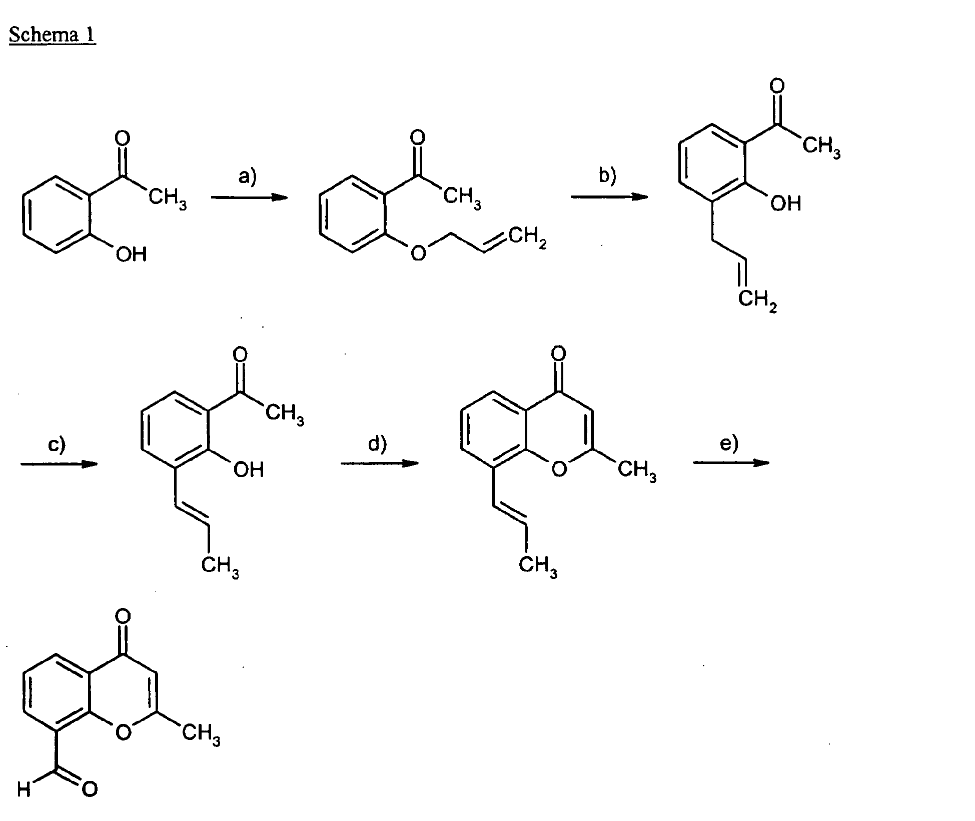 [a): Allylbromid, Kaliumcarbonat, cat. Kaliumiodid, Aceton, Rückfluss; b): 230°C, 4 h; c): Bis-(benzonitril)dichlorpalladium(II), Toluol, 120°C, 16 h; d): Acetylchlorid, Natriumhydrid, THF, 10-25°C, 16 h; e): 1. Ozon, Dichlormethan, -60°C, 30 min; 2. Dimethylsulfid]. 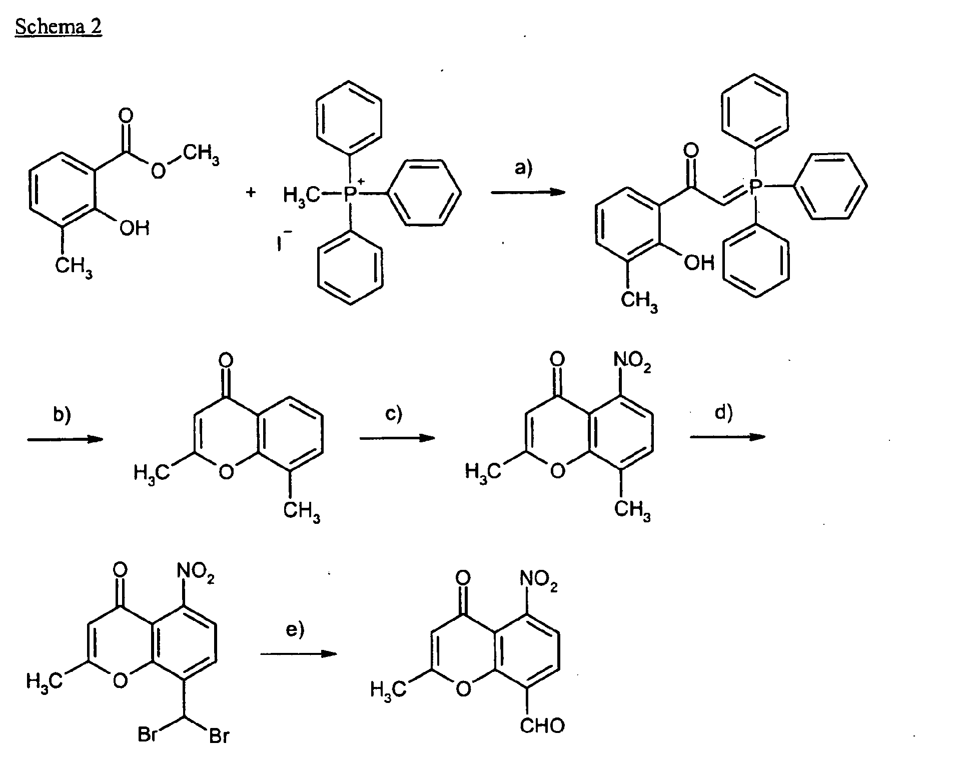 [a): n-Butyllithium, THF, 60°C, 3 h; b): Essigsäureanhydrid, Pyridin, Rückfluss, 6 h; c): konz. H₂SO₄, HNO₃, 0°C, 1 h; d): *N-*Bromsuccinimid, AIBN, Tetrachlorkohlenstoff, Rückfluss; e): *N-*Methylmorpholin-*N-*oxid, Acetonitril, Rückfluss]. 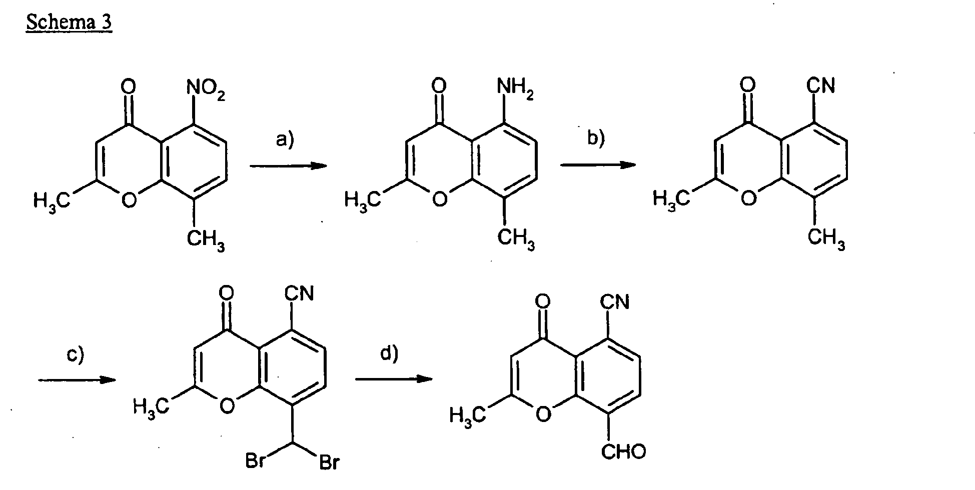 [a): Zinn(II)chlorid-Dihydrat, Ethylacetat, 70°C; b): 1. Natriumnitrit, Schwefelsäure, 0°C, 1.5 h; 2. Kupfer(I)cyanid, Natriumcyanid, Wasser/Ethylacetat, 0°C, 45 min; c): *N-*Bromsuccinimid, AIBN, Tetrachlorkohlenstoff, Rückfluss; d): *N-*Methylmorpholin-*N-*oxid, Acetonitril, Rückfluss]. 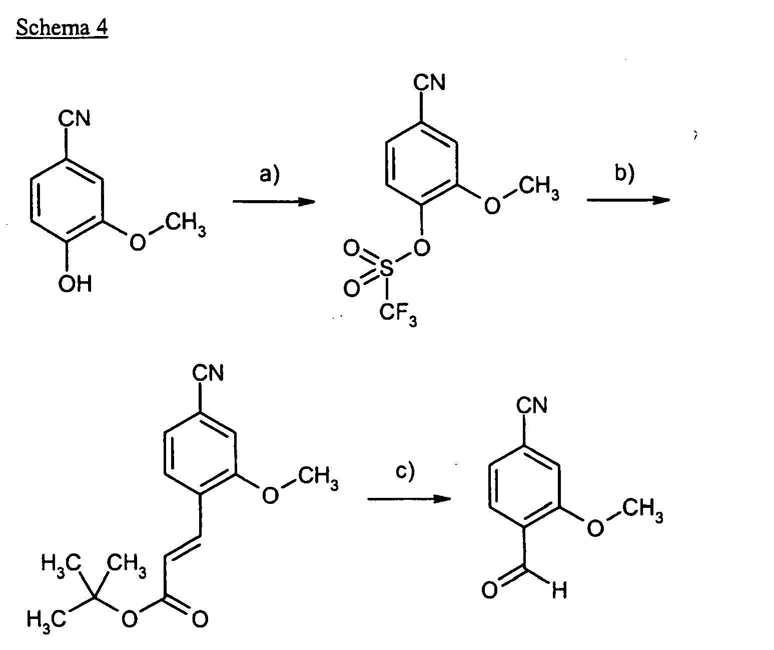 [a): Trifluormethansulfonsäureanhydrid, Pyridin, 0°C → RT, 30 min; b): Acrylsäure-*tert.*-butylester, Bis(triphenylphosphin)dichlorpalladium(II), DMF, 120°C, 24 h; c): cat. Osmiumtetroxid, cat. Benzyltriethylammoniumchlorid, Natriumperiodat, THF/Wasser, 20-25°C, 2 h]. 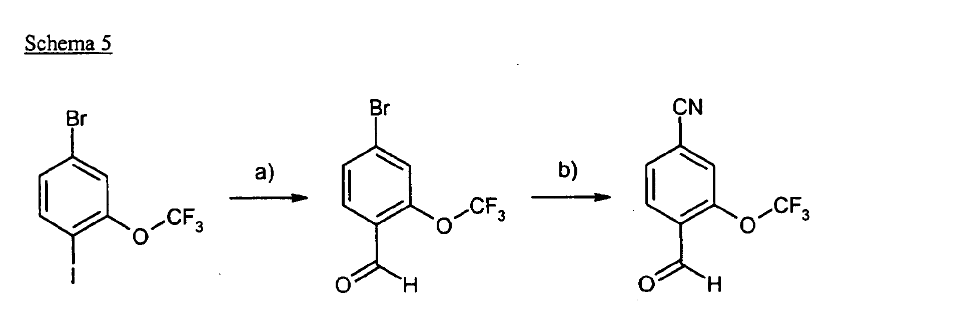 [a): n-Butyllithium, THF, -78°C, dann *N-*Formylmorpholin; b): Zinkcyanid, Tetrakis(triphenylphosphin)palladium(0), DMF, Mikrowelle 250°C / 5 min]. 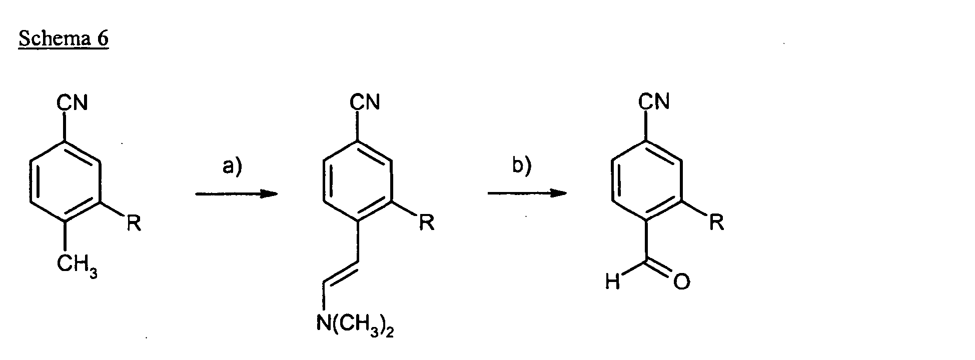 [a): *N,N*-Dimethylformamid-dimethylacetal, DMF, 140-180°C; b): Natriumperiodat, THF/Wasser]. 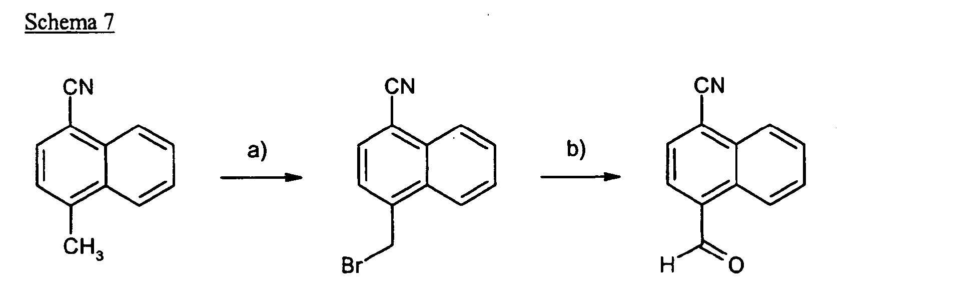 [a): *N-*Bromsuccinimid, AIBN, Tetrachlormethan, Rückfluss; b): *N-*Methylmorpholin-*N-*oxid, Acetonitril, 3Å-Molekularsieb]. 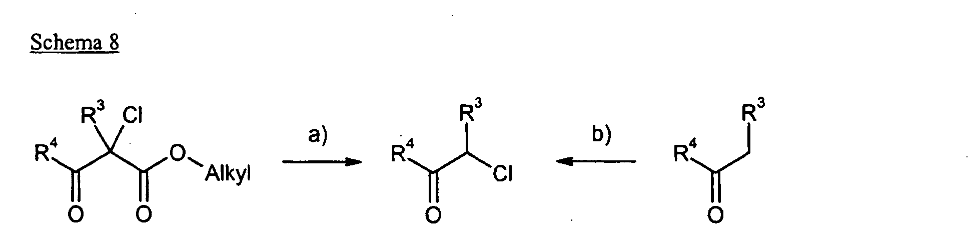 [a): Wasser, Schwefelsäure, 80°C; b): für Verbindungen mit R⁴ = H: Prolinamid, *N-*Chlorsuccinimid, Dichlormethan, RT; vgl. N. Halland et al., J. Am. Chem. Soc. 126, 4790-4791 (2004)]. 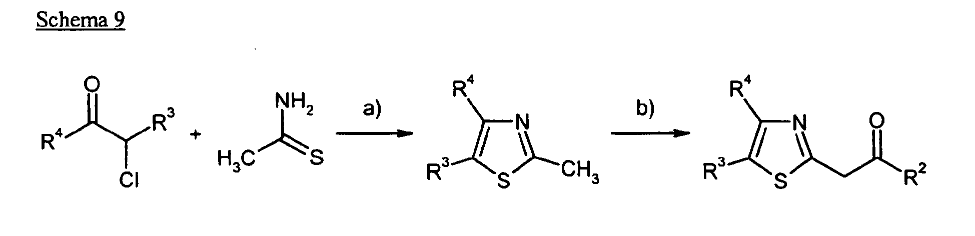 [a): Benzol oder Ethanol, optional Triethylamin, Rückfluss; b): 1. n-Butyllithium, THF, -78°C; 2. R²-CO-OAlkyl, THF, -78°C → RT]. 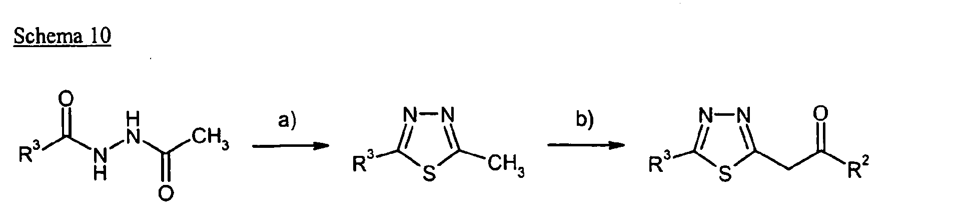 [a): P₂S₅; vgl. H. Lund, Acta Chem. Scand. 27, 391-395 (1973); b): 1. Natriumhydrid, Toluol, 50°C; 2. R²-CO-OAlkyl, THF, Rückfluss]. 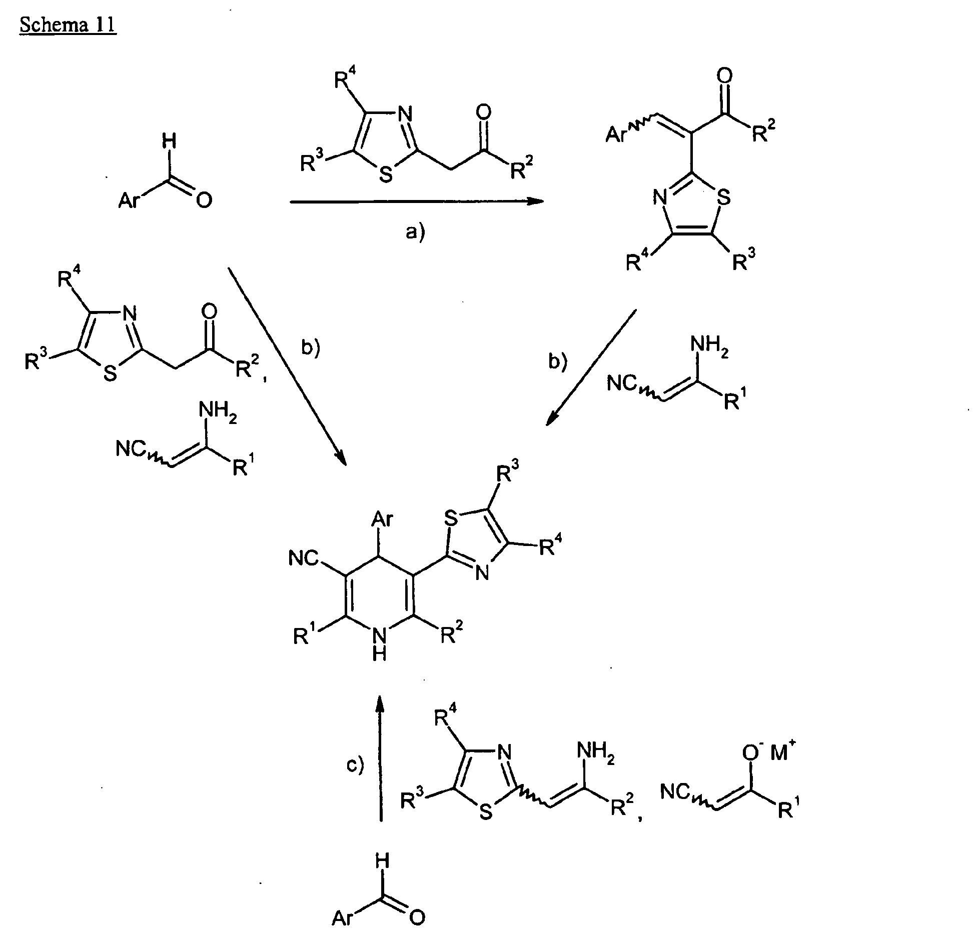 [a): cat. Piperidin / Essigsäure, Dichlormethan, Rückfluss, 24 h; b): Isopropanol, optional Kalium-*tert.*-butylat, Rückfluss, 12 h; c): Isopropanol, Essigsäure, Rückfluss, 12 h (M⁺ = Li⁺, Na⁺ oder K⁺)].

Die erfindungsgemäßen Verbindungen wirken als Antagonisten des Mineralokorticoid-Rezeptors und zeigen ein nicht vorhersehbares, wertvolles pharmakologisches Wirkspektrum. Sie eignen sich daher zur Verwendung als Arzneimittel zur Behandlung und/oder Prophylaxe von Krankheiten bei Menschen und Tieren.

Die erfindungsgemäßen Verbindungen sind geeignet für die Prophylaxe und/oder Behandlung von verschiedenen Erkrankungen und krankheitsbedingten Zuständen, insbesondere von Erkrankungen, die entweder durch eine Erhöhung der Aldosteron-Konzentration im Plasma oder durch eine Veränderung der Aldosteron-Plasmakonzentration relativ zur Renin-Plasmakonzentration gekennzeichnet sind oder mit diesen Veränderungen einhergehen. Beispielsweise seien genannt: idiopathischer primärer Hyperaldosteronismus, Hyperaldosteronismus bei Nebennierenhyperplasie, Nebennierenadenomen und/oder Nebennierencarzinomen, Hyperaldosteronismus bei Leberzirrhose, Hyperaldosteronismus bei Herzinsuffizienz sowie (relativer) Hyperaldosteronismus bei essentieller Hypertonie.

Die erfindungsgemäßen Verbindungen sind aufgrund ihres Wirkmechanismus ferner geeignet für die Prophylaxe des plötzlichen Herztodes bei Patienten, die unter einem erhöhten Risiko stehen, an einem plötzlichen Herztod zu versterben. Dies sind insbesondere Patienten, die z.B. an einer der folgenden Erkrankungen leiden: Hypertonie, Herzinsuffizienz, koronare Herzerkrankung, stabile und instabile Angina pectoris, myokardiale Ischämie, Myokardinfarkt, dilatative Kardiomyopathien, Schock, Arteriosklerose, atriale und ventrikuläre Arrhythmie, transitorische und ischämische Attacken, Hirnschlag, entzündliche kardiovaskuläre Erkrankungen, periphere und kardiale Gefäßerkrankungen, periphere Durchblutungsstörungen, pulmonale Hypertonie, Spasmen der Koronararterien und peripherer Arterien, Thrombosen, thromboembolische Erkrankungen sowie Vaskulitis.

Die erfindungsgemäßen Verbindungen können ferner verwendet werden für die Prophylaxe und/ oder Behandlung von Ödembildung wie zum Beispiel pulmonales Ödem, renales Ödem oder Herzinsuffizienz-bedingtes Ödem, und von Restenosen wie nach Thrombolysetherapien, percutantransluminalen Angioplastien (PTA) und transluminalen Koronarangioplastien (PTCA), Herztransplantationen und Bypass-Operationen.

Weiterhin eignen sich die erfindungsgemäßen Verbindungen zur Verwendung als Diuretikum und bei Elektrolytstörungen wie zum Beispiel Hyperkalzämie.

Außerdem können die erfindungsgemäßen Verbindungen eingesetzt werden für die Prophylaxe und/oder Behandlung von Diabetes mellitus und diabetischen Folgeerkrankungen wie z.B. Neuropathie und Nephropathie, von akutem und chronischem Nierenversagen sowie der chronischen Niereninsuffizienz.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker und Rho-Kinase-Inhibitoren;
- Diuretika, insbesondere Schleifendiuretika sowie Thiazide und Thiazid-ähnliche Diuretika;
- antithrombotisch wirkende Mittel, beispielhaft und vorzugsweise aus der Gruppe der Thrombozytenaggregationshemmer, der Antikoagulantien oder der profibrinolytischen Substanzen;
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten;
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- positiv-inotrop wirksame Verbindungen, wie beispielsweise Herzglycoside (Digoxin), betaadrenerge und dopaminerge Agonisten wie Isoproterenol, Adrenalin, Noradrenalin, Dopamin und Dobutamin;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil, sowie PDE 3-Inhibitoren wie Amrinone und Milrinone;
- natriuretische Peptide, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- Calcium-Sensitizer, wie beispielhaft und vorzugsweise Levosimendan;
- Kalium-Supplements;
- NO-unabhängige, jedoch Häm-abhängige Stimulatoren der Guanylatcyclase, wie insbesondere die in WO 00/06568, WO 00/06569, WO 02/42301 und WO 03/095451 beschriebenen Verbindungen;
- NO- und Häm-unabhängige Aktivatoren der Guanylatcyclase, wie insbesondere die in WO 01/19355, WO 01/19776, WO 01/19778, WO 01/19780, WO 02/070462 und WO 02/070510 beschriebenen Verbindungen;
- Inhibitoren der humanen neutrophilen Elastase (HNE), wie beispielsweise Sivelestat oder DX-890 (Reltran);
- die Signaltransduktionskaskade inhibierende Verbindungen, wie beispielsweise Tyrosinkinase-Inhibitoren, insbesondere Sorafenib, Imatinib, Gefitinib und Erlotinib; und/oder
- den Energiestoffwechsel des Herzens beeinflussende Verbindungen, wie beispielhaft und vorzugsweise Etomoxir, Dichloracetat, Ranolazine oder Trimetazidine.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Rho-Kinase-Inhibitoren sowie der Diuretika verstanden.

Bei einer bevorzugten Ausfiihrungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embusartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Rho-Kinase-Inhibitor, wie beispielhaft und vorzugsweise Fasudil, Y-27632, SLx-2119, BF-66851, BF-66852, BF-66853, KI-23095 oder BA-1049, verabreicht.

Unter antithrombotisch wirkenden Mitteln (Antithrombotika) werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705, BAY 60-5521, BAY 78-7499 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW-501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nicht-toxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- abs.: absolut
- AIBN: 2,2'-Azobis-2-methylpropannitril
- cat.: katalytisch
- CI: chemische Ionisation (bei MS)
- d: Tag(e)
- DC: Dünnschichtchromatographie
- DMF: Dimethylformamid
- DMSO: Dimethylsulfoxid
- d. Th.: der Theorie (bei Ausbeute)
- ee: Enantiomerenüberschuss
- EI: Elektronenstoß-Ionisation (bei MS)
- ent: Enantiomer / enantiomerenrein
- eq: Äquivalent(e)
- ESI: Elektrospray-Ionisation (bei MS)
- GC-MS: Gaschromatographie-gekoppelte Massenspektrometrie
- h: Stunde(n)
- HPLC: Hochdruck-, Hochleistungsflüssigchromatographie
- konz.: konzentriert
- LC-MS: Flüssigchromatographie-gekoppelte Massenspektrometrie
- min: Minute(n)
- MPLC: Mitteldruckflüssigchromatographie
- MS: Massenspektrometrie
- NMR: Kernresonanzspektrometrie
- R_{f}: Retentionsindex (bei DC)
- Rₜ: Retentionszeit (bei HPLC)
- RT: Raumtemperatur
- THF: Tetrahydrofuran
- v/v: Volumen-zu-Volumen-Verhältnis (einer Lösung)

### LC-MS-, GC-MS- und HPLC-Methoden:

### Methode 1 (LC-MS):

Gerätetyp MS: Micromass ZQ; Gerätetyp HPLC: HP 1100 Series; UV DAD; Säule: Phenomenex Gemini 3µ 30 mm x 3.00 mm; Eluent A: 1 l Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 2 (GC-MS):

Instrument: Micromass GCT, GC 6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Methode 3 (LC-MS):

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 l Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 4 (LC-MS):

Instrument: Micromass Quattro LCZ mit HPLC Agilent Serie 1100; Säule: Phenomenex Onyx Monolithic C 18, 100 mm x 3 mm; Eluent A: 1 l Wasser + 0.5 ml 50%-ige Ameisensäure, Eluent B: 11 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 208-400 nm.

### Methode 5 (LC-MS):

Instrument: Micromass Quattro LCZ mit HPLC Agilent Serie 1100; Säule: Phenomenex Synergi 2µ Hydro-RP Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 6 (GC-MS):

Instrument: Micromass GCT, GC 6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (12 min halten).

### Methode 7 (LC-MS):

Instrument: Micromass Platform LCZ mit HPLC Agilent Serie 1100; Säule: Thermo Hypersil GOLD 3µ 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### Methode 8 (LC-MS):

Gerätetyp MS: Waters ZQ; Gerätetyp HPLC: Waters Alliance 2795; Säule: Merck Chromolith RP18e, 100 mm x 3 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 210 nm.

### Ausgangsverbindungen und Intermediate:

### Beispiel 1A

### 4-Cyano-2-methoxyphenyl-trifluormethansulfonat

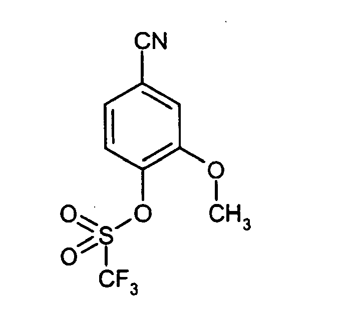

Zu einer Lösung von 20 g (134 mmol) 4-Hydroxy-3-methoxybenzonitril in Pyridin (80 ml) werden langsam 24 ml (141 mmol) Trifluormethansulfonsäureanhydrid getropft, wobei die Reaktionstemperatur mit Hilfe eines Eisbads unter 25°C gehalten wird. Die Suspension wird dann 1 h bei RT gerührt. Eiswasser (400 ml) wird zugegeben und die Suspension noch bis zum Erreichen der Raumtemperatur weiter gerührt. Dann wird filtriert, der Feststoff in Ethylacetat gelöst und diese Lösung mit gesättigter Natriumchlorid-Lösung gewaschen. Die organische Phase wird über Magnesiumsulfat getrocknet und eingeengt. Es werden 37.13 g (92% d. Th.) der Titelverbindung als weißer Feststoff erhalten.
LC-MS (Methode 1): Rₜ = 2.54 min; MS (EIpos): m/z = 282 [M+H]⁺
¹H-NMR (300 MHz, DMSO-d₆): δ = 3.97 (s, 3H), 7.60 (dd, 1H), 7.71 (d, 1H), 7.92 (d, 1H).

### Beispiel 2A

### tert.-Butyl (2E)-3-(4-cyano-2-methoxyphenyl)acrylat

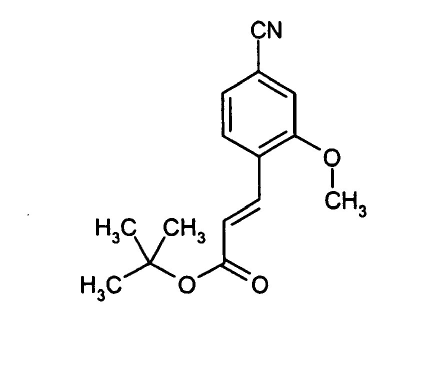

Zu einer entgasten Lösung von 37.13 g (132 mmol) 4-Cyano-2-methoxyphenyl-trifluormethansulfonat, 35 ml (245 mmol) *tert.*-Butylacrylat und 90 ml (645 mmol) Triethylamin in DMF (250 ml) werden 4 g (5.7 mmol) Bis(triphenylphosphin)palladium(II)chlorid hinzugefügt. Die Lösung wird unter Schutzgasatmosphäre 24 h lang bei 100°C gerührt. Anschließend wird Eiswasser (1000 ml) zugegeben und die Suspension mit Ethylacetat (3 x 100 ml) extrahiert. Die organische Phase wird mit gesättigter Natriumchlorid-Lösung gewaschen, über Magnesiumsulfat getrocknet und eingeengt. Der Rückstand wird säulenchromatographisch gereinigt (Kieselgel, Laufmittel: Cyclohexan-Essigsäureethylester 10:1). Es werden 24.6 g (72% d. Th.) der Titelverbindung als weißer Feststoff erhalten.
LC-MS (Methode 3): Rₜ = 2.59 min; MS (EIpos): m/z = 260 [M+H]⁺
¹H-NMR (300 MHz, DMSO-d₆): δ = 1.48 (s, 9H), 3.93 (s, 3H), 6.65 (d, 1H), 7.42 (d, 1H), 7.58 (s, 1H), 7.74 (d, 1H), 7.89 (d, 1H).

### Beispiel 3A

### 4-Formyl-3-methoxybenzonitril

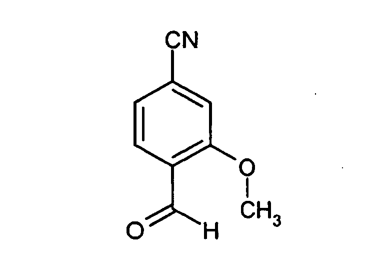

Zu einer kräftig gerührten Lösung von 48 g (185 mmol) *tert.*-Butyl (2*E*)-3-(4-cyano-2-methoxyphenyl)acrylat, 207 mg (0.81 mmol) Osmiumtetroxid und 1.4 g (6.14 mmol) Benzyltriethylammoniumchlorid in 750 ml Wasser/THF (2:1) werden 79 g (370 mmol) Natriummetaperiodat portionsweise zugegeben, wobei die Reaktionstemperatur unter 30°C gehalten wird. Die Lösung wird 1 h bei RT weitergerührt. Wasser (2000 ml) wird zugegeben und die Mischung anschließend filtriert. Der verbleibende Feststoff wird in Ethylacetat gelöst und die Lösung mit gesättigter Natriumchlorid-Lösung gewaschen. Die organische Phase wird über Magnesiumsulfat getrocknet und eingeengt. Der Rückstand wird mit Petrolether verrührt. Es werden 21.18 g (71% d. Th.) der Titelverbindung als weißer Feststoff erhalten.
LC-MS (Methode 1): Rₜ = 1.87 min; MS (EIpos): m/z = 162 [M+H]⁺
¹H-NMR (300 MHz, DMSO-d₆): δ = 3.98 (s, 3H), 7.53 (d, 1H), 7.80 (s, 1H), 7.81 (d, 1H), 10.37 (s, 1H).

### Beispiel 4A

### 1-(4-Methyl-1,3-thiazol-2-yl)aceton

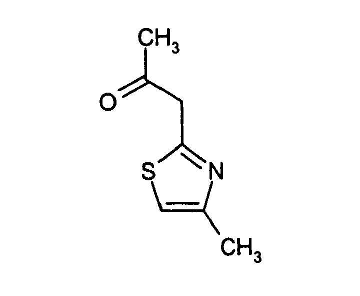

5.00 g (44.2 mmol) 2,4-Dimethylthiazol werden in 50 ml THF gelöst und bei -78°C tropfenweise mit 19.4 ml (48.6 mmol) n-Butyllithium (2.5 M Lösung in Hexan) versetzt. Nach zweistündigem Rühren bei -78°C werden 6.62 g (75.1 mmol) Essigsäureethylester als Lösung in 25 ml abs. THF addiert. Es wird 1 h bei -78°C nachgerührt und anschließend auf Raumtemperatur erwärmt. Danach wird mit Natriumhydrogencarbonat-Lösung hydrolysiert und das Gemisch dreimal mit Diethylether extrahiert. Die vereinigten organischen Phasen werden mit Magnesiumsulfat getrocknet und das Lösungsmittel am Rotationsverdampfer entfernt. Die Aufreinigung des Rohproduktes erfolgt durch Säulenchromatographie (Kieselgel, Laufmittel: Cyclohexan/Ethylacetat 5:1). Es werden 3.2 g (47% d. Th.) der Titelverbindung erhalten.
GC-MS (Methode 2): Rₜ = 3.87 min; MS (EIpos): m/z = 155 [M]⁺
¹H-NMR (300 MHz, CDCl₃): δ [ppm] = 2.27 (s, 3H), 2.44 (s, 3H), 4.10 (s, 2H), 6.84 (s, 1H).

### Beispiel 5A

### 1-(4-Methyl-1,3-thiazol-2-yl)butan-2-on

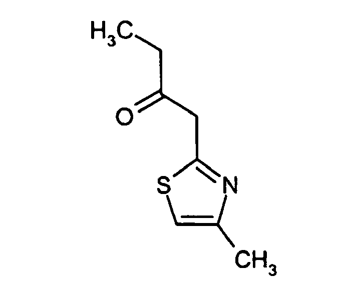

3.00 g (26.5 mmol) 2,4-Dimethylthiazol werden in 30 ml THF gelöst und bei -78°C tropfenweise mit 11.6 ml (29.2 mmol) n-Butyllithium (2.5 M Lösung in Hexan) versetzt. Nach zweistündigem Rühren bei -78°C werden 4.60 g (45.1 mmol) Propionsäureethylester als Lösung in 15 ml abs. THF addiert. Es wird 1 h bei -78°C nachgerührt und anschließend auf Raumtemperatur erwärmt. Danach wird mit Natriumhydrogencarbonat-Lösung hydrolysiert und das Gemisch dreimal mit Diethylether extrahiert. Die vereinigten organischen Phasen werden mit Magnesiumsulfat getrocknet und das Lösungsmittel am Rotationsverdampfer entfernt. Es werden 3.5 g (39% d. Th.) der Titelverbindung in 50%-iger Reinheit (GC-MS) erhalten, welche ohne weitere Aufreinigung eingesetzt werden.
GC-MS (Methode 2): Rₜ = 4.30 min; MS (EIpos): m/z = 169 [M]⁺.

### Beispiel 6A

### 3-Methoxy-4-[2-(4-methyl-1,3-thiazol-2-yl)-3-oxobut-1-en-1-yl]benzonitril

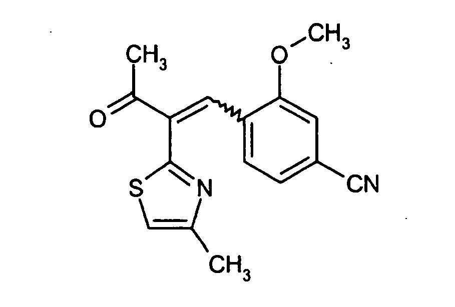

400 mg (2.48 mmol) der Verbindung aus Beispiel 3A und 424 mg (2.73 mmol) der Verbindung aus Beispiel 4A werden in 10 ml Dichlormethan gelöst und mit 0.245 ml (2.48 mmol) Piperidin sowie 0.142 ml (2.48 mmol) Essigsäure versetzt. Die Reaktionsmischung wird über Nacht am inversen Wasserabscheider unter Rückfluss erhitzt. Die flüchtigen Komponenten werden danach am Rotationsverdampfer abgetrennt und das Rohprodukt mittels präparativer HPLC aufgereinigt (Eluent: Acetonitril/Wasser mit 0.1% Ameisensäure, Gradient 20:80 → 95:5). Es werden 390 mg (53% d. Th.) der Titelverbindung als Gemisch der *E*/*Z*-Isomere erhalten.
LC-MS (Methode 4): Rₜ = 3.13 min und 3.52 min; MS (EIpos): m/z jeweils = 299 [M+H]⁺.

### Beispiel 7A

### 1-[2-(Allyloxy)phenyl]ethanon

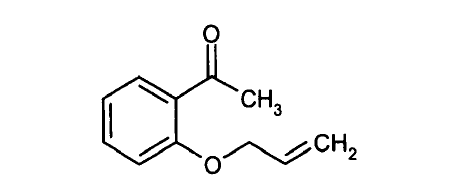

542 g (3.9 mol) 2-Hydroxyacetophenon werden mit 592 g (4.9 mol) Allylbromid, 1000 g (7.2 mol) Kaliumcarbonat und 13.2 g (79 mmol) Kaliumiodid in 2.4 Liter Aceton 24 h lang zum Rückfluss erhitzt. Nach Abkühlen auf Raumtemperatur wird filtriert und das Lösungsmittel im Vakuum entfernt. Der Rückstand wird in Toluol gelöst und mit 10%-iger Natronlauge und Wasser gewaschen. Nach Einengen werden 689 g (98% d. Th.) der Titelverbindung erhalten.
¹H-NMR (300 MHz, CDCl₃): δ = 2.68 (s, 3H), 4.68 (dd, 2H), 5.89 (dd, 2H), 6.09 (m, 1H), 6.99 (dd, 2H), 7.44 (m, 1H), 7.71 (d, 1H).

### Beispiel 8A

### 1-(3-Allyl-2-hydroxyphenyl)ethanon

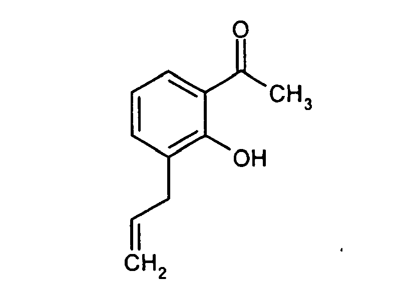

160 g (0.9 mol) 1-(2-(Allyloxy)phenyl]ethanon werden im Metallbad 4 h lang bei 230-240°C gerührt. Nach Abkühlen auf Raumtemperatur wird das Produkt über einen Dünnschichtverdampfer bei 140°C und 0.4 mbar destilliert. Es werden 155 g (97% d. Th.) der Titelverbindung erhalten.
¹H-NMR (300 MHz, CDCl₃): δ = 2.68 (s, 3H), 3.44 (d, 2H), 5.09 (m, 2H), 6.01 (m, 1H), 6.85 (t, 1H), 7.38 (dd, 1H), 7.62 (dd, 1H), 12.61 (s, 1H).

### Beispiel 9A

### 1-{2-Hydroxy-3-[(1E)-prop-1-en-1-yl]phenyl}ethanon

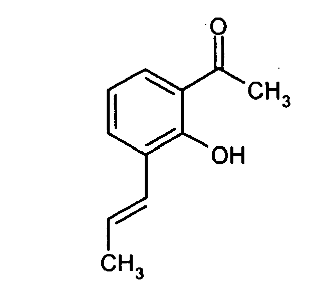

40 g (227 mmol) 1-(3-Allyl-2-hydroxyphenyl)ethanon werden in 120 ml Toluol gelöst und mit 2.17 g (5.6 mmol) Bis(benzonitril)dichlorpalladium(II) versetzt. Die Reaktionsmischung wird über Nacht auf 120°C erhitzt. Nach Abkühlen auf Raumtemperatur wird über Kieselgur filtriert und das Lösungsmittel im Vakuum entfernt. Es werden 20.9 g (95% d.Th.) der Titelverbindung erhalten, welche ohne weitere Reinigung in der nächsten Stufe umgesetzt wird.
LC-MS (Methode 3): Rₜ = 2.36 min; m/z = 177 [M+H]⁺
¹H-NMR (300 MHz, CDCl₃): δ = 1.91 (dd, 3H), 2.63 (s, 3H), 6.32 (m, 1H), 6.73 (dd, 1H), 6.85 (t, 1H), 7.59 (m, 2H), 12.74 (s, 1H).

### Beispiel 10A

### 2-Methyl-8-[(1E)-prop-1-en-1-yl]-4H-chromen-4-on

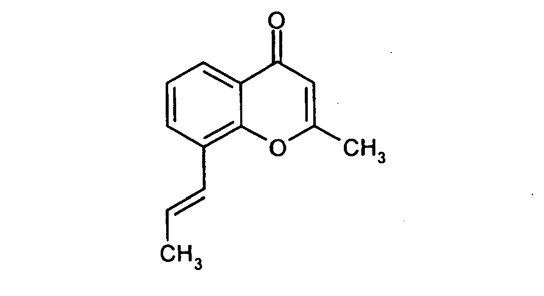

12.52 g (313.2 mmol) 60%-iges Natriumhydrid (Suspension in Mineralöl) werden unter Argon bei 10°C in 300 ml absolutem THF vorgelegt. Zu der Suspension werden 18.4 g (104.4 mmol) 1-{2-Hydroxy-3-[(1*E*)-prop-1-en-1-yl]phenyl}ethanon langsam zugetropft. Nach 15 min werden 9 g (114.9 mmol) Acetylchlorid zugegeben. Die Reaktionsmischung wird über Nacht bei Raumtemperatur gerührt. Man hydrolysiert mit 300 ml Wasser und extrahiert mehrfach mit Ethylacetat. Nach Waschen der organischen Phase mit gesättigter Natriumchlorid-Lösung wird über Natriumsulfat getrocknet. Anschließend wird das Lösungsmittel im Vakuum entfernt. Der Rückstand wird in 200 ml Methanol aufgenommen und mit 50 ml 20%-iger Salzsäure 30 min auf 80°C erhitzt.
Anschließend wird das Lösungsmittel im Vakuum entfernt und der Rückstand mit 400 ml Wasser versetzt. Es wird mehrfach mit Dichlormethan extrahiert. Nach Trocknen der organischen Phase über Magnesiumsulfat wird das Lösungsmittel im Vakuum entfernt und der Rückstand mittels Säulenchromatographie gereinigt (Laufinittel: Dichlormethan/Methanol 98:2). Es werden 10.5 g (50.2% d. Th.) der Titelverbindung als gelbes Öl erhalten.
LC-MS (Methode 5): Rₜ = 2.07 min; m/z = 201 [M+H]⁺
¹H-NMR (300 MHz, CDCl₃): δ = 1.98 (dd, 3H), 2.43 (s, 3H), 6.18 (s, 1H), 6.40 (m, 1H), 6.85 (dd, 1H), 7.31 (t, 1H), 7.72 (dd, 1H), 8.05 (dd, 1H).

### Beispiel 11A

### 2-Methyl-4-oxo-4H-chromen-8-carbaldehyd

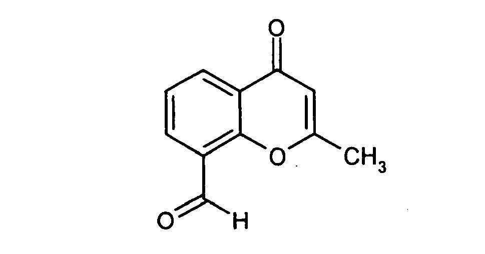

18.5 g (62.8 mmol) 2-Methyl-8-[(1*E*)-prop-1-en-1-yl]-4*H*-chromen-4-on werden in 400 ml Dichlormethan gelöst und auf -60°C abgekühlt. In die Reaktionslösung wird 30 min lang Ozon eingeleitet. Anschließend wird die Reaktionsmischung mit Dimethylsulfid versetzt. Nach Erwärmen auf Raumtemperatur wird das Lösungsmittel im Vakuum entfernt und der Rückstand mit wenig Methanol aufgeschlämmt. Nach Filtration wird der verbleibende Feststoff aus Diethylether umkristallisiert. Es werden 9.1 g (77.4% d. Th.) der Titelverbindung erhalten.
LC-MS (Methode 5): Rₜ = 1.47 min; MS (EIpos): m/z = 189 [M+H]⁺
¹H-NMR (300 MHz, CDCl₃): δ = 2.48 (s, 3H), 6.27 (s, 1H), 7.51 (m, 1H), 8.21 (dd, 1H), 8.46 (dd, 1H), 10.67 (s, 1H).

### Beispiel 12A

### 1-(4-Methyl-1,3-thiazol-2-yl)pentan-2-on

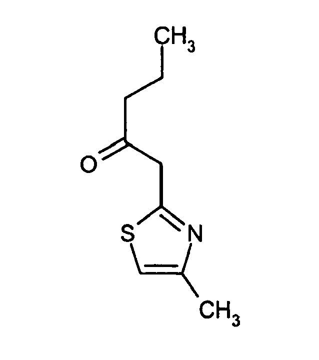

3.00 g (26.5 mmol) 2,4-Dimethylthiazol werden in 30 ml abs. THF gelöst und bei -78°C tropfenweise mit 11.7 ml (29.2 mmol) n-Butyllithium (2.5 M Lösung in Hexan) versetzt. Nach zweistündigem Rühren bei -78°C werden 5.23 g (45.1 mmol) Buttersäureethylester als Lösung in 15 ml abs. THF addiert. Es wird 1 h bei -78°C nachgerührt und anschließend auf Raumtemperatur erwärmt. Danach wird mit Natriumhydrogencarbonat-Lösung hydrolysiert und das Gemisch dreimal mit Diethylether extrahiert. Die vereinigten organischen Phasen werden mit Magnesiumsulfat getrocknet und das Lösungsmittel am Rotationsverdampfer entfernt. Die Aufreinigung des Rohproduktes erfolgt durch Säulenchromatographie (Kieselgel, Laufmittel: Cyclohexan/Ethylacetat 5:1). Es werden 3.0 g (62% d. Th.) der Titelverbindung erhalten.
GC-MS (Methode 6): Rₜ = 4.63 min; MS (EIpos): m/z = 183 [M]⁺.

### Beispiel 13A

### 1-Methoxy-3-(4-methyl-1,3-thiazol-2-yl)aceton

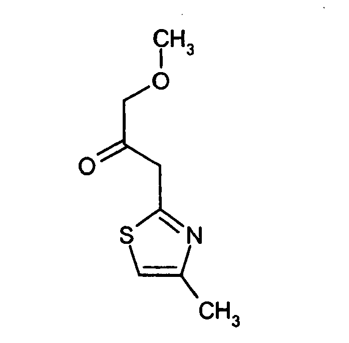

3.00 g (26.5 mmol) 2,4-Dimethylthiazol werden in 30 ml abs. THF gelöst und bei -78°C tropfenweise mit 11.7 ml (29.2 mmol) n-Butyllithium (2.5 M Lösung in Hexan) versetzt. Nach zweistündigem Rühren bei -78°C werden 5.32 g (45.1 mmol) Methoxyessigsäureethylester als Lösung in 15 ml abs. THF addiert. Es wird 1 h bei -78°C nachgerührt und dann auf Raumtemperatur erwärmt. Anschließend wird mit Natriumhydrogencarbonat-Lösung hydrolysiert und das Gemisch dreimal mit Diethylether extrahiert. Die vereinigten organischen Phasen werden mit Magnesiumsulfat getrocknet und das Lösungsmittel am Rotationsverdampfer entfernt. Es werden 4.2 g (68% d. Th.) der Titelverbindung in 80%-iger Reinheit (GC-MS) erhalten, welche ohne weitere Auf reinigung eingesetzt werden.
GC-MS (Methode 6): Rₜ = 4.86 min; MS (EIpos): m/z = 185 [M]⁺.

### Beispiel 14A

### 3-Methoxy-4-[4-methoxy-2-(4-methyl-1,3-thiazol-2-yl)-3-oxobut-1-en-1-yl]benzonitril

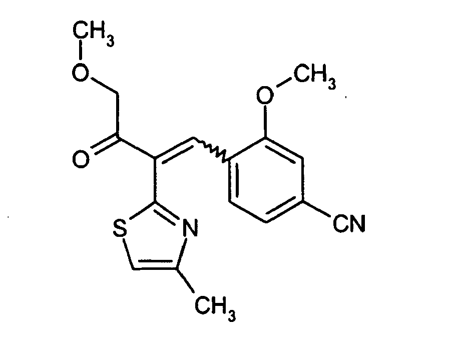

500 mg (3.10 mmol) der Verbindung aus Beispiel 3A und 632 mg (3.41 mmol) der Verbindung aus Beispiel 13A werden in 15 ml Dichlormethan gelöst und mit 0.307 ml (3.10 mmol) Piperidin sowie 0.178 ml (3.10 mmol) Essigsäure versetzt. Die Reaktionsmischung wird über Nacht am inversen Wasserabscheider unter Rückfluss erhitzt. Die flüchtigen Komponenten werden am Rotationsverdampfer abgetrennt und das Rohprodukt mittels präparativer HPLC aufgereinigt (Eluent: Acetonitril/Wasser mit 0.1% Ameisensäure, Gradient 20:80 → 95:5). Es werden 200 mg (20% d. Th.) der Titelverbindung als Gemisch der *E*/*Z*-Isomere erhalten.
LC-MS (Methode 1): Rₜ = 2.31 und 2.39 min; MS (EIpos): m/z jeweils = 329 [M+H]⁺.

### Beispiel 15A

### 1-Chlorbutan-2-on

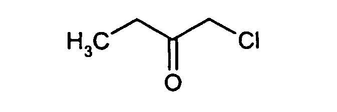

40.0 g (243 mmol) Methyl 2-chlor-3-oxopentanoat und 65 ml konz. Schwefelsäure werden in 120 ml Wasser gelöst und über Nacht bei 80°C temperiert. Nach dem Abkühlen werden 300 ml Wasser zugesetzt und das Gemisch dreimal mit Dichlormethan extrahiert. Die vereinigten organischen Phasen werden über Magnesiumsulfat getrocknet und am Rotationsverdampfer eingeengt. Der Rückstand wird anschließend fraktioniert bei Normaldruck destilliert. Dabei liefert die Fraktion im Siedebereich von 138-140°C 10.5 g (40% d. Th.) der Titelverbindung.
GC-MS (Methode 2): Rₜ = 1.53 min; MS (EIpos): m/z = 106 [M]⁺
¹H-NMR (300 MHz, DMSO-d₆): δ = 0.96 (t, 3H), 2.48-2.55 (q, 2H), 4.49 (s, 2H).

### Beispiel 16A

### 4-Ethyl-2-methyl-1,3-thiazol

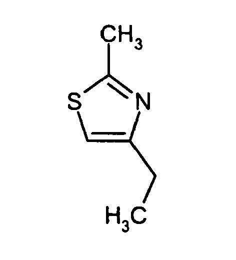

3.00 g (28.2 mmol) der Verbindung aus Beispiel 15A und 2.12 g (28.2 mmol) Thioacetamid werden in 25 ml Benzol über Nacht am Wasserabscheider unter Rückfluss erhitzt. Nach dem Abkühlen werden 4.32 ml (30.97 mmol) Triethylamin addiert. Der Ansatz wird 20 min gerührt und das ausgefallene Salz per Filtration abgetrennt. Anschließend wird das Lösungsmittel am Rotationsverdampfer entfernt. Es werden 1.73 g (48% d. Th.) der Titelverbindung erhalten.
GC-MS (Methode 2): Rₜ = 2.34 min; MS (EIpos): m/z = 127 [M]⁺
¹H-NMR (300 MHz, CDCl₃): δ = 1.28 (t, 3H), 2.68 (s, 3H), 2.77 (q, 2H), 6.69 (s, 1H).

### Beispiel 17A

### 1-(4-Ethyl-1,3-thiazol-2-yl)aceton

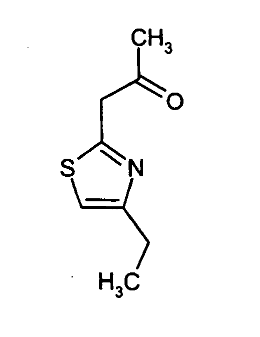

1.70 g (13.4 mmol) der Verbindung aus Beispiel 16A werden in 15 ml abs. THF gelöst und bei -78°C tropfenweise mit 5.88 ml (14.7 mmol) n-Butyllithium (2.5 M Lösung in Hexan) versetzt. Nach zweistündigem Rühren bei -78°C werden 2.00 g (22.7 mmol) Essigsäureethylester als Lösung in 10 ml abs. THF addiert. Es wird 1 h bei -78°C nachgerührt und anschließend auf Raumtemperatur erwärmt. Danach wird mit Natriumhydrogencarbonat-Lösung hydrolysiert und das Gemisch dreimal mit Diethylether extrahiert. Die vereinigten organischen Phasen werden mit Magnesiumsulfat getrocknet und das Lösungsmittel am Rotationsverdampfer entfernt. Es werden 1.89 g (84% d. Th.) der Titelverbindung erhalten.
GC-MS (Methode 2): Rₜ = 4.20 min; MS (EIpos): m/z = 169 [M]⁺.

### Beispiel 18A

### 2-Methyl-4,5,6,7-tetrahydro-1,3-benzothiazol

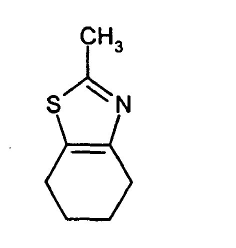

5.00 g (37.7 mmol) 2-Chlorcyclohexanon und 2.83 g (37.7 mmol) Thioacetamid werden in 25 ml Ethanol über Nacht auf Rückflusstemperatur erhitzt. Nach dem Abkühlen werden die flüchtigen Komponenten am Rotationsverdampfer abgetrennt und der verbleibende Rückstand in 2 N Natriumcarbonat-Lösung aufgenommen. Es wird dreimal mit Dichlormethan extrahiert und die vereinigten organischen Phasen mit Magnesiumsulfat getrocknet. Das Lösungsmittel wird am Rotationsverdampfer entfernt und der Rückstand anschließend säulenchromatographisch aufgereinigt (Kieselgel, Laufmittel: Cyclohexan/Ethylacetat 7:3). Es werden 3.35 g (58% d. Th.) der Titelverbindung erhalten.
LC-MS (Methode 7): Rₜ = 2.49 min; MS (EIpos): m/z = 154 [M+H]⁺
¹H-NMR (300 MHz, CDCl₃): δ = 1.84 (m, 4H), 2.63 (s, 3H), 2.67-2.77 (4H).

### Beispiel 19A

### 1-(4,5,6,7-Tetrahydro-1,3-benzothiazol-2-yl)aceton

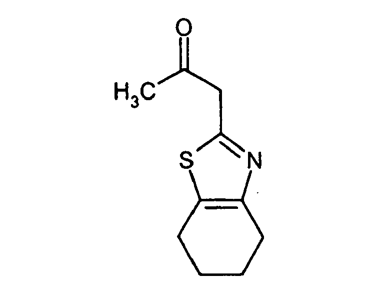

1.50 g (9.78 mmol) der Verbindung aus Beispiel 18A werden in 10 ml abs. THF gelöst und bei -78°C tropfenweise mit 4.31 ml (10.77 mmol) n-Butyllithium (2.5 M Lösung in Hexan) versetzt. Nach zweistündigem Rühren bei -78°C werden 1.46 g (16.6 mmol) Essigsäureethylester als Lösung in 5 ml abs. THF addiert. Es wird 1 h bei -78°C nachgerührt und dann auf Raumtemperatur erwärmt. Anschließend wird mit Natriumhydrogencarbonat-Lösung hydrolysiert und das Gemisch dreimal mit Diethylether extrahiert. Die vereinigten organischen Phasen werden mit Magnesiumsulfat getrocknet und das Lösungsmittel am Rotationsverdampfer entfernt. Es werden 1.60 g (67% d. Th.) der Titelverbindung in 80%-iger Reinheit (LC-MS) erhalten, welche ohne weitere Aufreinigung eingesetzt werden.
LC-MS (Methode 1): Rₜ = 1.73 min; MS (EIpos): m/z = 196 [M+H]⁺.

### Beispiel 20A

### 3-Methoxy-4-[2-(4-methyl-1,3-thiazol-2-yl)-3-oxopent-1-en-1-yl]benzonitril

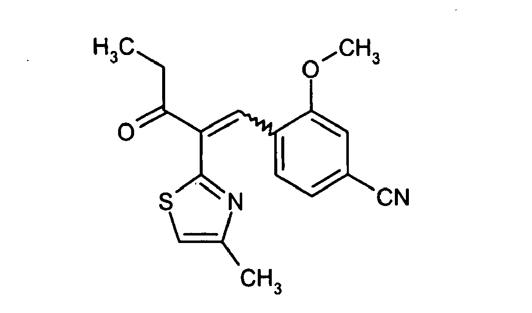

400 mg (2.48 mmol) der Verbindung aus Beispiel 3A und 924 mg (2.73 mmol, 50% Reinheit) der Verbindung aus Beispiel 5A werden in 10 ml Dichlormethan gelöst und mit 0.245 ml (2.48 mmol) Piperidin sowie 0.142 ml (2.48 mmol) Essigsäure versetzt. Die Reaktionsmischung wird über Nacht am inversen Wasserabscheider unter Rückfluss erhitzt. Die flüchtigen Komponenten werden am Rotationsverdampfer abgetrennt und das Rohprodukt mittels präparativer HPLC aufgereinigt (Eluent: Acetonitril/Wasser mit 0.1% Ameisensäure, Gradient 20:80 → 95:5). Es werden 657 mg (83% d. Th.) der Titelverbindung als Gemisch der *E*/*Z*-Isomere erhalten.
LC-MS (Methode 1): Rₜ = 2.36 und 2.56 min; MS (EIpos): m/z jeweils = 313 [M+H]⁺.

### Beispiel 21A

### 3-Methylchinolin-5-carbonsäure

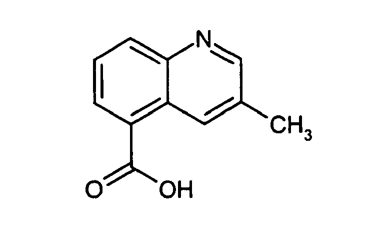

100.0 g (512 mmol) 3-Hydroxy-4-nitro-2-benzofuran-1(3*H*)-on [vgl. Watanabe et al., Chem. Pharm. Bull. 20, 2123-2126 (1972)] werden in 410 ml Ethanol gelöst und in einer Parr-Apparatur mit 10.3 g Palladium auf Kohle (5%-ig) bei einem Wasserstoff-Druck von 3 bar hydriert. Bei dieser sehr exothermen Reaktion wird die Innentemperatur über die Wasserstoffzufuhr kontrolliert; so wird bei Erreichen einer Innentemperatur von 75°C die Wasserstoffzufuhr unterbrochen. Nach Abklingen der Reaktion und Abfall der Innentemperatur auf ca. 40°C wird erneut 3 bar Wasserstoff aufgegeben. Die Prozedur wird wiederholt, bis kein Wasserstoffverbrauch mehr festzustellen ist. Die Gesamtdauer der Reaktion liegt bei ca. drei Stunden. Anschließend wird der Katalysator über Kieselgur abfiltriert. Die so erhaltene Lösung des korrespondierenden Amins [4-Amino-3-hydroxy-2-benzofuran-1(3*H*)-on] wird mit 29.7 g (512 mmol) Propionaldehyd versetzt und 3 Tage bei Rückflusstemperatur gerührt. Nach dem Abkühlen wird der ausgefallene Feststoff abgesaugt und mit Ethanol gewaschen. Nach dem Trocknen über Nacht werden 22.5 g (23% d. Th.) der Titelverbindung erhalten.
MS (EIpos): m/z = 188 [M+H]⁺.
¹H-NMR (300 MHz, DMSO-d₆): δ = 2.53 (s, 3H), 7.76 (dd, 1H), 8.18-8.26 (m, 2H), 8.84 (d, 1H), 9.05 (br. t, 1H), 13.27 (br. s, 1H).

### Beispiel 22A

### (3-Methylchinolin-5-yl)methanol

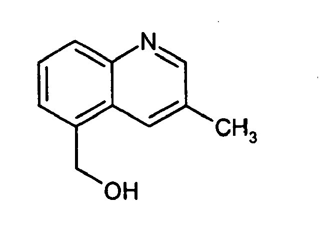

35.0 g (187 mmol) der Verbindung aus Beispiel 21A werden in 440 ml 1,2-Dimethoxyethan vorgelegt, mit 32.3 ml (233 mmol) Triethylamin versetzt und 30 min bei Raumtemperatur gerührt. Anschließend werden 22.3 ml (233 mmol) Chlorameisensäureethylester bei 15°C zugetropft und die Mischung 15 min bei Raumtemperatur nachgerührt. Der ausgefallene Feststoff wird abfiltriert und mit 1,2-Dimethoxyethan gewaschen. Anschließend wird das Filtrat eingeengt und der Rückstand in 440 ml Ethanol aufgenommen. Zu der so erhaltenen Lösung werden tropfenweise 13.1 g (345 mmol) Natriumborhydrid als Lösung in 210 ml Wasser zugetropft. Nach 16 h Reaktionszeit bei Raumtemperatur wird der ausgefallene Feststoff abgesaugt, mit Ethanol gewaschen und das Filtrat am Rotationsverdampfer eingeengt. Der erhaltene Rückstand wird in 800 ml Wasser und 600 ml Dichlormethan/Methanol (8:2) aufgenommen. Die wässrige Phase wird nochmals mit 600 ml Dichlormethan/Methanol (8:2) extrahiert und die vereinigten organischen Phasen mit gesättigter Natriumchlorid-Lösung gewaschen. Nach dem Einengen und Trocknen werden 23.0 g (71% d. Th.) der Titelverbindung erhalten.
¹H-NMR (300 MHz, DMSO-d₆): δ = 2.48 (s, 3H), 4.99 (s, 2H), 5.41 (br. s, 1H), 7.56-7.69 (m, 2H), 7.92 (dd, 1H), 8.29 (m, 1H), 8.77 (d, 1H).

### Beispiel 23A

### 3-Methylchinolin-5-carbaldehyd

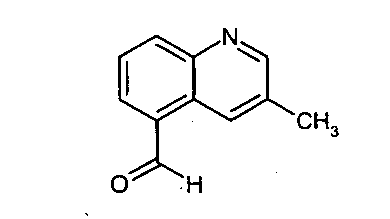

30.0 g (173 mmol) der Verbindung aus Beispiel 22A werden in 850 ml Dichlormethan aufgenommen und mit 240.0 g Mangan(IV)oxid versetzt. Es wird 16 h bei Raumtemperatur gerührt und anschließend über Kieselgur filtriert. Es wird mit Dichlormethan nachgewaschen und das Filtrat am Rotationsverdampfer eingeengt. Nach Trocknen des Rückstands werden 23.0 g (75% d. Th.) der Titelverbindung erhalten.
¹H-NMR (300 MHz, CDCl₃): δ = 2.57 (s, 3H), 7.78 (dd, 1H), 8.00 (dd, 1H), 8.31 (d, 1H), 8.84 (d, 1H), 9.36 (t, 1H), 10.32 (s, 1H).

### Beispiel 24A

### 1-(5-Ethyl-1,3-thiazol-2-yl)aceton

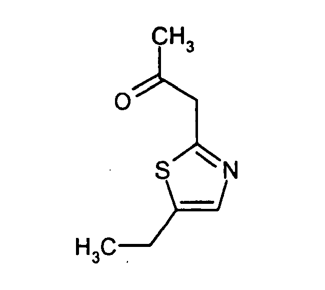

600 mg (4.72 mmol) 5-Ethyl-2-methyl-1,3-thiazol [M. Poite et al., Bull. Chem. Soc. Fr., 2078-2085 (1962)] werden in 20 ml abs. THF gelöst und bei -78°C tropfenweise mit 2.08 ml (5.18 mmol) n-Butyllithium (2.5 M Lösung in Hexan) versetzt. Nach zweistündigem Rühren bei -78°C werden 706 mg (8.02 mmol) Essigsäureethylester als Lösung in 10 ml abs. THF hinzugefügt. Es wird 1 h bei -78°C nachgerührt und dann auf Raumtemperatur erwärmt. Anschließend wird mit Natriumhydrogencarbonat-Lösung hydrolysiert und das Gemisch dreimal mit Diethylether extrahiert. Die vereinigten organischen Phasen werden mit Magnesiumsulfat getrocknet und das Lösungsmittel am Rotationsverdampfer entfernt. Das Rohprodukt wird mittels MPLC (Biotage 40M-Kartusche, Eluent: Isohexan/Ethylacetat 80:20) aufgereinigt. Es werden 50 mg (6% d. Th.) der Titelverbindung erhalten.
GC-MS (Methode 2): Rₜ = 4.42 min; MS (EIpos): m/z = 169 [M]⁺.

### Beispiel 25A

### 3-Methoxy-4-[2-(5-methyl-1,3,4-thiadiazol-2-yl)-3-oxobut-1-en-1-yl]benzonitril

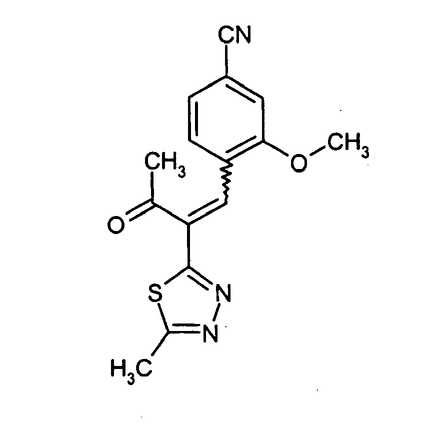

1.34 g (8.32 mmol) 1-(5-Methyl-1,3,4-thiadiazol-2-yl)aceton [T. Saito et al., J. Heterocycl. Chem. 20, 73-75 (1983)] und 1.30 g (8.32 mmol) der Verbindung aus Beispiel 3A werden in 20 ml Dichlormethan gelöst und mit 0.165 ml (1.66 mmol) Piperidin sowie 0.100 ml (1.66 mmol) Essigsäure versetzt. Die Reaktionsmischung wird über Nacht am inversen Wasserabscheider unter
Rückfluss erhitzt. Die flüchtigen Komponenten werden am Rotationsverdampfer entfernt und das Rohprodukt säulenchromatographisch aufgereinigt (Kieselgel; Eluent: zunächst Dichlormethan, dann Cyclohexan/Ethylacetat 4:1 → 1:1). Es werden 1.17 g (47% d. Th.) der Titelverbindung als Gemisch der E/Z-Isomere erhalten, welches aus Ethylacetat/n-Pentan kristallisiert werden kann. Es werden so 530 mg der Titelverbindung als kristalliner Feststoff erhalten.
LC-MS (Methode 1): Rₜ = 1.97 min; MS (EIpos): m/z = 300 [M+H]⁺.

### Ausführungsbeispiele:

### Beispiel 1

### 4-(4-Cyano-2-methoxyphenyl)-2,6-dimethyl-5-(4-methyl-1,3-thiazol-2-yl)-1,4-dihydropyridin-3-carbonitril

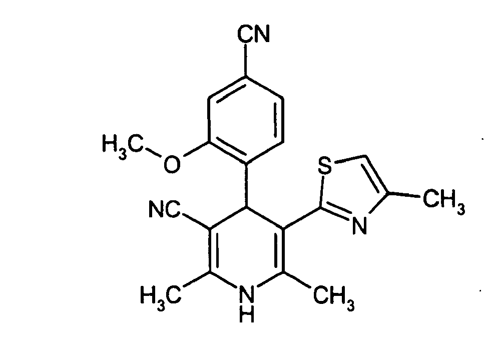

800 mg (4.96 mmol) der Verbindung aus Beispiel 3A, 770 mg (4.96 mmol) der Verbindung aus Beispiel 4A sowie 407 mg (4.96 mmol) 3-Aminocrotonsäurenitril werden in 12 ml Isopropanol gelöst und über Nacht bei Rückflusstemperatur gerührt. Nach dem Abkühlen auf Raumtemperatur werden die flüchtigen Komponenten am Rotationsverdampfer abgetrennt und das Rohprodukt in 20 ml Ethylacetat aufgenommen. Es wird 60 min bei Rückflusstemperatur gerührt und danach der entstandene Feststoff in der Wärme abfiltriert. Das so gewonnene Präzipitat wird mit wenig Diethylether gewaschen. Es werden 720 mg (40% d. Th.) der Titelverbindung erhalten.
LC-MS (Methode 3): Rₜ = 2.15 min; (EIpos): m/z = 363 [M+H]⁺
¹H-NMR (300 MHz, DMSO-d₆): δ [ppm] = 1.99 (s, 3H), 2.25 (s, 3H), 2.40 (s, 3H), 3.89 (s, 3H), 5.20 (s, 1H), 6.98 (s, 1H), 7.23 (d, 1H), 7.34 (d, 1H), 7.46 (s, 1H), 9.11 (s, 1H).

### Beispiel 2

### 4-(4-Cyano-2-methoxyphenyl)-6-ethyl-2-methyl-5-(4-methyl-1,3-thiazol-2-yl)-1,4-dihydropyridin-3-carbonitril

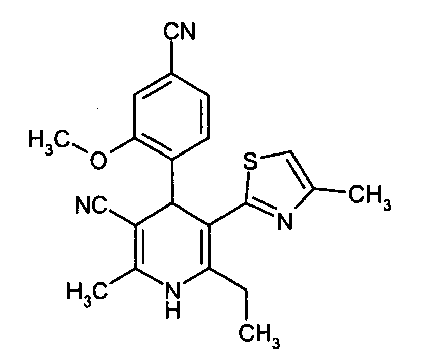

100 mg (0.620 mmol) der Verbindung aus Beispiel 3A, 100 mg (0.310 mmol, 50% Reinheit) der Verbindung aus Beispiel 5A und 51 mg (0.620 mmol) 3-Aminocrotonsäurenitril werden in 4 ml Isopropanol gelöst und über Nacht bei Rückflusstemperatur gerührt. Nach dem Abkühlen auf Raumtemperatur werden die flüchtigen Komponenten am Rotationsverdampfer abgetrennt und das Rohprodukt mittels präparativer HPLC aufgereinigt (Eluent: Acetonitril/Wasser mit 0.1% Ameisensäure, Gradient 20:80 → 95:5). Es werden 78 mg (66% d. Th.) der Titelverbindung erhalten.
LC-MS (Methode 3): Rₜ = 2.32 min; (EIpos): m/z = 377 [M+H]⁺
¹H-NMR (300 MHz, DMSO-d₆): δ [ppm] = 1.21 (t, 3H), 2.00 (s, 3H), 2.26 (s, 3H), 2.77 (m, 1H), 2.85 (m, 1H), 3.88 (s, 3H), 5.13 (s, 1H), 6.97 (s, 1H), 7.23 (d, 1H), 7.36 (dd, 1H), 7.47 (d, 1H), 9.07 (s, 1H).

### Beispiel 3

### 4-(4-Cyano-2-methoxyphenyl)-6-methyl-5-(4-methyl-1,3-thiazol-2-yl)-2-(trifluormethyl)-1,4-dihydropyridin-3-carbonitril

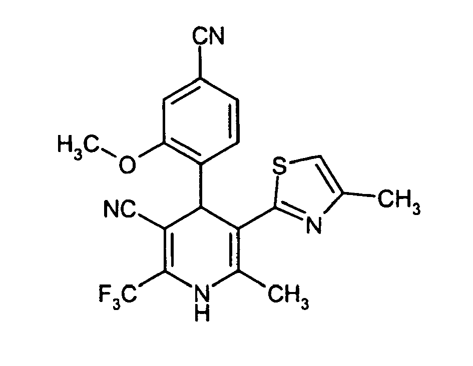

100 mg (0.335 mmol) der Verbindung aus Beispiel 6A, 46 mg (0.335 mmol) 3-Amino-4,4,4-trifluorbut-2-ennitril [Darstellung analog US-Patent 3,635,977 sowie K. Krespan, J. Org. Chem. 34, 42-45 (1969)] und 5.6 mg (0.05 mmol) Kalium-*tert.*-butylat werden in 4 ml Isopropanol gelöst und über Nacht bei Rückflusstemperatur gerührt. Nach dem Abkühlen auf Raumtemperatur werden die flüchtigen Komponenten am Rotationsverdampfer abgetrennt und das Rohprodukt mittels präparativer HPLC aufgereinigt (Eluent: Acetonitril/Wasser mit 0.1% Ameisensäure, Gradient 20:80 → 95:5). Es werden 119 mg (82% d. Th.) der Titelverbindung erhalten.
LC-MS (Methode 3): Rₜ = 2.40 min; (EIpos): m/z = 417 [M+H]⁺
¹H-NMR (300 MHz, DMSO-d₆): δ [ppm] = 2.27 (s, 3H), 2.45 (s, 3H), 3.91 (s, 3H), 5.40 (s, 1H), 7.11 (s, 1H), 7.27 (d, 1H), 7.39 (dd, 1H), 7.53 (d, 1H), 9.90 (s, 1H).

### Beispiel 4

### 2,6-Dimethyl-4-(2-methyl-4-oxo-4H-chromen-8-yl)-5-(4-methyl-1,3-thiazol-2-yl)-1,4-dihydropyridin-3-carbonitril

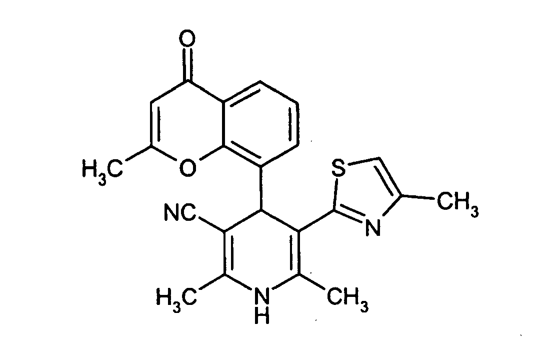

181 mg (0.97 mmol) der Verbindung aus Beispiel 11A, 150 mg (0.97 mmol) der Verbindung aus Beispiel 4A und 79 mg (0.97 mmol) 3-Aminocrotonsäurenitril werden in 4 ml Isopropanol gelöst und über Nacht bei Rückflusstemperatur gerührt. Nach dem Abkühlen auf Raumtemperatur werden die flüchtigen Komponenten am Rotationsverdampfer abgetrennt, das Rohmaterial in 5 ml Ethylacetat aufgenommen und das ausfallende Produkt anschließend abfiltriert. Es werden 240 mg (64% d. Th.) der Titelverbindung erhalten.
LC-MS (Methode 1): Rₜ = 2.09 min; (EIpos): m/z = 390 [M+H]⁺
¹H-NMR (300 MHz, DMSO-d₆): δ [ppm] = 2.03 (s, 3H), 2.22 (s, 3H), 2.39 (s, 3H), 2.44 (s, 3H), 5.39 (s, 1H), 6.27 (s, 1H), 6.98 (d, 1H), 7.37 (t, 1H), 7.56 (dd, 1H), 7.86 (dd, 1H), 9.23 (s, 1H).

### Beispiel 5

### 4-(4-Cyano-2-methoxyphenyl)-2-methyl-5-(4-methyl-1,3-thiazol-2-yl)-6-propyl-1,4-dihydropyridin-3-carbonitril

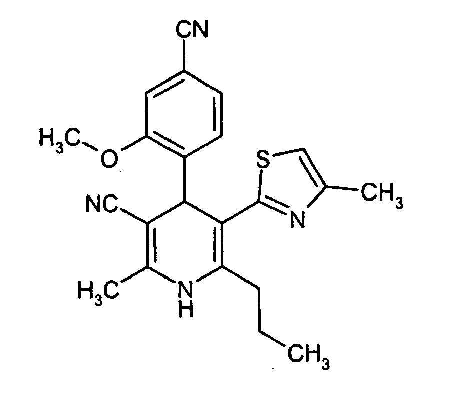

100 mg (0.620 mmol) der Verbindung aus Beispiel 3A, 114 mg (0.620 mmol) der Verbindung aus Beispiel 12A und 51 mg (0.620 mmol) 3-Aminocrotonsäurenitril werden in 4 ml Isopropanol gelöst und über Nacht bei Rückflusstemperatur gerührt. Nach dem Abkühlen auf Raumtemperatur werden die flüchtigen Komponenten am Rotationsverdampfer abgetrennt und das Rohprodukt mittels präparativer HPLC aufgereinigt (Eluent: Acetonitril/Wasser mit 0.1% Ameisensäure, Gradient 20:80 → 95:5). Es werden 134 mg (66% d. Th.) der Titelverbindung erhalten.
LC-MS (Methode 4): Rₜ = 3.86 min; (EIpos): m/z = 391 [M+H]⁺
¹H-NMR (300 MHz, DMSO-d₆): δ [ppm] = 0.98 (t, 3H), 1.66 (m, 2H), 1.99 (s, 3H), 2.26 (s, 3H), 2.69 (m, 1H), 2.81 (m, 1H), 3.88 (s, 3H), 5.16 (s, 1H), 6.96 (d, 1H), 7.23 (d, 1H), 7.36 (dd, 1H), 7.46 (d, 1H), 9.05 (s,1H).

### Beispiel 6

### 4-(2-Bromphenyl)-2,6-dimethyl-5-(4-methyl-1,3-thiazol-2-yl)-1,4-dihydropyridin-3-carbonitril

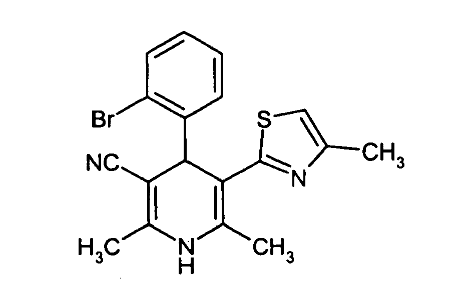

150 mg (0.811 mmol) 2-Brombenzaldehyd, 126 mg (0.811 mmol) der Verbindung aus Beispiel 4A und 67 mg (0.811 mmol) 3-Aminocrotonsäurenitril werden in 4 ml Isopropanol gelöst und über Nacht bei Rückflusstemperatur gerührt. Nach dem Abkühlen auf Raumtemperatur werden die flüchtigen Komponenten am Rotationsverdampfer abgetrennt, das Rohmaterial in 5 ml Ethylacetat aufgenommen und das ausfallende Produkt anschließend abfiltriert. Es werden 190 mg (61% d. Th.) der Titelverbindung erhalten.
LC-MS (Methode 1): Rₜ = 2.44 min; (EIpos): m/z = 386 [M+H]⁺
¹H-NMR (300 MHz, DMSO-d₆): δ [ppm] = 2.01 (s, 3H), 2.26 (s, 3H), 2.36 (s, 3H), 5.22 (s, 1H), 7.00 (s, 1H), 7.11 (m, 1H), 7.32 (d, 2H), 7.53 (d, 1H), 9.13 (s, 1H).

### Beispiel 7

### 4-(2-Chlor-4-fluorphenyl)-2,6-dimethyl-5-(4-methyl-1,3-thiazol-2-yl)-1,4-dihydropyridin-3-carbonitril

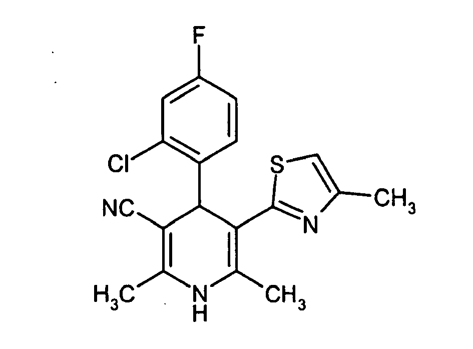

150 mg (0.946 mmol) 2-Chlor-4-fluorbenzaldehyd, 147 mg (0.811 mmol) der Verbindung aus Beispiel 4A und 78 mg (0.811 mmol) 3-Aminocrotonsäurenitril werden in 4 ml Isopropanol gelöst und über Nacht bei Rückflusstemperatur gerührt. Nach dem Abkühlen auf Raumtemperatur werden die flüchtigen Komponenten am Rotationsverdampfer abgetrennt, das Rohmaterial in 5 ml Ethylacetat aufgenommen und das ausfallende Produkt anschließend abfiltriert. Es werden 199 mg (58% d. Th.) der Titelverbindung erhalten.
LC-MS (Methode 1): Rₜ = 2.50 min; (EIpos): m/z = 360 [M+H]⁺
¹H-NMR (300 MHz, DMSO-d₆): δ [ppm] = 2.01 (s, 3H), 2.25 (s, 3H), 2.36 (s, 3H), 5.24 (s, 1H), 7.01 (s, 1H), 7.17 (dt, 1H), 7.31-7.38 (m, 2H), 9.16 (s, 1H).

### Beispiel 8

### 4-(4-Cyano-2-methoxyphenyl)-6-(methoxymethyl)-2-methyl-5-(4-methyl-1,3-thiazol-2-yl)-1,4-dihydropyridin-3-carbonitril

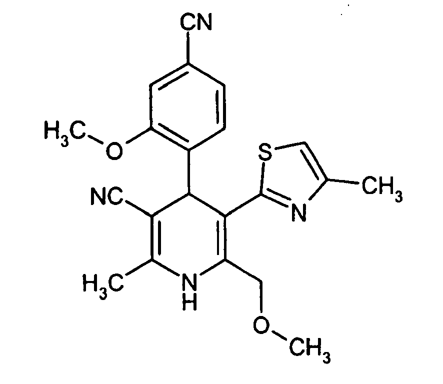

100 mg (0.305 mmol) der Verbindung aus Beispiel 14A und 25 mg (0.305 mmol) 3-Aminocrotonsäurenitril werden in 3 ml Isopropanol gelöst und über Nacht bei Rückflusstemperatur gerührt. Nach dem Abkühlen auf Raumtemperatur werden die flüchtigen Komponenten am Rotationsverdampfer abgetrennt und das Rohprodukt mittels präparativer HPLC aufgereinigt (Eluent: Acetonitril/Wasser mit 0.1% Ameisensäure, Gradient 20:80 → 95:5). Es werden 59 mg (49% d. Th.) der Titelverbindung erhalten.
LC-MS (Methode 1): Rₜ = 2.37 min; (EIpos): m/z = 393 [M+H]⁺
¹H-NMR (300 MHz, DMSO-d₆): δ [ppm] = 2.03 (s, 3H), 2.28 (s, 3H), 3.35 (s, 3H), 3.88 (s, 3H), 4.59 (d, 1H), 4.63 (d, 1H), 5.18 (s, 1H), 7.03 (s, 1H), 7.26 (d, 1H), 7.38 (dd, 1H), 7.49 (d, 1H), 9.10 (s, 1H).

### Beispiel 9

### 4-(4-Cyano-2-methoxyphenyl)-6-(methoxymethyl)-5-(4-methyl-1,3-thiazol-2-yl)-2-(trifluormethyl)-1,4-dihydropyridin-3-carbonitril

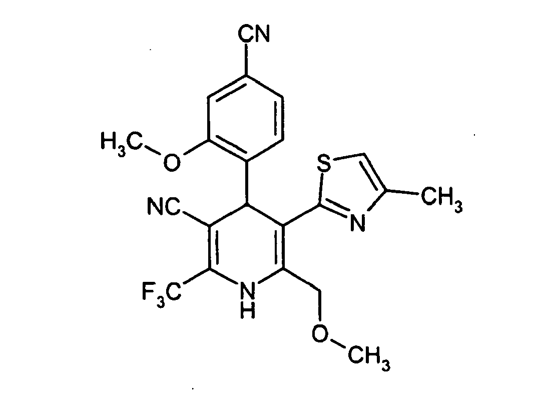

100 mg (0.305 mmol) der Verbindung aus Beispiel 14A, 41 mg (0.335 mmol) 3-Amino-4,4,4-trifluorbut-2-ennitril [Darstellung analog US-Patent 3,635,977 sowie K. Krespan, J. Org. Chem. 34, 42-45 (1969)] und 5.1 mg (0.05 mmol) Kalium-*tert*.-butylat werden in 4 ml Isopropanol gelöst und über Nacht bei Rückflusstemperatur gerührt. Nach dem Abkühlen auf Raumtemperatur werden die flüchtigen Komponenten am Rotationsverdampfer abgetrennt und das Rohmaterial mittels präparativer HPLC aufgereinigt (Eluent: Acetonitril/Wasser mit 0.1% Ameisensäure, Gradient 20:80 → 95:5). Es werden 18 mg (13% d. Th.) der Titelverbindung erhalten.
LC-MS (Methode 1): Rₜ = 2.62 min; (EIpos): m/z = 447 [M+H]⁺
¹H-NMR (300 MHz, DMSO-d₆): δ [ppm] = 2.29 (s, 3H), 3.34 (s, 3H), 3.90 (s, 3H), 4.51 (d, 1H), 4.60 (d, 1H), 5.40 (s, 1H), 7.17 (s, 1H), 7.29 (d, 1H), 7.42 (dd, 1H), 7.55 (d, 1H), 9.97 (s, 1H).

### Beispiel 10

### 4-(4-Cyano-2-methoxyphenyl)-6-ethyl-5-(4-methyl-1,3-thiazol-2-yl)-2-(trifluormethyl)-1,4-dihydropyridin-3-carbonitril

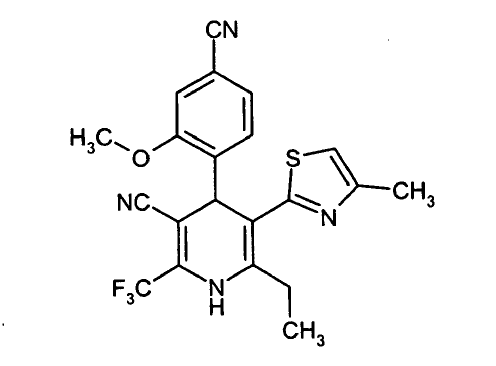

150 mg (0.480 mmol) der Verbindung aus Beispiel 20A, 65 mg (0.480 mmol) 3-Amino-4,4,4-trifluorbut-2-ennitril [Darstellung analog US-Patent 3,635,977 sowie K. Krespan, J. Org. Chem. 34, 42-45 (1969)] und 8.1 mg (0.072 mmol) Kalium-*tert*.-butylat werden in 4 ml Isopropanol gelöst und über Nacht bei Rückflusstemperatur gerührt. Nach dem Abkühlen auf Raumtemperatur werden die flüchtigen Komponenten am Rotationsverdampfer abgetrennt, das Rohmaterial zunächst mittels präparativer HPLC (Eluent: Acetonitril/Wasser mit 0.1% Ameisensäure, Gradient 20:80 → 95:5) und anschließend mittels MPLC (Biotage 12M-Kartusche, Eluent: Isohexan/Ethylacetat 80:20) aufgereinigt. Es werden 25 mg (12% d. Th.) der Titelverbindung erhalten.
LC-MS (Methode 1): Rₜ = 2.68 min; (EIpos): m/z = 431 [M+H]⁺
¹H-NMR (300 MHz, DMSO-d₆): δ [ppm] = 1.21 (t, 3H), 2.28 (s, 3H), 2.85 (d, 1H), 2.89 (d, 1H), 3.90 (s, 3H), 5.32 (s, 1H), 7.10 (s, 1H), 7.25 (d, 1H), 7.41 (dd, 1H), 7.54 (d, 1H), 9.89 (s, 1H).

### Beispiel 11

### 4-(4-Cyano-2-methoxyphenyl)-5-(4-ethyl-1,3-thiazol-2-yl)-2,6-dimethyl-1,4-dihydropyridin-3-carbonitril

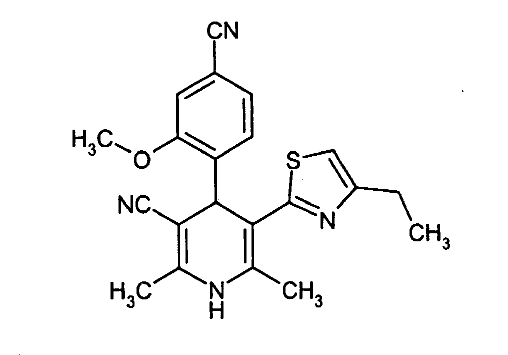

770 mg (4.78 mmol) der Verbindung aus Beispiel 3A und 890 mg (5.26 mmol) der Verbindung aus Beispiel 17A werden in 15 ml Dichlormethan gelöst und mit 0.473 ml (4.78 mmol) Piperidin sowie 0.247 ml (4.78 mmol) Essigsäure versetzt. Die Reaktionsmischung wird über Nacht am inversen Wasserabscheider unter Rückfluss erhitzt. Die flüchtigen Komponenten werden am Rotationsverdampfer abgetrennt und das Rohprodukt mittels MPLC aufgereinigt (Biotage 12M-Kartusche, Eluent: Isohexan/Ethylacetat 80:20 → 70:30). Es werden 920 mg (66% d. Th.) 4-[2-(4-Ethyl-1,3-thiazol-2-yl)-3-oxobut-1-en-1-yl]-3-methoxybenzonitril als Gemisch der *E*/*Z*-Isomere erhalten {LC-MS (Methode 1): Rₜ = 2.39 min; (EIpos): m/z = 313 [M+H]⁺}, welche ohne weitere Auf arbeitung umgesetzt werden.
150 mg (0.480 mmol) der so erhaltenen Benzylidenverbindung und 39 mg (0.480 mmol) 3-Aminocrotonsäurenitril werden in 4 ml Isopropanol gelöst und über Nacht bei Rückflusstemperatur gerührt. Nach dem Abkühlen auf Raumtemperatur werden die flüchtigen Komponenten am Rotationsverdampfer abgetrennt und das Rohmaterial mittels präparativer HPLC aufgereinigt (Eluent: Acetonitril/Wasser mit 0.1% Ameisensäure, Gradient 20:80 → 95:5). Es werden 26 mg (14% d. Th.) der Titelverbindung erhalten.
LC-MS (Methode 4): Rₜ = 3.66 min; (EIpos): m/z = 377 [M+H]⁺
¹H-NMR (300 MHz, DMSO-d₆): δ [ppm] = 1.12 (t, 3H), 1.99 (s, 3H), 2.40 (s, 3H), 2.59 (q, 2H), 3.89 (s, 3H), 5.22 (s, 1H), 6.98 (s, 1H), 7.24 (d, 1H), 7.34 (dd, 1H), 7.47 (d, 1H), 9.12 (s, 1H).

### Beispiel 12

### 5-(1,3-Benzothiazol-2-yl)-4-(4-cyano-2-methoxyphenyl)-2,6-dimethyl-1,4-dihydropyridin-3-carbonitril

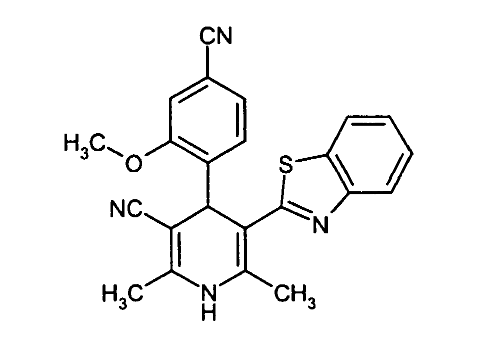

150 mg (0.931 mmol) der Verbindung aus Beispiel 3A, 178 mg (0.931 mmol) 1-(1,3-Benzothiazol-2-yl)aceton [Costa et al., J. Heterocycl. Chem. 28, 1541-1544 (1991)] und 76 mg (0.931 mmol) 3-Aminocrotonsäurenitril werden in 6 ml Isopropanol gelöst und über Nacht bei Rückflusstemperatur gerührt. Nach dem Abkühlen auf Raumtemperatur werden die flüchtigen Komponenten am Rotationsverdampfer abgetrennt. Das so gewonnene Rohprodukt wird aus Acetonitril umkristallisiert. Es werden 40 mg (11% d. Th.) der Titelverbindung erhalten.
LC-MS (Methode 4): Rₜ = 3.78 min; (EIpos): m/z = 399 [M+H]⁺
¹H-NMR (300 MHz, DMSO-d₆): δ [ppm] = 2.02 (s, 3H), 2.53 (s, 3H), 3.94 (s, 3H), 5.33 (s, 1H), 7.26-7.32 (m, 2H), 7.35 (dd, 1H), 7.41 (dt, 1H), 7.50 (d, 1H), 7.79 (d, 1H), 7.94 (d, 1H), 9.36 (s, 1H).

### Beispiel 13

### 4-(2,4-Dichlorphenyl)-2,6-dimethyl-5-(4-methyl-1,3-thiazol-2-yl)-1,4-dihydropyridin-3-carbonitril

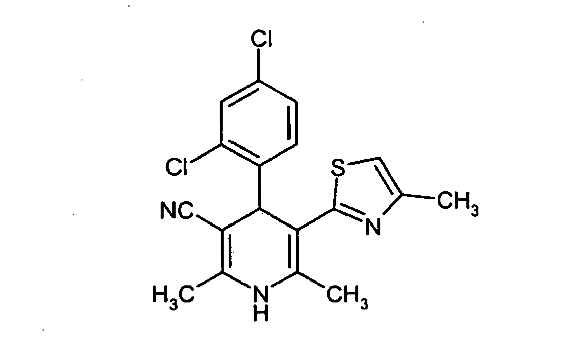

150 mg (0.857 mmol) 2,4-Dichlorbenzaldehyd, 133 mg (0.857 mmol) der Verbindung aus Beispiel 4A und 70 mg (0.857 mmol) 3-Aminocrotonsäurenitril werden in 4 ml Isopropanol gelöst und über Nacht bei Rückflusstemperatur gerührt. Nach dem Abkühlen auf Raumtemperatur werden die flüchtigen Komponenten am Rotationsverdampfer abgetrennt und das Rohprodukt in 3 ml Ethylacetat aufgenommen. Der kristallisierende Feststoff wird per Filtration abgetrennt. Das so gewonnene Produkt wird mit wenig Diethylether gewaschen. Es werden 85 mg (26% d. Th.) der Titelverbindung erhalten.
LC-MS (Methode 8): Rₜ = 3.74 min; (EIpos): m/z = 376 [M+H]⁺
¹H-NMR (300 MHz, DMSO-d₆): δ [ppm] = 2.01 (s, 3H), 2.25 (s, 3H), 2.35 (s, 3H), 5.26 (s, 1H), 7.02 (d, 1H), 7.32 (d, 1H), 7.37 (dd, 1H), 7.53 (d, 1H), 9.18 (s, 1H).

### Beispiel 14

### 4-(2-Brom-4-fluorphenyl)-2,6-dimethyl-5-(4-methyl-1,3-thiazol-2-yl)-1,4-dihydropyridin-3-carbonitril

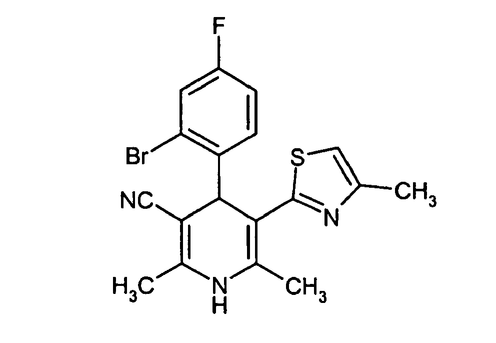

150 mg (0.739 mmol) 2-Brom-4-fluorbenzaldehyd, 115 mg (0.739 mmol) der Verbindung aus Beispiel 4A und 61 mg (0.739 mmol) 3-Aminocrotonsäurenitril werden in 4 ml Isopropanol gelöst und über Nacht bei Rückflusstemperatur gerührt. Nach dem Abkühlen auf Raumtemperatur werden die flüchtigen Komponenten am Rotationsverdampfer abgetrennt und das Rohprodukt mittels Flash-Chromatographie aufgereinigt (Kieselgel, Laufmittel: Cyclohexan/Ethylacetat 1: 1). Nach Abtrennung des Lösungsmittels wird das Produkt durch Kristallisation aus Diisopropylether gewonnen. Nach Trocknen im Hochvakuum werden 91 mg (30% d. Th.) der Titelverbindung erhalten.
LC-MS (Methode 3): Rₜ = 2.39 min; (EIpos): m/z = 405 [M+H]^{+ 1}H-NMR (300 MHz, DMSO-d₆): δ [ppm] = 2.01 (s, 3H), 2.26 (s, 3H), 2.35 (s, 3H), 5.22 (s, 1H), 7.02 (d, 1H), 7.21 (dt, 1H), 7.34 (dd, 1H), 7.48 (dd, 1H), 9.16 (s, 1H).

### Beispiel 15

### 4-(4-Cyano-2-methoxyphenyl)-2,6-dimethyl-5-(4,5,6,7-tetrahydro-1,3-benzothiazol-2-yl)-1,4-dihydropyridin-3-carbonitril

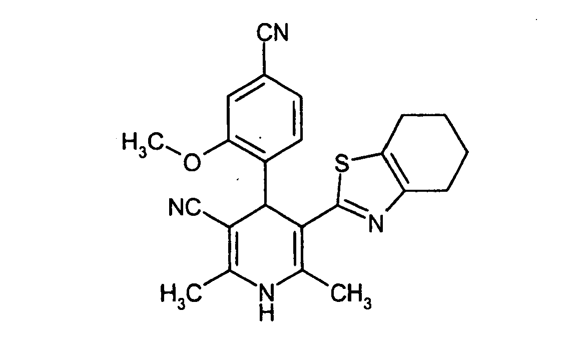

150 mg (0.931 mmol) der Verbindung aus Beispiel 3A, 181 mg (0.931 mmol) der Verbindung aus Beispiel 19A und 76 mg (0.931 mmol) 3-Aminocrotonsäurenitril werden in 4 ml Isopropanol gelöst und über Nacht bei Rückflusstemperatur gerührt. Nach dem Abkühlen auf Raumtemperatur werden die flüchtigen Komponenten am Rotationsverdampfer abgetrennt und das Rohprodukt in 3 ml Ethylacetat aufgenommen. Der kristallisierende Feststoff wird per Filtration abgetrennt. Das so gewonnene Präzipitat wird mit heißem Acetonitril gewaschen und im Hochvakuum getrocknet. Es werden 131 mg (35% d. Th.) der Titelverbindung erhalten.
LC-MS (Methode 3): Rₜ = 2.43 min; (EIpos): m/z = 403 [M+H]⁺
¹H-NMR (300 MHz, DMSO-d₆): δ [ppm] = 1.71 (m, 4H), 1.98 (s, 3H), 2.37 (s, 3H), 2.57 (m, 2H), 2.62 (m, 2H), 3.89 (s, 3H), 5.16 (s, 1H), 7.20 (d, 1H), 7.35 (dd, 1H), 7.47 (d, 1H), 9.09 (s, 1H).

### Beispiel 16

### 4-(4-Fluor-2-methoxyphenyl)-2,6-dimethyl-5-(4-methyl-1,3-thiazol-2-yl)-1,4-dihydropyridin-3-carbonitril

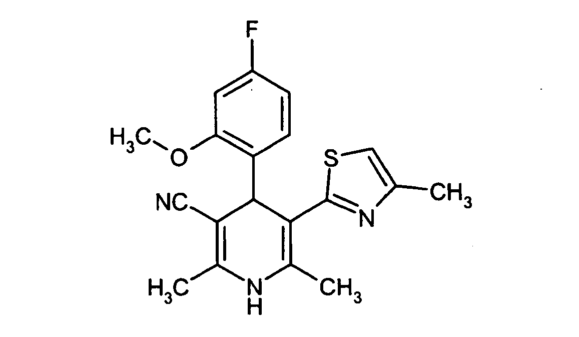

150 mg (0.973 mmol) 4-Fluor-2-methoxybenzaldehyd, 151 mg (0.973 mmol) der Verbindung aus Beispiel 4A und 80 mg (0.973 mmol) 3-Aminocrotonsäurenitril werden in 4 ml Isopropanol gelöst und über Nacht bei Rückflusstemperatur gerührt. Nach dem Abkühlen auf Raumtemperatur werden die flüchtigen Komponenten am Rotationsverdampfer abgetrennt und das Rohprodukt mittels Flash-Chromatographie aufgereinigt (Kieselgel, Laufmittel: Cyclohexan/Ethylacetat 1:1). Nach Abtrennung des Lösungsmittels wird das Produkt durch Kristallisation aus Diisopropylether gewonnen. Nach der Trocknen im Hochvakuum werden 123 mg (36% d. Th.) der Titelverbindung erhalten.
LC-MS (Methode 1): Rₜ = 2.43 min; (EIpos): m/z = 356 [M+H]⁺
¹H-NMR (300 MHz, DMSO-d₆): δ [ppm] = 1.97 (s, 3H), 2.26 (s, 3H), 2.39 (s, 3H), 3.83 (s, 3H), 5.04 (s, 1H), 6.68 (dt, 1H), 6.87 (dd, 1H), 6.95 (s, 1H), 7.07 (dd, 1H), 9.00 (s, 1H).

### Beispiel 17

### 2-Methoxy-2',6'-dimethyl-5'-(4-methyl-1,3-thiazol-2-yl)-1',4'-dihydro-3,4'-bipyridin-3'-carbonitril

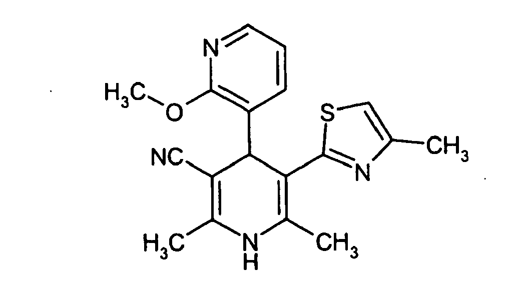

150 mg (1.09 mmol) 2-Methoxy-3-pyridincarboxaldehyd, 170 mg (1.09 mmol) der Verbindung aus Beispiel 4A und 90 mg (1.09 mmol) 3-Aminocrotonsäurenitril werden in 4 ml Isopropanol gelöst und über Nacht bei Rückflusstemperatur gerührt. Nach dem Abkühlen auf Raumtemperatur werden die flüchtigen Komponenten am Rotationsverdampfer abgetrennt und das Rohprodukt in 3 ml Ethylacetat aufgenommen. Der kristallisierende Feststoff wird per Filtration abgetrennt. Das so gewonnene Präzipitat wird mit wenig Diethylether gewaschen. Es werden 216 mg (58% d. Th.) der Titelverbindung erhalten.
LC-MS (Methode 8): Rₜ = 2.87 min; (EIpos): m/z = 339 [M+H]⁺
¹H-NMR (300 MHz, DMSO-d₆): δ [ppm] = 2.00 (s, 3H), 2.26 (s, 3H), 2.39 (s, 3H), 3.90 (s, 3H), 5.02 (s, 1H), 6.91 (m, 1H), 6.98 (s, 1H), 7.40 (d, 1H), 7.99 (m, 1H), 9.09 (s, 1H).

### Beispiel 18

### 2,6-Dimethyl-4-(3-methylchinolin-5-yl)-5-(4-methyl-1,3-thiazol-2-yl)-1,4-dihydropyridin-3-carbonitril

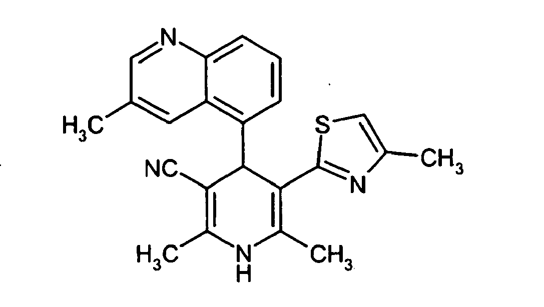

150 mg (0.876 mmol) der Verbindung aus Beispiel 23A, 135 mg (0.876 mmol) der Verbindung aus Beispiel 4A und 71 mg (0.876 mmol) 3-Aminocrotonsäurenitril werden in 4 ml Isopropanol gelöst und über Nacht bei Rückflusstemperatur gerührt. Nach dem Abkühlen auf Raumtemperatur werden die flüchtigen Komponenten am Rotationsverdampfer abgetrennt und das Rohprodukt mittels präparativer HPLC aufgereinigt (Eluent: Acetonitril/Wasser mit 0.1% Ameisensäure, Gradient 20:80 → 95:5). Es werden 145 mg (44% d. Th.) der Titelverbindung erhalten.
LC-MS (Methode 8): Rₜ = 2.23 min; (EIpos): m/z = 373 [M+H]⁺
¹H-NMR (300 MHz, DMSO-d₆): δ [ppm] = 2.02 (s, 3H), 2.17 (s, 3H), 2.40 (s, 3H), 5.64 (s, 1H), 6.89 (d, 1H), 7.47 (dd, 1H), 7.61 (t, 1H), 7.82 (d, 1H), 8.72 (s, 1H), 8.77 (d, 1H), 9.18 (s, 1H) (1 CH₃-Signal durch Solvens-Signal überlagert).

### Beispiel 19

### 4-(4-Fluor-1-naphthyl)-2,6-dimethyl-5-(4-methyl-1,3-thiazol-2-yl)-1,4-dihydropyridin-3-carbonitril

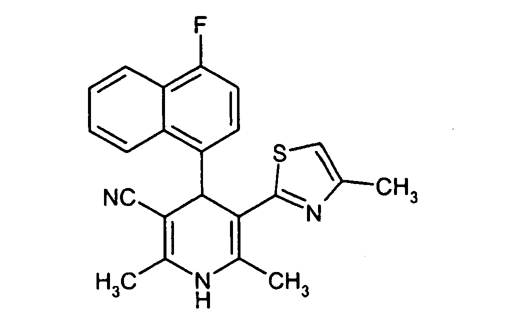

150 mg (0.861 mmol) 4-Fluor-1-naphthaldehyd, 134 mg (0.861 mmol) der Verbindung aus Beispiel 4A und 70 mg (0.861 mmol) 3-Aminocrotonsäurenitril werden in 4 ml Isopropanol gelöst und über Nacht bei Rückflusstemperatur gerührt. Nach dem Abkühlen auf Raumtemperatur werden die flüchtigen Komponenten am Rotationsverdampfer abgetrennt und das Rohprodukt mittels präparativer HPLC aufgereinigt (Eluent: Acetonitril/Wasser mit 0.1% Ameisensäure, Gradient 20:80 → 95:5). Es werden 14 mg (4% d. Th.) der Titelverbindung erhalten.
LC-MS (Methode 1): Rₜ = 2.64 min; (EIpos): m/z = 376 [M+H]⁺
¹H-NMR (300 MHz, DMSO-d₆): δ [ppm] = 2.01 (s, 3H), 2.17 (s, 3H), 2.41 (s, 3H), 5.62 (s, 1H), 6.87 (s, 1H), 7.28 (dd, 1H), 7.39 (dd, 1H), 7.64 (t, 1H), 7.70 (dt, 1H), 8.04 (d, 1H), 8.58 (d, 1H), 9.16 (s, 1H).

### Beispiel 20

### 4-(2-Cyano-4-fluorphenyl)-2,6-dimethyl-5-(4-methyl-1,3-thiazol-2-yl)-1,4-dihydropyridin-3-carbonitril

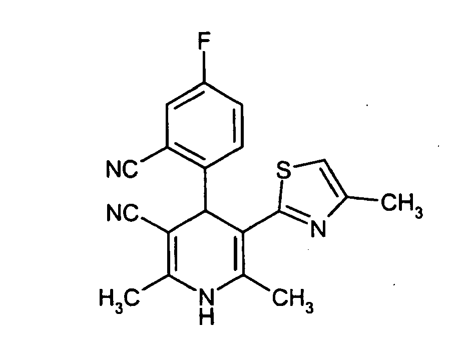

50 mg (0.124 mmol) der Verbindung aus Beispiel 14 und 10.7 mg (0.092 mmol) Zinkcyanid in 0.5 ml abs. DMF werden mit 4.7 mg (0.004 mmol) Tetrakis(triphenylphosphin)palladium(0) versetzt und in einer Single Mode-Mikrowelle (Emrys Optimizer) 5 min bei 220°C zur Reaktion gebracht. Nach erfolgter Umsetzung (DC-Kontrolle) wird die Reaktionsmischung direkt mittels präparativer HPLC aufgereinigt (Eluent: Acetonitril/Wasser mit 0.1% Ameisensäure, Gradient 20:80 → 95:5). Es werden 24 mg (55% d. Th.) der Titelverbindung erhalten.
LC-MS (Methode 4): Rₜ = 3.47 min; (EIpos): m/z = 351 [M+H]⁺
¹H-NMR (300 MHz, DMSO-d₆): δ [ppm] = 2.04 (s, 3H), 2.24 (s, 3H), 2.31 (s, 3H), 5.21 (s, 1H), 7.06 (d, 1H), 7.44-7.55 (m, 2H), 7.76 (dd, 1H), 9.28 (s, 1H).

### Beispiel 21

### ent-4-(4-Cyano-2-methoxyphenyl)-2,6-dimethyl-5-(4-methyl-1,3-thiazol-2-yl)-1,4-dihydropyridin-3-carbonitril [(-)-Enantiomer und (+)-Enantiomer]

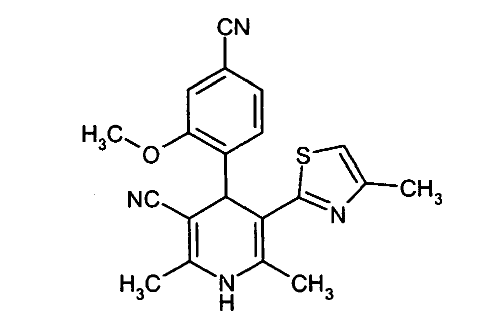

Das Racemat aus Beispiel 1 wird durch Chromatographie an chiraler Phase in seine Antipoden aufgetrennt [Säule: 680 mm x 40 mm; chirale Kieselgel-Phase basierend auf dem Selektor Poly(*N-*methacryloyl-D-leucin-*tert*.-butylamid); Eluent: Isohexan/Essigsäureethylester 55:45 (v/v); Temperatur: 24°C; Fluss: 80 ml/min; UV-Detektion: 260 nm].
Die Retentionszeiten aus einer vergleichbaren analytischen HPLC basierend auf dem gleichen Selektor [Säule: 250 mm x 4.6 mm; Eluent: Isohexan/Ethylacetat 4:1 (v/v); Fluss: 1 ml/min] und die spezifischen Drehwerte der enantiomerenreinen Verbindungen sind wie folgt bestimmt worden:
(-)-Enantiomer:
Rₜ = 7.01 min; ee∼97%
spezifischer Drehwert (Chloroform, 589 nm, 20°C, c = 0.50500 g / 100 ml): -578.2°.
(+)-Enantiomer:
Rₜ = 7.61 min; ee ∼ 96%
spezifischer Drehwert (Chloroform, 589 nm, 20°C, c = 0.50000 g / 100 ml): +590.3°.

### Beispiel 22

### ent-4-(4-Cyano-2-methoxyphenyl)-6-(methoxymethyl)-5-(4-methyl-1,3-thiazol-2-yl)-2-(trifluormethyl)-1,4-dihydropyridin-3-carbonitril [(-)-Enantiomer und (+)-Enantiomer]

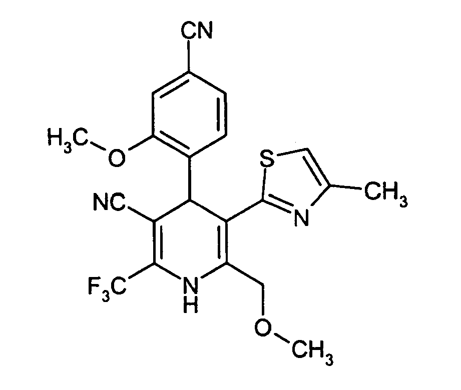

Das Racemat aus Beispiel 9 wird durch Chromatographie an chiraler Phase in seine Antipoden aufgetrennt [Säule: 680 mm x 40 mm; chirale Kieselgel-Phase basierend auf dem Selektor Poly(*N-*methacryloyl-D-leucin-*tert*.-butylamid); Eluent: Isohexan/Essigsäureethylester 90:10 (v/v); Temperatur: 24°C; Fluss: 80 ml/min; UV-Detektion: 260 nm].
Die Retentionszeiten aus einer vergleichbaren analytischen HPLC basierend auf dem gleichen Selektor [Säule: 250 mm x 4.6 mm; Eluent: Isohexan/Ethylacetat 10:1 (v/v); Fluss: 2 ml/min] und die spezifischen Drehwerte der enantiomerenreinen Verbindungen sind wie folgt bestimmt worden:
(-)-Enantiomer:
Rₜ = 10.07 min; ee = 98%
spezifischer Drehwert (Chloroform, 589 nm, 20°C, c = 0.49500 g / 100 ml): -759.1°.
(+)-Enantiomer:
Rₜ = 13.66 min; ee = 98.5%
spezifischer Drehwert (Chloroform, 589 nm, 20°C, c = 0.50000 g / 100 ml): +757.2°.

### Beispiel 23

### 4-(4-Cyano-2-methoxyphenyl)-5-(5-ethyl-1,3-thiazol-2-yl)-2,6-dimethyl-1,4-dihydropyridin-3-carbonitril

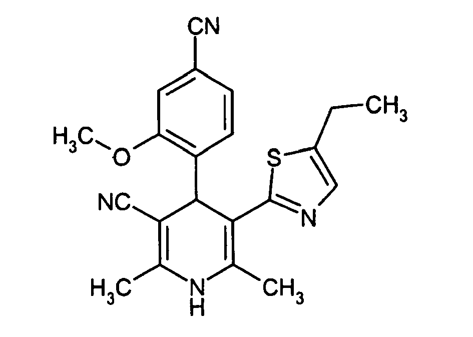

47.6 mg (0.295 mmol) der Verbindung aus Beispiel 3A, 50 mg (0.295 mmol) der Verbindung aus Beispiel 24A sowie 24 mg (0.295 mmol) 3-Aminocrotonsäurenitril werden in 2 ml Isopropanol gelöst und über Nacht bei Rückflusstemperatur gerührt. Nach dem Abkühlen auf Raumtemperatur werden die flüchtigen Komponenten am Rotationsverdampfer abgetrennt und das Rohprodukt mittels präparativer HPLC aufgereinigt (Eluent: Acetonitril/Wasser mit 0.1% Ameisensäure, Gradient 20:80 → 95:5). Es werden 23 mg (19% d. Th.) der Titelverbindung in 90%-iger Reinheit (nach LC-MS) erhalten, die durch Kristallisation aus Ethylacetat weiter gereinigt werden können. So werden 3 mg (3% d. Th.) der Zielverbindung in Reinform isoliert.
LC-MS (Methode 8): Rₜ = 3.39 min; (EIpos): m/z = 377 [M+H]⁺
¹H-NMR (300 MHz, DMSO-d₆): δ [ppm] = 1.15 (t, 3H), 1.98 (s, 3H), 2.36 (s, 3H), 2.72 (q, 2H), 3.88 (s, 3H), 5.20 (s, 1H), 7.19 (d, 1H), 7.33 (dd, 1H), 7.35 (br. s, 1H), 7.47 (d, 1H), 9.11 (s, 1H).

### Beispiel 24

### 4-(4-Cyano-2-methoxyphenyl)-2,6-dimethyl-5-(5-methyl-1,3,4-thiadiazol-2-yl)-1,4-dihydropyridin-3-carbonitril

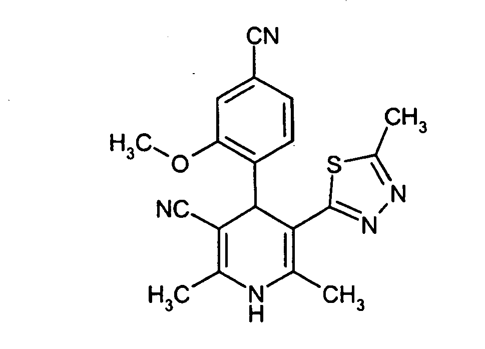

150 mg (0.501 mmol) der Verbindung aus Beispiel 25A und 41 mg (0.501 mmol) 3-Aminocrotonsäurenitril werden in 5 ml Isopropanol gelöst und über Nacht bei Rückflusstemperatur gerührt. Nach dem Abkühlen auf Raumtemperatur werden die flüchtigen Komponenten am Rotationsverdampfer entfernt und das Rohprodukt mittels präparativer HPLC aufgereinigt (Eluent: Acetonitril/ Wasser mit 0.1% Ameisensäure, Gradient 20:80 → 95:5). Nach Kristallisation aus Ethylacetat/ Diethylether werden 30 mg (16% d. Th.) der Titelverbindung erhalten.
LC-MS (Methode 8): Rₜ = 2.62 min; (EIpos): m/z = 364 [M+H]⁺
¹H-NMR (300 MHz, DMSO-d₆): δ [ppm] = 2.00 (s, 3H), 2.30 (s, 3H), 2.59 (s, 3H), 3.87 (s, 3H), 5.18 (s, 1H), 7.25 (d, 1H), 7.36 (dd, 1H), 7.49 (d, 1H), 9.28 (s, 1H).

### B. Bewertung der pharmakologischen Wirksamkeit

### Abkürzungen:

- DMEM: Dulbecco's Modified Eagle Medium
- DNA: Desoxyribo Nucleic Acid
- FCS: Fetal Calf Serum
- HEPES: 4-(2-Hydroxyethyl)-1-piperazin-ethansulfonsäure
- PCR: Polymerase Chain Reaction
- Tris: Tris-(hydroxymethyl)-methylamin

Die vorteilhaften pharmakologischen Eigenschaften der erfindungsgemäßen Verbindungen können in folgenden Assays gezeigt werden:

### 1. Zellulärer in vitro-Test zur Bestimmung der inhibitorischen MR-Aktivität und MR-Selektivität gegenüber anderen Steroidhormon-Rezeptoren

Die Identifizierung von Antagonisten des humanen Mineralokorticoid-Rezeptors (MR) sowie die Quantifizierung der Wirksamkeit der hier beschriebenen Verbindungen erfolgt mit Hilfe einer rekombinanten Zelllinie. Die Zelle leitet sich ursprünglich von einer Ovarepithelzelle des Hamsters ab (Chinese Hamster Ovary, CHO K1, ATCC: American Type Culture Collection, VA 20108, USA).

In dieser CHO K1-Zelllinie wird ein etabliertes Chimärensystem verwendet, in dem die Liganden-Bindungsdomänen humaner Steroidhormon-Rezeptoren an die DNA-Bindungsdomäne des Hefe-Transkriptionsfaktors GAL4 fusioniert werden. Die so entstehenden GAL4-Steroidhormonrezeptor-Chimären werden in den CHO-Zellen mit einem Reporterkonstrukt co-transfiziert und stabil exprimiert.

### Klonierungen:

Zur Generierung der GAL4-Steroidhormonrezeptor-Chimären wird die GAL4-DNA-Bindungsdomäne (Aminosäuren 1-147) aus dem Vektor pFC2-dbd (Fa. Stratagene) mit den PCR-amplifizierten Liganden-Bindungsdomänen des Mineralokorticoid-Rezeptors (MR, Aminosäuren 734-985), des Glucokorticoid-Rezeptors (GR, Aminosäuren 443-777), des Progesteron-Rezeptors (PR, Aminosäuren 680-933) und des Androgen-Rezeptors (AR, Aminosäuren 667-919) in den Vektor pIRES2 (Fa. Clontech) kloniert. Das Reporterkonstrukt, welches fünf Kopien der GAL4-Bindestelle, vorgeschaltet vor einem Thymidinkinase-Promotor enthält, führt zur Expression der Firefly-Luciferase *(Photinus pyralis)* nach Aktivierung und Bindung der GAL4-Steroidhormonrezeptor-Chimären durch die jeweiligen spezifischen Agonisten Aldosteron (MR), Dexamethason (GR), Progesteron (PR) und Dihydrotestosteron (AR).

### Testablauf:

Die MR-, GR-, PR- und AR-Zellen werden am Tag vor dem Test in Medium (Optimem, 2.5% FCS, 2 mM Glutamin, 10 mM HEPES) in 96- (oder 384- bzw. 1536-) Loch-Mikrotiterplatten ausplattiert und in einem Zellinkubator (96% Luftfeuchtigkeit, 5% v/v CO₂, 37°C) gehalten. Am Testtag werden die zu prüfenden Substanzen in oben genanntem Medium aufgenommen und zu den Zellen hinzugegeben. Etwa 10 bis 30 Minuten nach Zugabe der Testsubstanzen werden die jeweiligen spezifischen Agonisten der Steroidhormon-Rezeptoren hinzugesetzt. Nach einer weiteren Inkubationszeit von 5 bis 6 Stunden wird die Luciferaseaktivität mit Hilfe einer Videokamera gemessen. Die gemessenen relativen Lichteinheiten ergeben in Abhängigkeit von der Substanzkonzentration eine sigmoide Stimulationskurve. Die Berechnung der IC₅₀-Werte erfolgt mit Hilfe des Computerprogramms GraphPad PRISM (Version 3.02).
Tabelle A zeigt die IC₅₀-Werte (MR) repräsentativer Beispielverbindungen:

**Tabelle A**

| **Beispiel-Nr.** | **MR IC₅₀ [nM]** |
|---|---|
| 1 | 94 |
| 3 | 10 |
| 4 | 95 |
| 12 | 74 |
| 18 | 278 |
| 23 | 18 |
| 24 | 320 |

### 2. In vitro-Test zur Bestimmung möglicher Bindungsaktivität am L-Typ Calcium-Kanal

Membranpräparationen des zerebralen Cortex von Wistar-Ratten dienen als Ausgangsmaterial für einen radioaktiven Bindungstest, der als Standardassay in der Literatur ausführlich beschrieben ist [Ehlert, F.J., Roeske, W.R., Itoga E., Yamamura, H.I., Life Sci. 30, 2191-2202 (1982); Gould, R.J., Murphy, K.M.M., Snyder, S.H., Proc. Natl. Acad. Sci. US.A. 79, 3656-3660] und von kommerziellen Anbietern (z.B. Fa. MDS Pharma Services) im Rahmen von Auftragsuntersuchungen verwendet wird. In diesem Bindungsassay werden Verdünnungsreihen der Testverbindungen in DMSO typischerweise für 90 Minuten bei 25°C in einem 50 mM TrisHCl-Puffer, pH 7.7, mit den Membranpräparationen und dem Tritium-markierten Liganden Nitrendipin (0.1 nM) inkubiert und die spezifische Bindung der Testverbindungen über Quantifizierung des spezifisch verdrängten, radioaktiv markierten Liganden bestimmt. IC₅₀-Werte werden über eine nicht-lineare Regressionsanalyse ermittelt.

In diesem L-Typ Calcium-Kanal-Bindungsassay wird für einen klassischen Calcium-Antagonisten vom Dihydropyridin-Typ, wie z.B. Nitrendipin, ein IC₅₀-Wert von 0.3 nM bestimmt, während untersuchte Beispiele der hier beschriebenen erfindungsgemäßen Verbindungen IC₅₀-Werte von > 1 µM und somit eine mindestens um den Faktor 3000 verminderte Affinität zum L-Typ Calcium-Kanal aufweisen. Verbindungen mit einer solchen geringen Residualbindungsaffinität zum L-Typ Calcium-Kanal zeigen *in vivo* keine ausgeprägten hämodynamischen Effekte mehr, die über den L-Typ Calcium-Kanal vermittelt sind.

### 3. In vitro-Test zur funktionellen Charakterisierung möglicher Calcium-Kanalagonistischer oder -antagonistischer Wirkungen von Testverbindungen: Kaliumchlorid-induzierte Stimulation der isolierten Kaninchenaorta

Die frisch isolierte Thoraxaorta von männlichen New Zeeland White-Kaninchen wird entfernt und vom umgebenden Gewebe gereinigt. Dann werden Aortenringe von 2 mm Länge unter einer Anfangsspannung von 4 g in 10 ml-Organbäder mit auf 37°C erwärmter Krebs-Henseleit-Lösung gebracht. Kontraktionen von 40 mM KCl (submaximale Kontraktion) und 15 mM KCl (minimale Kontraktion) werden viermal im Abstand von 45 Minuten ausgelöst, um die Gefäße zu trainieren und eine stabile Ruhespannung zu erzeugen. Jeder Kontraktion folgt eine Serie von elf Spülzyklen und eine Ruhezeit von 30 Minuten bei vorheriger Nachspannung. Nach den vier Vorläufen erfolgt ohne weitere Nachspannung jeweils zu Beginn der Ruhephase die Zugabe der Testsubstanzen in die Organbäder. Die Konzentration der Testsubstanzen erhöht sich bei den vier folgenden Kontraktionen jeweils um den Faktor 10. Zur Wirkungsberechnung wird die Differenz zwischen der Basisspannung und dem vierten Vorlauf-Kontraktionswert als 100% gesetzt, die folgenden Kontraktionsspitzen beziehen sich auf diesen Wert. Diese Versuchsdurchführung ermöglicht die Differenzierung von Calcium-agonistischer (leichte Steigerung bei submaximaler Kontraktion, stärkere Steigerung bei minimaler Kontraktion) und Calcium-antagonistischer Substanzwirkung (Senkung bei submaximaler Kontraktion, stärkere Senkung bei minimaler Kontraktion).

In diesem funktionellen Assay am isolierten Organ wird für einen klassischen Calcium-Antagonisten vom Dihydropyridin-Typ, wie z.B. Nifedipin, ein IC₅₀-Wert von 0.1 nM bis 0.4 nM gemessen, während untersuchte Beispiele der hier beschriebenen erfindungsgemäßen Verbindungen IC₅₀-Werte
von > 1 µM und somit eine mindestens um den Faktor 2500 verminderte Affinität zum L-Typ Calcium-Kanal aufweisen. Verbindungen mit einer solchen geringen Residualbindungsaffinität zum L-Typ Calcium-Kanal zeigen *in vivo* keine ausgeprägten hämodynamischen Effekte mehr, die über den L-Typ Calcium-Kanal vermittelt sind.

### 4. In vivo-Test zum Nachweis der kardiovaskulären Wirkung: Diurese-Untersuchungen an wachen Ratten in Stoffwechselkäfigen

Wistar-Ratten (250-350 g Körpergwicht) werden mit freiem Zugang zu Futter (Altromin) und Trinkwasser gehalten. Ab ca. 72 Stunden vor Versuchsbeginn erhalten die Tiere anstelle des normalen Futters ausschließlich Kochsalz-reduziertes Futter mit einem Gehalt von 0.02% Natriumchlorid (ssniff R/M-H, 10 mm mit 0.02% Na, S0602-E081, Fa. ssniff Spezialdiäten GmbH, D-59494 Soest). Während des Versuches werden die Tiere für ca. 24 Stunden einzeln in für Ratten dieser Gewichtsklasse geeigneten Stoffwechselkäfigen (Fa. Tecniplast Deutschland GmbH, D-82383 Hohenpeißenberg) mit freiem Zugang zu Kochsalz-reduziertem Futter und Trinkwasser gehalten. Am Versuchsbeginn wird den Tieren die zu prüfende Substanz in einem Volumen von 0.5 ml/kg Körpergewicht eines geeigneten Lösemittels mittels einer Schlundsonde in den Magen verabreicht. Als Kontrolle dienende Tiere erhalten nur Lösemittel. Kontrollen und Substanztestungen werden am selben Tag parallel durchgeführt. Kontrollgruppen und Substanz-Dosisgruppen bestehen aus jeweils 3 bis 6 Tieren. Während des Versuchs wird der von den Tieren ausgeschiedene Urin kontinuierlich in einem Auffangbehälter am Käfigboden gesammelt. Für jedes Tier wird gesondert das Urinvolumen pro Zeiteinheit bestimmt und die Konzentration der im Urin ausgeschiedenen Natrium- bzw. Kalium-Ionen mittels flammenphotometrischer Standardmethoden gemessen. Aus den Messwerten wird der Natrium/Kalium-Quotient als ein Maß für die Substanzwirkung berechnet. Die Messintervalle betragen typischerweise den Zeitraum bis zu 8 Stunde nach Versuchsbeginn (Tagintervall) und den Zeitraum von 8 bis 24 Stunden nach Versuchsbeginn (Nachtintervall). In einer abgewandelten Versuchsanordnung wird der Urin während des Tagintervalls im Abstand von zwei Stunden gesammelt und gemessen. Um eine hierfür ausreichende Urinmenge zu erhalten, wird den Tieren zu Versuchsbeginn und dann im Abstand von zwei Stunden per Schlundsonde eine definierte Menge Wasser zugeführt.

### 5. DOCA/Salz-Modell

Die Verabreichung von Desoxycorticosteronacetat (DOCA) in Kombination mit einer Hochsalzdiät und einseitiger Nierenentfernung induziert bei der Ratte einen Hypertonus, der durch relativ niedrige Reninspiegel charakterisiert ist. Als Folge dieser endokrinen Hypertonie (DOCA ist eine direkte Vorstufe von Aldosteron) kommt es in Abhängigkeit von der gewählten DOCA-Konzentration zu einer Hypertrophie des Herzens und weiteren Endorgan-Schäden, z.B. der Niere, die u.a. durch Proteinurie und Glomerulosklerose charakterisiert sind. In diesem Rattenmodell lassen sich somit Testsubstanzen auf vorhandene antihypertrophe und Endorgan-schützende Wirkung hin untersuchen.

Etwa 8 Wochen alte (Körpergewicht zwischen 250 und 300 Gramm), männliche Sprague Dawley (SD)-Ratten werden linksseitig uninephrektomiert. Dazu werden die Ratten mit 1.5-2%-igem Isofluran in einer Mischung aus 66% N₂O und 33% O₂ anästhesiert und die Niere über einen Flankenschnitt entfernt. Als spätere Kontrolltiere dienen sogenannte sham-operierte Tiere, denen keine Niere entfernt wird.

Uninephrektomierte SD-Ratten erhalten 1% Natriumchlorid im Trinkwasser und einmal wöchentlich eine subkutane Injektion von Desoxycorticosteronacetat (gelöst in Sesamöl; Fa. Sigma) zwischen die Schulterblätter gespritzt (Hochdosis: 100 mg/kg/Woche s.c.; Normaldosis: 30 mg/ kg/Woche s.c.).

Die Substanzen, die auf ihre protektive Wirkung *in vivo* untersucht werden sollen, werden per Gavage oder über das Futter (Fa. Ssniff) verabreicht. Die Tiere werden einen Tag vor Versuchsbeginn randomisiert und Gruppen mit gleicher Tierzahl, in der Regel n = 10, zugeordnet. Während des gesamten Versuchs steht den Tieren Trinkwasser und Futter *ad libitum* zur Verfügung. Die Substanzen werden einmal täglich 4-8 Wochen lang per Gavage oder per Futter verabreicht. Als Plazebogruppe dienen Tiere, die genauso behandelt werden, aber entweder nur das Lösungsmittel oder das Futter ohne Testsubstanz erhalten.

Die Wirkung der Testsubstanzen wird durch Messung hämodynamischer Parameter [Blutdruck, Herzfrequenz, Inotropie (dp/dt), Relaxationszeit (tau), maximaler linksventrikulärer Druck, linksventrikulärer enddiastolischer Druck (LVEDP)], Gewichtsbestimmung von Herz, Niere und Lunge, Messung der Proteinausscheidung sowie durch Messung der Genexpression von Biomarkem (z.B. ANP, Atrial Natriuretic Peptide, und BNP, Brain Natriuretic Peptide) mittels RT/TaqMan-PCR nach RNA-Isolation aus kardialem Gewebe bestimmt.

Die statistische Auswertung erfolgt mit Student's t-Test nach vorheriger Überprüfung der Varianzen auf Homogenität.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.
Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 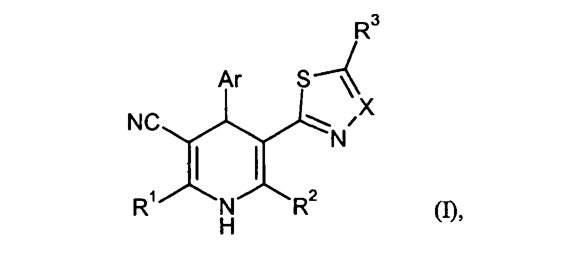 in welcher
Ar für (C₆-C₁₀)-Aryl oder 5- bis 10-gliedriges Heteroaryl mit bis zu zwei Ring-Hetero- atomen aus der Reihe N, O und/oder S steht, welche jeweils ein- bis dreifach, gleich oder verschieden, mit Substituenten ausgewählt aus der Reihe Halogen, Cyano, (C₁-C₄)-Alkyl, (C₁-C₄)-Alkoxy und (C₁-C₄)-Alkylthio substituiert sein können, wobei die genannten Alkyl-, Alkoxy- und Alkylthio-Reste ihrerseits mit Cyano oder bis zu dreifach mit Fluor substituiert sein können,
oder
Ar für eine Gruppe der Formel 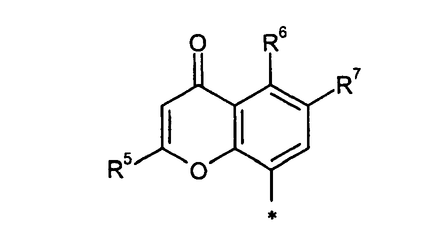
steht, worin
* die Verknüpfungsstelle mit dem Dihydropyridin-Ring,
R⁵ Wasserstoff oder (C₁-C₄)-Alkyl,
R⁶ Wasserstoff, Fluor, Chlor, Cyano, Nitro, Trifluormethyl oder (C₁-C₄)- Alkyl, und
R⁷ Wasserstoff oder Fluor bedeuten,
R¹ für (C₁-C₆)-Alkyl, das mit Phenyl substituiert sein kann, oder für (C₁-C₆)-Alkylthio steht, wobei die genannten Alkyl- und Alkylthio-Reste ihrerseits bis zu dreifach mit Fluor substituiert sein können,
R² für (C₁-C₆)-Alkyl, das mit Cyano, Hydroxy, (C₁-C₄)-Alkoxy, Trifluormethoxy, Amino, Mono-(C₁-C₄)-alkylamino, Di-(C₁-C₄)-alkylamino, (C₃-C₆)-Cycloalkyl, Phenyl oder bis zu dreifach mit Fluor substituiert sein kann, oder für (C₃-C₆)- Cycloalkyl steht,
X für N oder C-R⁴ steht, und
R³ und R⁴ gleich oder verschieden sind und unabhängig voneinander für Wasserstoff, Halogen, Amino, Mono-(C₁-C₄)-alkylamino oder Di-(C₁-C₄)-alkylamino, für (C₁- C₄)-Alkyl, das mit Hydroxy, (C₁-C₄)-Alkoxy, Amino, Mono-(C₁-C₄)-alkylamino, Di-(C₁-C₄)-alkylamino oder bis zu dreifach mit Fluor substituiert sein kann, oder für Phenyl, das mit Halogen, Cyano, (C₁-C₄)-Alkyl, Trifluormethyl, (C₁-C₄)- Alkoxy oder Trifluormethoxy substituiert sein kann, stehen
oder
R³ und R⁴, wenn letzteres vorhanden ist, miteinander verknüpft sind und zusammen mit den Kohlenstoffatomen, an die sie gebunden sind, einen anellierten 5- bis 7- gliedrigen Cycloalkyl-Ring, welcher mit (C₁-C₄)-Alkyl oder (C₁-C₄)-Alkoxy substi- tuiert sein kann und in welchem eine Ring-CH₂-Gruppe gegen ein O-Atom aus- getauscht sein kann, oder einen anellierten Phenyl- oder Pyridyl-Ring, welcher jeweils mit Halogen, Cyano, (C₁-C₄)-Alkyl, Trifluormethyl, (C₁-C₄)-Alkoxy oder Trifluormethoxy substituiert sein kann, bilden,
sowie ihre Salze, Solvate und Solvate der Salze.

2. Verbindung der Formel (I) nach Anspruch 1, in welcher
Ar für eine Gruppe der Formel 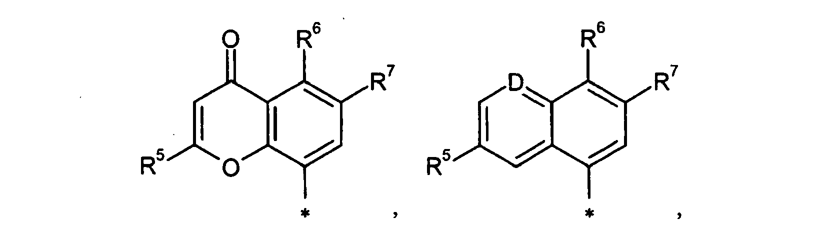 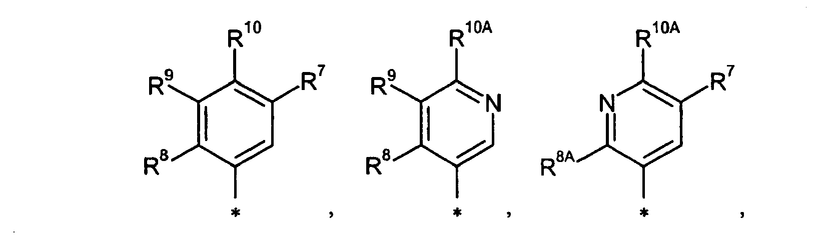 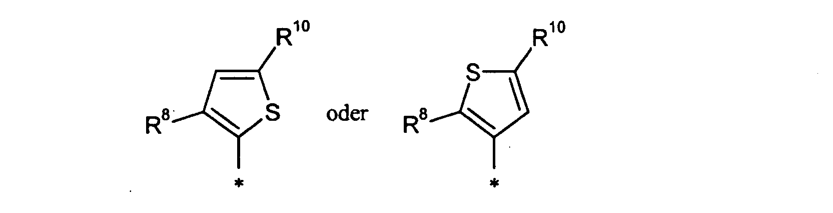
steht, worin
* die Verknüpfungsstelle mit dem Dihydropyridin-Ring,
D N oder CH,
R⁵ Wasserstoff, Methyl oder Ethyl,
R⁶ Wasserstoff, Fluor, Chlor oder Cyano,
R⁷ Wasserstoff oder Fluor,
R⁸ Fluor, Chlor, Brom, Cyano oder (C₁-C₄)-Alkyl, (C₁-C₄)-Alkoxy oder (C₁- C₄)-Alkylthio, welche jeweils bis zu dreifach mit Fluor substituiert sein können,
R^{8A} Cyano oder (C₁-C₄)-Alkyl, (C₁-C₄)-Alkoxy oder (C₁,-C₄)-Alkylthio, welche jeweils bis zu dreifach mit Fluor substituiert sein können,
R⁹ Wasserstoff, Fluor, Chlor oder Methyl,
R¹⁰ Wasserstoff, Cyano, Fluor, Chlor oder Brom, und
R^{10A} Wasserstoff oder Cyano bedeuten,
R¹ für (C₁-C₄)-Alkyl, das bis zu dreifach mit Fluor substituiert sein kann, steht,
R² für (C₁-C₄)-Alkyl, das mit (C₁-C₄)-Alkoxy oder bis zu dreifach mit Fluor substi- tuiert sein kann, steht,
X für N oder C-R⁴ steht, und
R³ und R⁴ gleich oder verschieden sind und unabhängig voneinander für Wasserstoff, Fluor, Chlor, Amino, Mono-(C₁-C₄)-alkylamino, Di-(C₁-C₄)-alkylamino oder (C₁- C₄)-Alkyl stehen
oder
R³ und R⁴, wenn letzteres vorhanden ist, miteinander verknüpft sind und zusammen mit dem Thiazol-Ring, an den sie gebunden sind, eine Gruppe der Formel 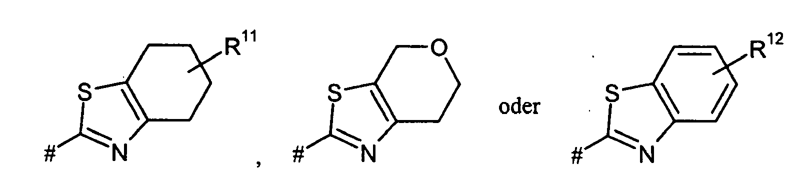
bilden, worin
# die Verknüpfungsstelle mit dem Dihydropyridin-Ring,
R¹¹ Wasserstoff, (C₁-C₄)-Alkyl oder (C₁-C₄)-Alkoxy, und
R¹² Wasserstoff, Fluor, Chlor, Brom, (C₁-C₄)-Alkyl, Trifluormethyl, (C₁-C₄)- Alkoxy oder Trifluormethoxy
bedeuten,
sowie ihre Salze, Solvate und Solvate der Salze.

3. Verbindung der Formel (I) nach Anspruch 1 oder 2, in welcher
Ar für eine Gruppe der Formel 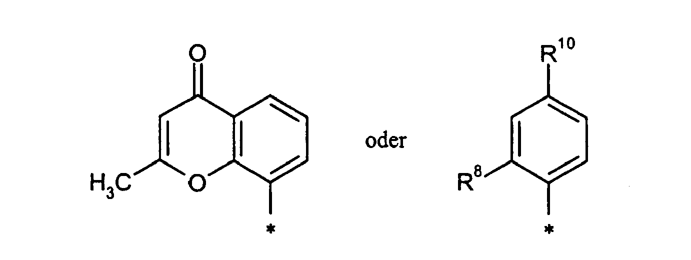
steht, worin
* die Verknüpfungsstelle mit dem Dihydropyridin-Ring,
R⁸ Fluor, Chlor, Brom, Cyano, Methyl, Ethyl, Trifluormethyl, Methoxy oder Trifluormethoxy, und
R¹⁰ Fluor, Chlor oder Cyano bedeuten,
R¹ für Methyl oder Trifluormethyl steht,
R² für Methyl, Ethyl, n-Propyl oder Methoxymethyl steht,
X für C-R⁴ steht,
R³ für Wasserstoff, Methyl oder Ethyl steht,
R⁴ für Wasserstoff, Methyl, Ethyl oder n-Propyl steht, oder
R³ und R⁴ miteinander verknüpft sind und zusammen mit dem Thiazol-Ring, an den sie gebunden sind, eine Gruppe der Formel 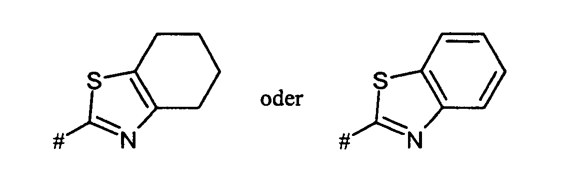
bilden, worin
# die Verknüpfungsstelle mit dem Dihydropyridin-Ring bedeutet,
sowie ihre Salze, Solvate und Solvate der Salze.

4. Verfahren zur Herstellung von Verbindungen der Formel (I), wie in den Ansprüchen 1 bis 3 definiert, **dadurch gekennzeichnet, dass** man eine Verbindung der Formel (II) 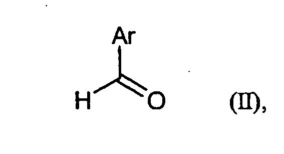 in welcher Ar die in den Ansprüchen 1 bis 3 angegebene Bedeutung hat, entweder
[A] in einem einstufigen Verfahren (Eintopf-Reaktion) mit einer Verbindung der Formel (III) 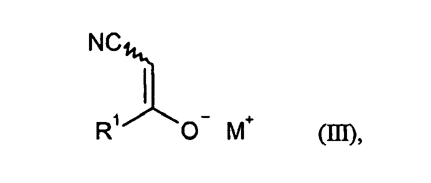 in welcher R¹ die in den Ansprüchen 1 bis 3 angegebene Bedeutung hat und
M⁺ für ein Alkalimetall-Ion wie Li⁺, Na⁺ oder K⁺ steht,
und einer Verbindung der Formel (IV) 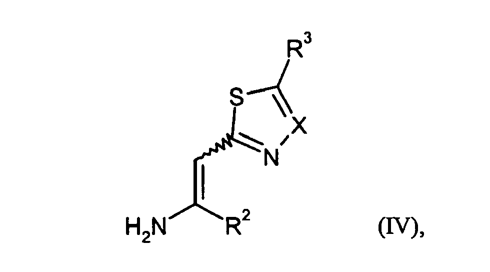 in welcher R², R³ und X jeweils die in den Ansprüchen 1 bis 3 angegebenen Bedeutungen haben, umsetzt
oder
[B] in einem einstufigen Verfahren (Eintopf-Reaktion) mit einer Verbindung der Formel (V) 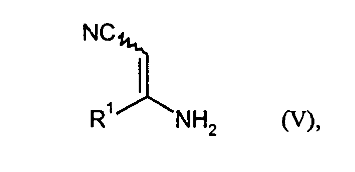 in welcher R¹ die in den Ansprüchen 1 bis 3 angegebenen Bedeutungen hat, und einer Verbindung der Formel (VI) 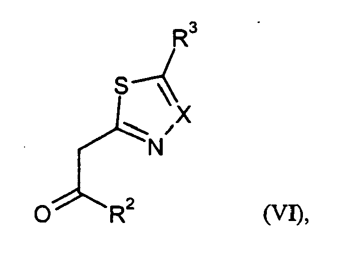 in welcher R², R³ und X jeweils die in den Ansprüchen 1 bis 3 angegebenen Bedeutungen haben, umsetzt
oder
[C] in einem zweistufigen Verfahren zunächst mit einer Verbindung der Formel (VI) in eine Verbindung der Formel (VII) 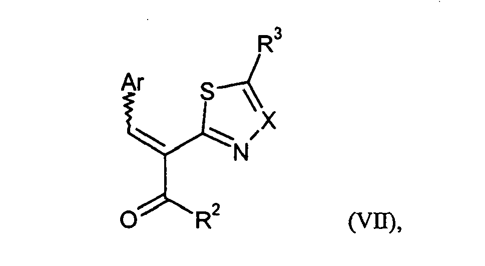 in welcher Ar, R², R³ und X jeweils die in den Ansprüchen 1 bis 3 angegebenen Bedeutungen haben,
überführt und diese dann in einem zweiten Schritt mit einer Verbindung der Formel (V) umsetzt,
und gegebenenfalls die resultierenden Verbindungen der Formel (I) nach dem Fachmann bekannten Methoden in ihre Enantiomere und/oder Diastereomere trennt und/oder mit den entsprechenden (i) Lösungsmitteln und/oder (ii) Basen oder Säuren in ihre Solvate, Salze und/oder Solvate der Salze überführt.

5. Verbindung der Formel (I), wie in einem der Ansprüche 1 bis 3 definiert, zur Behandlung und/oder Prophylaxe von Krankheiten.

6. Verwendung einer Verbindung der Formel (I), wie in einem der Ansprüche 1 bis 3 definiert, zur Herstellung eines Arzneimittels zur Behandlung und/oder Prophylaxe von Aldosteronismus, Bluthochdruck, chronischer Herzinsuffizienz, den Folgen eines Myokardinfarkts, Leberzirrhose, Niereninsuffizienz und Hirnschlag.

7. Arzneimittel enthaltend eine Verbindung der Formel (I), wie in einem der Ansprüche 1 bis 3 definiert, in Kombination mit einem inerten, nicht-toxischen, pharmazeutisch geeigneten Hilfsstoff.

8. Arzneimittel enthaltend eine Verbindung der Formel (I), wie in einem der Ansprüche 1 bis 3 definiert, in Kombination mit einem oder mehreren weiteren Wirkstoffen ausgewählt aus der Gruppe bestehend aus ACE-Inhibitoren, Renin-Inhibitoren, Angiotensin II-Rezeptor-Antagonisten, Beta-Blocker, Acetylsalicylsäure, Diuretika, Kalium-Supplements, Calcium-Ahtagonisten, Statine, Digitalis (Digoxin)-Derivate, Calcium-Sensitizer, Nitrate sowie Antithrombotika.

9. Arzneimittel nach Anspruch 7 oder 8 zur Behandlung und/oder Prophylaxe von Aldosteronismus, Bluthochdruck, chronischer Herzinsuffizienz, den Folgen eines Myokardinfarkts, Leberzirrhose, Niereninsuffizienz und Hirnschlag.

## Claims

1. Compound of the formula (I) 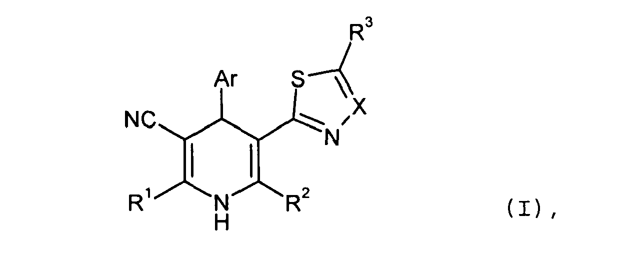 in which
Ar is (C₆-C₁₀) -aryl or 5- to 10-membered heteroaryl having up to two ring heteroatoms from the series N, O and/or S, each of which may be substituted once to three times, identically or differently, by substituents selected from the series halogen, cyano, (C₁- C₄)-alkyl, (C₁-C₄)-alkoxy and (C₁-C₄) alkylthio, where said alkyl, alkoxy and alkylthio radicals may in turn be substituted by cyano or up to three times by fluorine,
or
Ar is a group of the formula 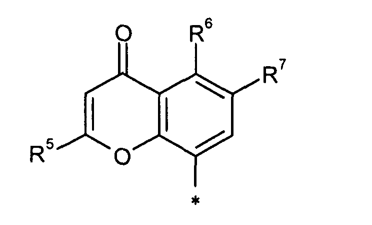
in which
* is the point of linkage to the dihydropyridine ring,
R⁵ is hydrogen or (C₁-C₄)-alkyl,
R⁶ is hydrogen, fluorine, chlorine, cyano, nitro, trifluoromethyl or (C₁-C₄)-alkyl,
and
R⁷ is hydrogen or fluorine,
R¹ is (C₁-C₆)-alkyl which may be substituted by phenyl, or is (C₁-C₆)-alkylthio, where said alkyl and alkylthio radicals may in turn be substituted up to three times by fluorine,
R² is (C₁-C₆)-alkyl which may be substituted by cyano, hydroxy, (C₁-C₄)-alkoxy, trifluoromethoxy, amino, mono-(C₁-C₄)- alkylamino, di-(C₁-C₄)-alkylamino, (C₃-C₆)- cycloalkyl, phenyl or up to three times by fluorine, or is (C₃-C₆)-cycloalkyl,
X is N or C-R⁴, and
R³ and R⁴ are identical or different and
or
independently of one another are hydrogen, halogen, amino, mono-(C₁-C₄)-alkylamino or di- (C₁-C₄)-alkylamino, are (C₁-C₄)-alkyl which may be substituted by hydroxy, (C₁-C₄)-alkoxy, amino, mono-(C₁-C₄)-alkylamino, di-(C₁-C₄)- alkylamino or up to three times by fluorine, or are phenyl which may be substituted by halogen, cyano, (C₁-C₄)-alkyl, trifluoromethyl, (C₁-C₄)-alkoxy or trifluoromethoxy,
R³ and R⁴, if the latter is present, are linked together and form, together with the carbon atoms to which they are bonded, a fused 5- to 7-membered cycloalkyl ring which may be substituted by (C₁-C₄)-alkyl or (C₁-C₄)-alkoxy, and in which one ring CH₂ group may be replaced by an O atom, or a fused phenyl or pyridyl ring, each of which may be substituted by halogen, cyano, (C₁-C₄)-alkyl, trifluoromethyl, (C₁-C₄)-alkoxy or trifluoromethoxy, and the salts, solvates and solvates of the salts thereof.

2. Compound of the formula (I) according to Claim 1, in which
Ar is a group of the formula 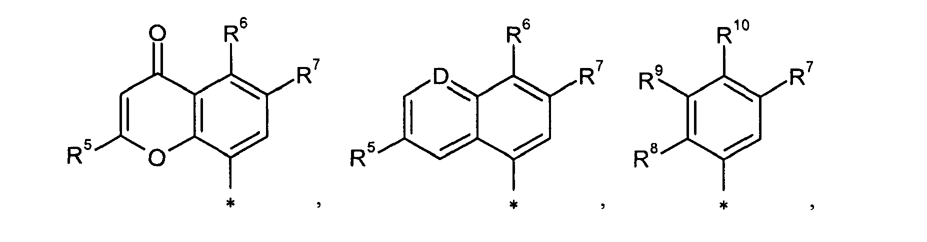 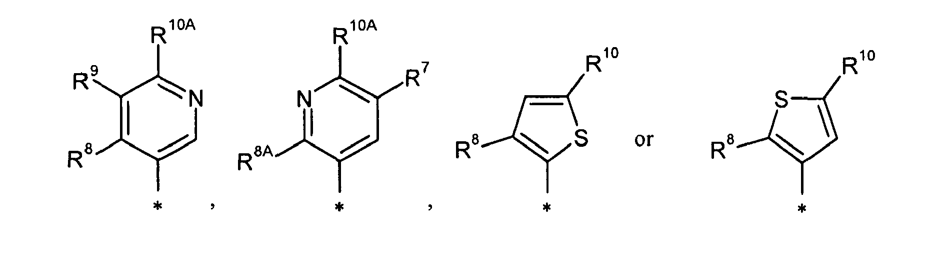
in which
* is the point of linkage to the dihydropyridine ring,
D is N or CH,
R⁵ is hydrogen, methyl or ethyl,
R⁶ is hydrogen, fluorine, chlorine or cyano,
R⁷ is hydrogen or fluorine,
R⁸ is fluorine, chlorine, bromine, cyano or (C₁-C₄)-alkyl, (C₁-C₄)-alkoxy or (C₁-C₄)- alkylthio, each of which may be substituted up to three times by fluorine,
R^{8A} is cyano or (C₁-C₄)-alkyl, (C₁-C₄)-alkoxy or (C₁-C₄)-alkylthio, each of which may be substituted up to three times by fluorine,
R⁹ is hydrogen, fluorine, chlorine or methyl,
R¹⁰ is hydrogen, cyano, fluorine, chlorine or bromine,
and
R^{10A} is hydrogen or cyano,
R¹ is (C₁-C₄)-alkyl which may be substituted up to three times by fluorine,
R² is (C₁-C₄)-alkyl which may be substituted by (C₁-C₄)-alkoxy or up to three times by fluorine,
X is N or C-R⁴, and
R³ and R⁴ are identical or different and independently of one another are hydrogen, fluorine, chlorine, amino, mono-(C₁-C₄)- alkylamino, di-(C₁-C₄)-alkylamino or (C₁-C₄)- alkyl, or
R³ and R⁴, if the latter is present, are linked together and form, together with the thiazole ring to which they are bonded, a group of the formula 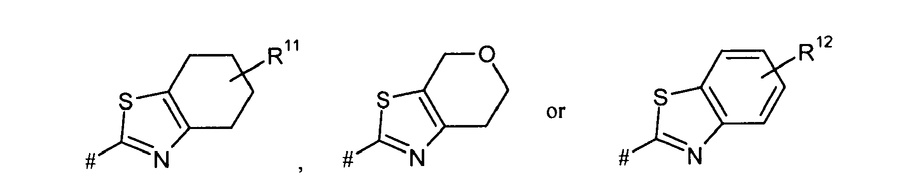
in which
# is the point of linkage to the dihdyropyridine ring,
R¹¹ is hydrogen, (C₁-C₄)-alkyl or (C₁-C₄)- alkoxy, and
R¹² is hydrogen, fluorine, chlorine, bromine, (C₁-C₄)-alkyl, trifluoromethyl, (C₁-C₄)-alkoxy or trifluoromethoxy,
and the salts, solvates and solvates of the salts thereof.

3. Compound of the formula (I) according to Claim 1 or 2, in which
Ar is a group of the formula
in which
* is the point of linkage to the dihydropyridine ring,
R⁸ is fluorine, chlorine, bromine, cyano, methyl, ethyl, trifluoromethyl, methoxy or trifluoromethoxy,
and
R¹ R¹⁰ is fluorine, chlorine or cyano, is methyl or trifluoromethyl,
R² is methyl, ethyl, n-propyl or methoxymethyl,
X is C-R⁴,
R³ is hydrogen, methyl or ethyl,
R⁴ is hydrogen, methyl, ethyl or n-propyl,
or
R³ and R⁴ are linked together and form, together with the thiazole ring to which they are bonded, a group of the formula 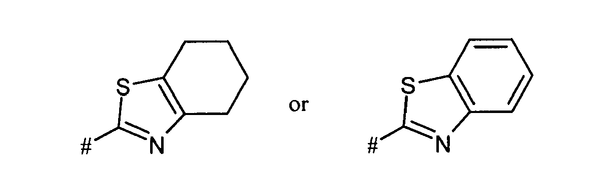
in which
# is the point of linkage to the dihydropyridine ring,
and the salts, solvates and solvates of the salts thereof.

4. Process for preparing compounds of the formula (I) as defined in Claims 1 to 3, **characterized in that** a compound of the formula (II) 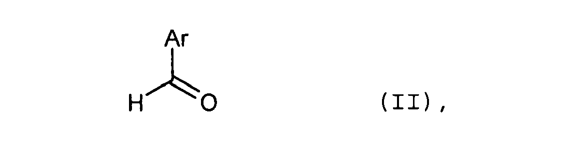 in which Ar has the meaning indicated in Claims 1 to 3,
is either
[A] reacted in a one-stage process (one-pot reaction) with a compound of the formula (III) 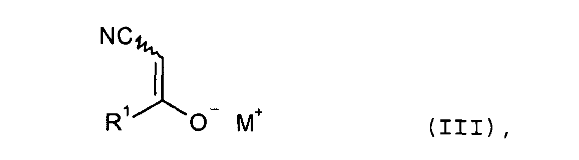 in which R¹ has the meaning indicated in Claims 1 to 3
and
M⁺ is an alkali metal ion such as Li⁺, Na⁺ or K⁺,
and a compound of the formula (IV) 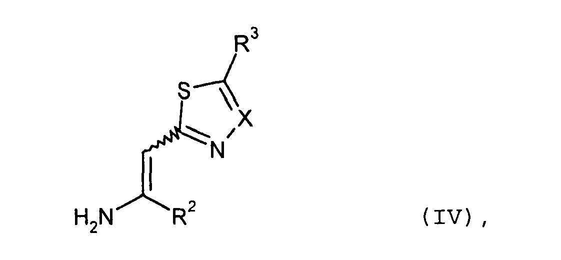 in which R², R³ and X each have the meanings indicated in Claims 1 to 3,
or
[B] reacted in a one-stage process (one-pot reaction) with a compound of the formula (V) 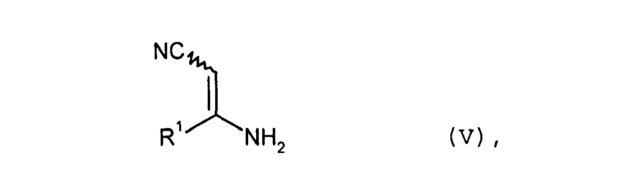 in which R¹ has the meanings indicated in Claims 1 to 3,
and a compound of the formula (VI) 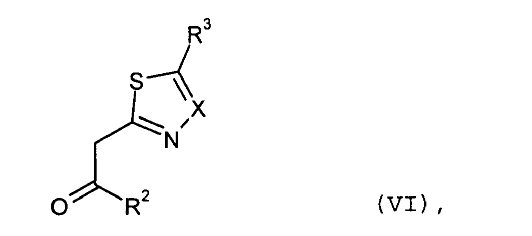 in which R², R³ and X each have the meanings indicated in Claims 1 to 3,
or
[C] in a two-stage process firstly converted with a compound of the formula (VI) into a compound of the formula (VII) 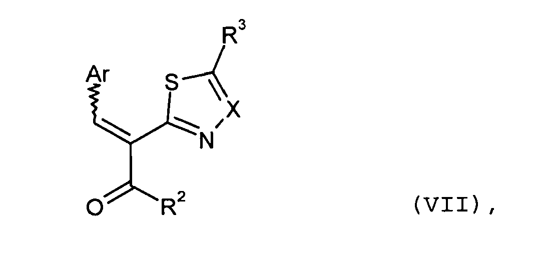 in which Ar, R², R³ and X each have the meanings indicated in Claims 1 to 3,
and the latter is then reacted in a second step with a compound of the formula (V),
and where appropriate the resulting compounds of the formula (I) are separated by methods known to the skilled worker into their enantiomers and/or diastereomers, and/or converted with the appropriate (i) solvents and/or (ii) bases or acids into the solvates, salts and/or solvates of the salts thereof.

5. Compound of the formula (I) as defined in any of Claims 1 to 3 for the treatment and/or prophylaxis of diseases.

6. Use of a compound of the formula (I) as defined in any of Claims 1 to 3 for the manufacture of a medicament for the treatment and/or prophylaxis of aldosteronism, high blood pressure, chronic heart failure, the sequelae of a myocardial infarction, cirrhosis of the liver, renal failure and stroke.

7. Medicament comprising a compound of the formula (I) as defined in any of Claims 1 to 3 in combination with an inert, non-toxic, pharmaceutically suitable excipient.

8. Medicament comprising a compound of the formula (I) as defined in any of Claims 1 to 3 in combination with one or more further active ingredients selected from the group consisting of ACE inhibitors, renin inhibitors, angiotensin II receptor antagonists, beta-blockers, acetylsalicylic acid, diuretics, potassium supplements, calcium antagonists, statins, digitalis (digoxin) derivatives, calcium sensitizers, nitrates and antithrombotics.

9. Medicament according to Claim 7 or 8 for the treatment and/or prophylaxis of aldosteronism, high blood pressure, chronic heart failure, the sequelae of a myocardial infarction, cirrhosis of the liver, renal failure and stroke.

## Revendications

1. Composé de formule (I) 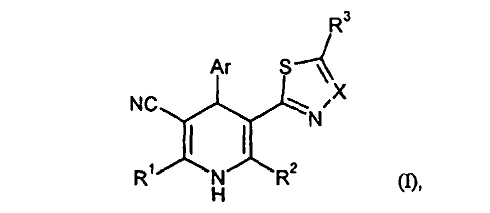 dans laquelle
Ar représente (C₆-C₁₀)-aryle ou hétéroaryle de 5 à 10 chaînons comprenant jusqu'à deux hétéroatomes de cycle de la série N, O et/ou S, qui peuvent à chaque fois être monosubstitués à trisubstitués, de manière identique ou différente, par des substituants choisis dans la série halogène, cyano, (C₁-C₄)-alkyle, (C₁-C₄)-alcoxy et (C₁-C₄)- alkylthio, où les radicaux alkyle, alcoxy et alkylthio mentionnés peuvent à leur tour être substitués par cyano ou jusqu'à trisubstitués par fluor, ou
Ar représente un groupe de formule 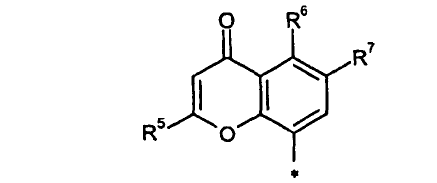
où
* signifie le site de liaison avec le cycle dihydropyridine,
R⁵ signifie hydrogène ou (C₁-C₄)-alkyle,
R⁶ signifie hydrogène, fluor, chlore, cyano, nitro, trifluorométhyle ou (C₁-C₄)-alkyle, et
R⁷ signifie hydrogène ou fluor,
R¹ représente (C₁-C₆)-alkyle, qui peut être substitué par phényle, ou représente (C₁-C₆)-alkylthio, où les radicaux alkyle et alkylthio mentionnés peuvent à leur tour être jusqu'à trisubstitués par fluor,
R² représente (C₁-C₆)-alkyle, qui peut être substitué par cyano, hydroxy, (C₁-C₄)-alcoxy, trifluorométhoxy, amino, mono-(C₁-C₄)-alkylamino, di-(C₁-C₄)-alkylamino, (C₃-C₆)-cycloalkyle, phényle ou jusqu'à trisubstitué par fluor, ou représente (C₃-C₆)-cycloalkyle,
X représente N ou C-R⁴, et
R³ et R⁴ sont identiques ou différents et représentent, indépendamment l'un de l'autre, hydrogène, halogène, amino, mono-(C₁-C₄)-alkylamino ou di-(C₁- C₄)-alkylamino, représentent (C₁-C₄)-alkyle, qui peut être substitué par hydroxy, (C₁-C₄)-alcoxy, amino, mono-(C₁-C₄)-alkylamino, di-(C₁-C₄)- alkylamino ou jusqu'à trisubstitué par fluor, ou représentent phényle, qui peut être substitué par halogène, cyano, (C₁-C₄)-alkyle, trifluorométhyle, (C₁-C₄)-alcoxy ou trifluorométhoxy, ou
R³ et R⁴, lorsque ce dernier est présent, sont liés l'un à l'autre et forment ensemble avec les atomes de carbone auxquels ils sont liés, un cycle cycloalkyle annelé de 5 à 7 chaînons, qui peut être substitué par (C₁-C₄)-alkyle ou (C₁-C₄)-alcoxy et dans lequel un groupe CH₂ de cycle peut être remplacé par un atome d'O ou forment un cycle phényle ou pyridyle annelé, qui peut à chaque fois être substitué par halogène, cyano, (C₁-C₄)-alkyle, trifluorométhyle, (C₁-C₄)-alcoxy ou trifluorométhoxy,
ainsi que ses sels, solvates et les solvates des sels.

2. Composé de formule (I) selon la revendication 1, dans laquelle
Ar représente un groupe de formule 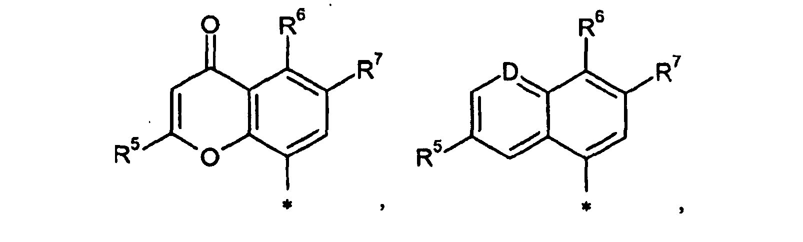 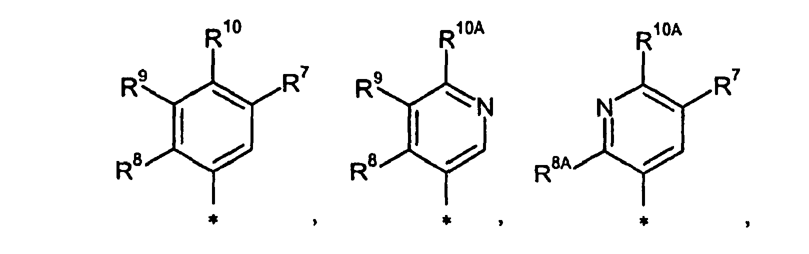 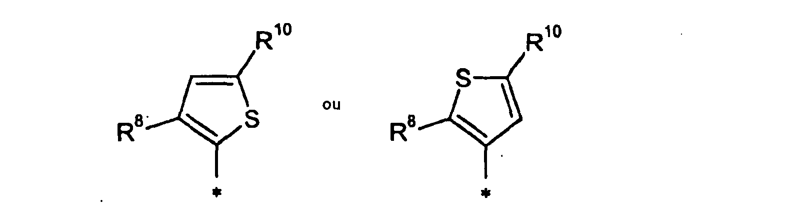
où
* signifie le site de liaison avec le cycle dihydropyridine,
D signifie N ou CH,
R⁵ signifie hydrogène, méthyle ou éthyle,
R⁶ signifie hydrogène, fluor, chlore ou cyano,
R⁷ signifie hydrogène ou fluor,
R⁸ signifie fluor, chlore, brome, cyano ou (C₁- C₄)-alkyle, (C₁-C₄)-alcoxy ou (C₁-C₄)- alkylthio, qui peuvent à chaque fois être jusqu'à trisubstitués par fluor,
R^{8A} signifie cyano ou (C₁-C₄)-alkyle, (C₁-C₄)- alcoxy ou (C₁-C₄)-alkylthio, qui peuvent à chaque fois être jusqu'à trisubstitués par fluor,
R⁹ signifie hydrogène, fluor, chlore ou méthyle,
signifie hydrogène, cyano, fluor, chlore ou brome, et
R^{10A} signifie hydrogène ou cyano,
R¹ représente (C₁-C₄)-alkyle, qui peut être jusqu'à trisubstitué par fluor,
R² représente (C₁-C₄)-alkyle, qui peut être substitué par (C₁-C₄)-alcoxy ou jusqu'à trisubstitué par fluor,
X représente N ou C-R⁴, et
R³ et R⁴ sont identiques ou différents et représentent indépendamment l'un de l'autre hydrogène, fluor, chlore, amino, mono- (C₁-C₄)-alkylamino, di-(C₁-C₄)- alkylamino ou (C₁-C₄)-alkyle ou
R³ et R⁴, lorsque ce dernier est présent, sont liés l'un à l'autre et forment ensemble avec le cycle thiazole, auquel ils sont liés, un groupe de formule 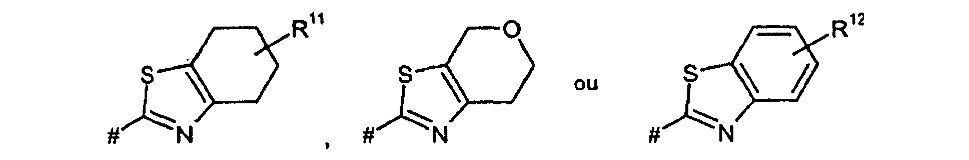
où
# signifie le site de liaison avec le cycle dihydropyridine,
R¹¹ signifie hydrogène, (C₁-C₄)-alkyle ou (C₁-C₄)- alcoxy, et
R¹² signifie hydrogène, fluor, chlore, brome, (C₁-C₄)- alkyle, trifluorométhyle, (C₁-C₄)-alcoxy ou trifluorométhoxy ainsi que ses sels, solvates et les solvates des sels.

3. Composé de formule (I) selon la revendication 1 ou 2, dans laquelle
Ar représente un groupe de formule 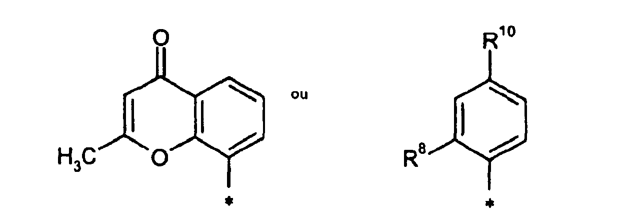
où
* signifie le site de liaison avec le cycle dihydropyridine,
R⁸ signifie fluor, chlore, brome, cyano, méthyle, éthyle, trifluorométhyle, méthoxy ou trifluorométhoxy, et
R¹ R¹⁰ signifie fluor, chlore ou cyano, représente méthyle ou trifluorométhyle,
R² représente méthyle, éthyle, n-propyle ou méthoxyméthyle,
X représente C-R⁴,
R³ représente hydrogène, méthyle ou éthyle,
R⁴ représente hydrogène, méthyle, éthyle ou n- propyle, ou
R³ et R⁴ sont liés l'un à l'autre et forment ensemble avec le cycle thiazole, auquel ils sont liés, un groupe de formule 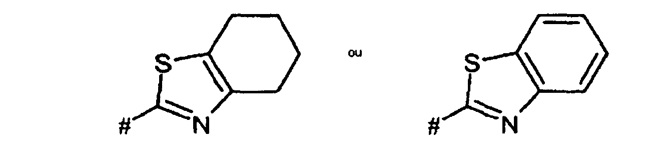 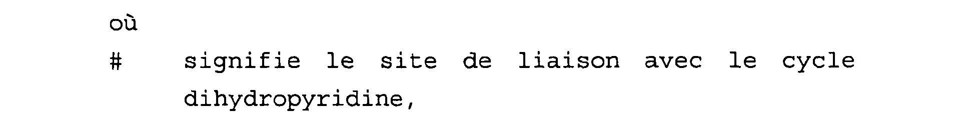
ainsi que ses sels, solvates et les solvates des sels.

4. Procédé pour la préparation de composés de formule (I), tels que définis dans les revendications 1 à 3, **caractérisé en ce qu'**on transforme un composé de formule (II) 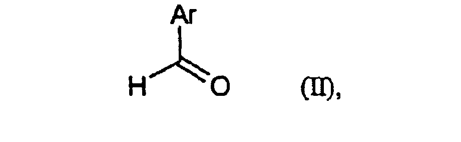 où Ar présente la signification indiquée dans les revendications 1 à 3, soit
[A] dans un procédé en une étape (réaction dans un pot) avec un composé de formule (III) 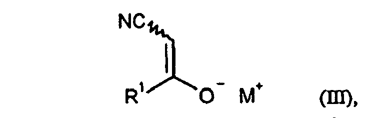 dans laquelle R¹ présente la signification indiquée dans les revendications 1 à 3 et
M⁺ représente un ion de métal alcalin tel que Li⁺, Na⁺ ou K⁺,
et un composé de formule (IV) 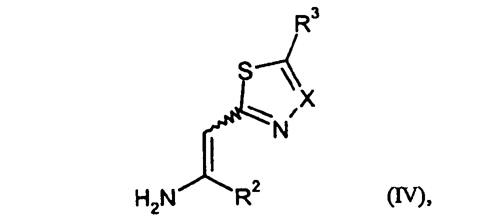 dans laquelle R², R³ et X présentent à chaque fois les significations indiquées dans les revendications 1 à 3, soit
[B] dans un procédé en une étape (réaction dans un pot) avec un composé de formule (V) 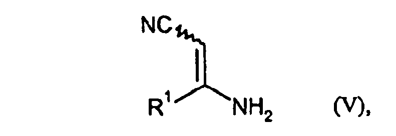 dans laquelle R¹ présente les significations indiquées dans les revendications 1 à 3 et un composé de formule (VI) 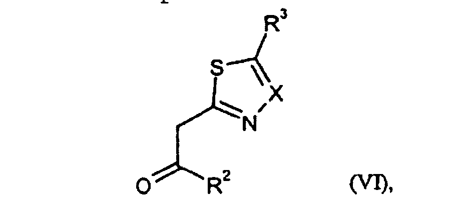 dans laquelle R², R³ et X présentent à chaque fois les significations indiquées dans les revendications 1 à 3, soit
[C] dans un procédé en deux étapes, d'abord avec un composé de formule (VI) en un composé de formule (VII) 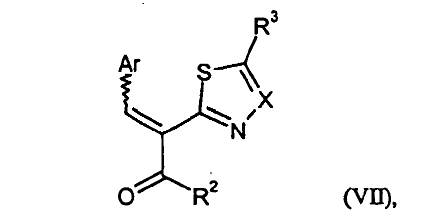 dans laquelle Ar, R², R³ et X présentent à chaque fois les significations indiquées dans les revendications 1 à 3,
puis on transforme celui-ci dans une deuxième étape avec un composé de formule (V),
et on transforme le cas échéant les composés de formule (I) obtenus selon des procédés connus par l'homme du métier en leurs énantiomères et/ou diastéréo-isomères et/ou avec (i) les solvants et/ou (ii) les bases ou acides correspondants en leurs solvates, sels et/ou solvates des sels.

5. Composé de formule (I), tel que défini dans l'une quelconque des revendications 1 à 3, destiné au traitement et/ou à la prophylaxie de maladies.

6. Utilisation d'un composé de formule (I), tel que défini dans l'une quelconque des revendications 1 à 3, pour la préparation d'un médicament pour le traitement et/ou la prophylaxie de l'aldostéronisme, de l'hypertension, de l'insuffisance cardiaque chronique, des conséquences d'un infarctus du myocarde, d'une cirrhose du foie, de l'insuffisance rénale et d'un accident cérébrovasculaire.

7. Médicament contenant un composé de formule (I), tel que défini dans l'une quelconque des revendications 1 à 3, en combinaison avec un adjuvant inerte, non toxique, pharmaceutiquement approprié.

8. Médicament contenant un composé de formule (I), tel que défini dans l'une quelconque des revendications 1 à 3, en combinaison avec une ou plusieurs autres substances actives, choisies dans le groupe constitué par les inhibiteurs d'ACE, les inhibiteurs de rénine, les antagonistes du récepteur de l'angiotensine II, les bêtabloquants, l'acide acétylsalicylique, les diurétiques, les suppléments de potassium, les antagonistes de calcium, les statines, les dérivés de digitalis (Digoxine), les agents de sensibilisation au calcium, les nitrates ainsi que les antithrombotiques.

9. Médicament selon la revendication 7 ou 8 pour le traitement et/ou la prophylaxie de l'aldostéronisme, de l'hypertension, de l'insuffisance cardiaque chronique, des conséquences d'un infarctus du myocarde, d'une cirrhose du foie, de l'insuffisance rénale et d'un accident cérébrovasculaire.
